(19) Europäisches Patentamt
European Patent Office
Office européen des brevets

(11) EP 2 307 443 B1

(12) # EUROPEAN PATENT SPECIFICATION

(45) Date of publication and mention of the grant of the patent:
**02.10.2013 Bulletin 2013/40**

(21) Application number: **09787518.1**

(22) Date of filing: **09.08.2009**

(51) Int Cl.:
***C07K 1/22*** *(2006.01)* ***C07K 14/33*** *(2006.01)*

(86) International application number:
**PCT/IL2009/000773**

(87) International publication number:
**WO 2010/016067 (11.02.2010 Gazette 2010/06)**

(54) **AFFINITY PURIFICATION BY COHESIN-DOCKERIN INTERACTION**

AFFINITÄTSREINIGUNG MITTELS COHESIN-DOCKERIN-INTERAKTION

PURIFICATION PAR AFFINITÉ PAR INTERACTION COHÉSINE-DOCKÉRINE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.08.2008 US 86813 P**
**01.09.2008 US 93371 P**

(43) Date of publication of application:
**13.04.2011 Bulletin 2011/15**

(73) Proprietors:
- **Yeda Research And Development Company Ltd.**
  **76100 Rehovot (IL)**
- **Ramot at Tel-Aviv University Ltd.**
  **61392 Tel Aviv (IL)**

(72) Inventors:
- **BAYER, Edward, A.**
  **45930 Ramot Hashavim (IL)**
- **KARPOL, Alon**
  **76100 Rehovot (IL)**
- **LAMED, Raphael**
  **81517 Yavne (IL)**

(74) Representative: **Becker Kurig Straus**
**Bavariastrasse 7**
**80336 München (DE)**

(56) References cited:
**EP-A- 1 441 030**

- **A KARPOL ET AL.: "Functional asymmetry in cohesin binding ..." BIOCHEMICAL JOURNAL, vol. 410, no. 2, 1 March 2008 (2008-03-01) , pages 331-338, XP8118409 THE BIOCHEMICAL SOCIETY ISSN: 0264-6021**
- **A LEVASSEUR ET AL.: "Design and production in Aspergillus niger ..." APPLIED AND ENVIRONMENTAL MICROBIOLOGY, vol. 70, no. 12, December 2004 (2004-12), pages 6984-6991, XP2364246 American Society for Microbiology ISSN: 0099-2240**
- **S J CRAIG ET AL.: "Engineered proteins containing the cohesin ..." JOURNAL OF BIOTECHNOLOGY, vol. 121, 2006, pages 165-173, XP5236109 ELSEVIER SCIENCE PUBLISHERS, AMSTERDAM. ISSN: 0168-1656 cited in the application**
- **A KARPOL ET AL.: "Engineering a reversible, high affinity system ..." JOURNAL OF MOLECULAR RECOGNITION, vol. 22, 31 October 2008 (2008-10-31), pages 91-98, XP002568925 HEYDEN & SON LTD., LONDON. ISSN: 0952-3499**
- **V GARCIA-SAMPAYO & P BÉGUIN: "Synergism between the cellulosome-integrating protein CipA ..." JOURNAL OF BIOTECHNOLOGY, vol. 57, 1997, pages 39-47, XP4097725 ELSEVIER SCIENCE PUBLISHERS, AMSTERDAM. ISSN: 0168-1656**
- **Y BARAK ET AL.: "Matching fusion proteins systems for affinity ..." JOURNAL OF MOLECULAR RECOGNITION, vol. 18, 2005, XP2458643 HEYDEN & SON LTD., LONDON. ISSN: 0952-3499**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 307 443 B1

## Description

### FIELD OF THE INVENTION

The present invention is directed to truncated dockerin polypeptides, recombinant polypeptides and affinity systems comprising the truncated dockerin polypeptide, methods of generating same, and methods of use thereof to purify, isolate, and detect molecules of interest.

### BACKGROUND OF THE INVENTION

#### Affinity chromatography

Affinity chromatography is a preferred separation technique for isolating biologically active compounds. The binding constant of the immobilized ligand to the target biomolecule, being purified in affinity chromatography systems is determined based on the desired selectivity of the column, good column retention, the capacity of the column, and elution conditions. For example, a very high affinity between the ligand and the biomolecule may require harsh elution conditions (such as low/high pH or very high salt concentrations), which may lead to unfolding or denaturation in the case of a recombinant protein. On the other hand, overly weak affinities may be insufficient for efficient retention of the target protein on the column.

One approach that has been utilized is the use of affinity tags. Affinity tags can be fused to any recombinant protein of interest, allowing rapid, facile purification using the affinity properties of the tag, rather than those of the target protein. Some tags are relatively small in size, such as the His tag, which can be fused either at the N- or C- terminus of a recombinant protein for purification using immobilized metal ion affinity chromatography (IMAC) . Small affinity tags show minimal interaction with the targeted protein; hence they usually do not disrupt or impair protein activity. Nevertheless, His tags fail to provide a highly specific interaction; the column capacity is consequently low, and impurities frequently contaminate the sample. Immuno- affinity chromatography employing immobilized protein A or protein G is another preferred system for antibody purification. Other affinity chromatography systems employ affinity tags such as maltose- binding protein (MBP) that binds to amylose resins; glutathione- S- transferase (GST) that binds to glutathione-immobilized matrices; and a FLAG™ fusion tag (Asp- Tyr- Lys- Asp- Asp- Asp- Asp- Lys) which binds to an anti- flag antibody, through which the target protein can be eluted under mild conditions. In some cases, these affinity tags are relatively large, sometimes even larger than the target protein of interest.

#### Cohesins and dockerins

Cellulose, the most abundant biopolymer on earth, holds great potential as an energy source for living organisms. Indeed many microorganisms have evolved to grow on cellulose. While many fungi and aerobic bacteria attack the recalcitrant cellulose substrate by secreting different synergistically acting cellulases, some anaerobic bacteria produce a multi- component enzyme complex, the cellulosome, for efficient degradation of cellulose. The most studied cellulosome- producing microbe is *Clostridium thermocellum,* a thermophilic, anaerobic, gram- positive bacterium that lives solely on cellulose and its degradation products (Bayer *et al*., 1998) . Cellulosomes are defined as multienzyme complexes having high activity against crystalline cellulose and related plant cell wall polysaccharides such as xylan, mannan, and pectin.

As described in Jindou *et al.,* 2004, cellulosomes have been identified and characterized in cellulolytic clostridia such as *Clostridium thermocellum* (Lamed *et al.,* 1983), *C. cellulolyticum* (Pages *et al.,* 1996), C. *cellulovorans* (Doi *et al.,* 1994), *C. papyrosolvens* (Pohlschroder 1994) *C. josui* (Kakiuchi *et al.,* 1998), *Acetivibrio cellulolyticus* (Ding *et al.,* 1999), *Bacteroides cellulosolvens* (Ding *et al.,* 2000), *R. flavefaciens* (Kirby *et al.,* 1997), *Ruminococcus albus* (Ohara *et al.,* 2000), and *Clostridium cellobioparum* (Lamed *et al.,* 1987). A common feature of the clostridial cellulosomes is that they consist of a large number of catalytic components arranged around noncatalytic scaffolding proteins. The scaffolding proteins have been identified as CipA (or scaffoldin) in *C. thermocellum* (Gerngross *et al.,* 1993), CipC in *C. cellulolyticum* (Pages *et al.,* 1999), CbpA in C. *cellulovorans* (Shoseyov *et al.,* 1992), and CipA in *C. josui* (Kakiuchi *et al.,* 1998). These proteins fundamentally consist of repetitive noncatalytic domains of about 140 residues, termed cohesin domains, and a carbohydrate-binding module (CBM). For example, *C. josui* CipA N-terminus is composed of 3 CBMs followed by a hydrophilic domain and six cohesin domains; and *C. thermocellum* CipA contains a CBM between the second and third of nine repeated cohesin domains and a type II dockerin domain at its C terminus. The amino acid sequences of all the cohesin domains from these bacteria are in many cases highly homologous to each other, especially within the same species. Each cohesin domain is a subunit-binding domain that interacts with a docking domain, called dockerin, of each catalytic component. The dockerin domain contains two segments, also known as conserved duplicated regions (CDRs), each of which contains a $Ca^{2+}$-binding loop and an alpha helix (namely, the calcium binding motif). An additional alpha helix intervenes between the two segments. The alpha helix in each duplicated sequence contains a conserved KR or KK dipeptide. The species-specific attachment of the dockerin module to the cohesin module is mediated via a high affinity $Ca^{2+}$ -dependent interaction.

The *C. thermocellum* cellulosome contains a CBM and a series of nine cohesin modules that anchor the cellulosomal enzymes to the multienzyme complex (Bayer *et al*., 2004). The various cellulosomal enzymes contain *inter alia* a conserved dockerin module that binds to the cohesin counterpart. Biochemical and structural studies on the cohesins-dockerin interaction from *C. thermocellum* scaffoldin have shown that the dockerins can bind to each of the cohesins on the scaffoldin (Yaron *et al*., 1995) with a strong affinity constant (Mechaly *et al*., 2000). Binding is typically reversible by addition of divalent ion chelators such as EDTA.

CBMs, which bind to cellulose or chitin, have been utilized in affinity purification. Craig *et al.,* 2006 have described an affinity chromatography system based upon the calcium dependence of the cohesin- dockerin interaction for purification of antibodies. In this system, a CBM- Coh from *C. thermocellum* was coupled covalently to an activated Sepharose matrix, following which an antibody- binding protein fused to a dockerin module was introduced to the column, and the protein, was eluted with EDTA. Due to the strong cohesin- dockerin interaction, however, the efficiency of the elution step and suitability for repeated use in such studies are unclear.

Fierobe 1999 discloses that the dockerin domain of *C. cellulolyticum* CipC is sufficient for interaction with cohesin, that removal of the second conserved duplicated region abolishes affinity with cohesin1, and that deletion of the linker sequence immediately preceding the dockerin domain disrupts folding of the domain and sharply reduces affinity for cohesin. Thus, this reference teaches against use of a dockerin domain on the extreme N- or C-terminus of any protein.

Karpol *et al*., 2008 discloses a series of deletions in the N- and/or C- terminal portions of a *C. thermocellum* dockerin module fused to a xylanase. The mutated dockerins having deletions in the calcium-coordinating residues exhibited efficient binding to cohesin, which was abolished by EDTA.

US Patent Application No. 2005/0106700 discloses use of C-terminal and N-terminal dockerin fusions in purification of target proteins on affinity columns. International Patent Application No. WO 2009/028532 discloses purification systems and methods using a dockerin polypeptide characterized in that the amino acid at the 14-position in the second sub-domain of a dockerin originating from *Clostridium josui* is substituted by another amino acid.

Karpol et al. investigated the asymmetry of cohesion-dockerin binding and its insight into cellulosome assembly, *inter alia*, by progressively truncating xylanase-fused dockerin derivatives (Karpol et al. Biochem. J. (2008) 410, 331-8)

EP- A- 1441030 discloses a method of purifying a recombinant fused protein characterized in that a recombinant fused protein, wherein a target protein has been fused with dockerin by genetic engineering techniques, is treated with a support having a cohesion domain immobilized thereon; and a method of producing a target protein characterized by comprising obtaining a recombinant fused protein having the target protein bound to dockerin with the use of an expression vector having a gene encoding the target protein and the dockerin inserted thereto, subsequently purifying the recombinant fused protein by binding to a support having a cohesion domain immobilized thereon, and then eliminating the dockerin from the recombinant fused protein.

None of the prior publications teach or suggest use of the truncated dockerin domains of the present invention in affinity columns or related technologies, their attachment to the N- or C-terminus of a target protein, or the advantageous reversible binding attained thereby.

There remains a need for a cost-effective affinity tag for use in affinity purification systems. Additionally, there remains a need for a purification process capable of obtaining high yields and high purity of protein concentrate without reducing the specific activity of the isolated proteins.

## SUMMARY OF THE INVENTION

The present invention is directed to affinity purification systems using truncated dockerin polypeptides, the truncated dockerin comprising a single calcium binding motif. The present invention further provides recombinant polypeptides comprising the mutated dockerin domain and affinity columns comprising the truncated dockerin polypeptides, methods of generating same, and methods of use thereof to purify, isolate, and detect molecules of interest. It should be appreciated that the molecule of interest may be any type of molecule which it is desirable to purify. According to certain embodiments the molecule of interest is covalently bound to the truncated dockerin domain chemically or in case the molecule of interest is a peptide it may be fused to the truncated dockerin domain to form a recombinant polypeptide.

It is now disclosed for the first time that the mutated dockerin domain is advantageous for use in a cohesin-dockerin purification system due to the ability of the CBM-cohesin polypeptide to bind tenaciously to a cellulose matrix. Furthermore, the system affords facile, cost-effective and efficient regeneration of the column for repeated use.

According to the principles of the present invention a truncated dockerin domain, comprising only one of the two $Ca^{2+}$ binding motifs, exhibited high binding capacities to a cohesin domain that was reduced compared to that of the wild type dockerins. The wild-type dockerin was shown to bind cohesin with an extremely tight association, and thus was found to be unsuitable for affinity chromatography systems. Unexpectedly, truncation of the dockerin domain conferred reversible binding to the cohesin-dockerin system and enabled recovery of more that 90% of an exemplary covalently bound target protein. Protein recovery from columns utilizing the truncated dockerin domain was highly efficient and characterized by high levels of purity in a single step, directly from crude cell extracts. Moreover, the truncated dockerin tag had no significant effect on the activity of the purified enzyme compared to the activity of the wild-type enzyme.

Reference is made to FIG. 3A, which depicts the structure of the intact wild-type *C. thermocellum* Cel48S dockerin domain (SEQ ID NO: 1) used to design the truncated dockerin domains of the present invention. As shown, dockerin domains begin with a conserved glycine residue, which is designated residue 1 in the numbering used herein. The dockerin domain contains two segments, also known as conserved duplicated regions (CDRs), each of which contains a $Ca^{2+}$-binding loop and an alpha helix (namely, the calcium binding motif). An additional alpha helix intervenes between the two segments referred to in the figure as duplicated sequence 1 and duplicated sequence 2. Each duplicated sequence forms a single calcium binding motif. The alpha helix in each duplicated sequence of the Cel48S dockerin domain contains a conserved KR or KK dipeptide. In other dockerin domains the conserved dipeptide may consist of KR, KK, KY, KM, KN, SR, RR, or KG. According to certain embodiments, the truncated dockerin domain of the present invention lacks 14-16 amino acids N-terminal to said conserved dipeptide.

It will be apparent to those skilled in the art that based on the present disclosure, dockerin analogues of the truncated dockerin polypeptides of the present invention can be made in dockerin domains other than that of Cel48S. FIG. 11, for example, provides a sequence alignment of the two dockerin segments of multiple species (SEQ ID NO: 10-SEQ ID NO: 34; starting from the -3 position, according the numbering utilized herein). FIG. 12 (SEQ ID NO:35- SEQ ID NO: 122) further provides a sequence alignment of the dockerin domains from *C. thermocellum.* According to certain embodiments, the *C. thermocellum* Type-I dockerin domain has the amino acid sequence as set forth in any one of SEQ ID NO:35 - SEQ ID NO: 122, or an analog thereof having at least 70% homology to SEQ. ID No:1. According to certain embodiments, the dockerin domain is from a thermophilic microorganism. According to certain embodiments the thermophilic microorganism is selected from a group consisting of *Clostridium thermocellum* and *Archaeoglobus fulgidus.* Each possibility represents a separate embodiment of the present invention.

According to one aspect, the present invention provides an affinity purification system comprising a solid substrate, a bound protein comprising a cohesin domain, and a recombinant polypeptide comprising a molecule of interest and a truncated dockerin polypeptide derived from a dockerin domain, the truncated dockerin comprising only one calcium binding motif.

In some embodiments, said molecule of interest is a molecule other than a protein. In other embodiments the molecule of interest is a peptide or polypeptide. In another embodiment, said molecule of interest is selected from the group consisting of a peptide, an enzyme, a hormone and an antibody. In another embodiment, said molecule of interest is an enzyme other than xylanase. Each possibility represents a separate embodiment of the present invention.

In another embodiment, said molecule of interest is an antibody-binding moiety, and said affinity purification system further comprises the at least the antigen binding portion of an antibody bound to the antibody-binding moiety. According to certain embodiments, an antibody-binding moiety is attached to an affinity column of the present invention via fusion of the antibody-binding moiety to a truncated dockerin polypeptide. The truncated dockerin polypeptide is preferably able to reversibly attach to a cohesin-containing protein bound to the affinity column. Preferably, the antibody-binding moiety is selected from the group consisting of an anti-IgG antibody, protein A, protein G, and protein L. The affinity column can thus be used as a column for purifying a ligand recognized by the bound antibody. In another embodiment said molecule of interest is a ligand, wherein the affinity column can thus be used as a column for binding and/or purifying antibodies that bind specifically to the ligand of choice. Each possibility represents a separate embodiment of the present invention.

In one embodiment, the solid substrate is selected from the group consisting of a bead, a cell, an extracellular matrix, and a container. In another embodiment, the solid substrate is an affinity resin. In another embodiment, the solid substrate is an affinity column. In another embodiment, an affinity column of methods and compositions of the present invention comprises cellulose, and the protein bound to the affinity column further comprises a carbohydrate-binding module (CBM). In another embodiment, the means of attachment of the protein to the affinity column is via interaction between the CBM and the cellulose. In another embodiment, the dockerin domain is a Type-I dockerin domain. In another embodiment, said truncated dockerin polypeptide contains a 14-16 amino acid deletion N-terrminal to the lysine residue at position 18 of the wild-type Type I dockerin domain sequence. In another embodiment, said truncated dockerin polypeptide further comprises an N-terminal glycine residue, wherein the glycine residue is attached directly to the truncated dockerin polypeptide. In another embodiment, the cohesin domain on the bound protein is a Type I cohesin domain, capable of interacting with the truncated dockerin domain attached to the molecule of interest. Each possibility represents a separate embodiment of the present invention.

According to a further aspect, the present invention provides a recombinant or synthetic polypeptide comprising a molecule of interest and a truncated dockerin polypeptide derived from a dockerin domain, wherein the truncated dockerin polypeptide comprises only one calcium binding motif, wherein said truncated dockerin polypeptide contains a 14-16 amino acid deletion N-terminal to the lysine residue at position 18 or at position 50 of the wild-type Type I dockerin domain sequence. According to certain embodiments, the dockerin domain is a Type-I dockerin domain. According to some embodiments, the Type-I dockerin domain has the amino acid sequence as set forth in any one of SEQ ID NO:1, SEQ ID NO:35- SEQ ID NO:122, or an analog thereof comprising at least 70% homology to SEQ ID NO:1. Each possibility represents a separate embodiment of the present invention.

In some embodiments, said molecule of interest is a molecule other than a protein. In other embodiments, said molecule of interest is selected from the group consisting of a peptide, an enzyme, a hormone and an antibody. In another embodiment, said molecule of interest is a molecule other than xylanase. Each possibility represents a separate embodiment of the present invention.

In another embodiment, the truncated dockerin polypeptide is linked to the N-terminus of the peptide. In another embodiment, the molecule of interest is a peptide, said truncated dockerin polypeptide is linked to the C-terminus of said peptide. In another embodiment, said truncated dockerin polypeptide is linked to said molecule of interest via a peptide bond. In another embodiment, said truncated dockerin polypeptide is linked to said molecule of interest via a linker peptide. In another embodiment, the linker peptide is a cleavable linker peptide. The cleavable linker peptide may be self-cleavable. Each possibility represents a separate embodiment of the present invention.

In another embodiment, said molecule of interest is an enzyme. Said molecule of interest may be an antibody-binding moiety bound to an antibody. Preferably, the antibody-binding moiety is selected from the group consisting of an anti-IgG antibody, protein A, protein G, and protein L.

According to a further aspect, the present invention provides a method of attaching a molecule of interest to a solid substrate, the method comprising the step of: a) providing a solid substrate associated with a protein comprising a cohesin domain; b) providing a molecule of interest covalently bound to a truncated dockerin polypeptide, wherein the truncated dockerin polypeptide comprises only one calcium binding motif; c) allowing the truncated dockerin molecule to bind to the cohesin domain; thereby attaching a molecule of interest to a solid substrate. In one embodiment, the molecule of interest is a fusion peptide. Said molecule of interest may be a molecule other than a peptide. Said molecule of interest may be a molecule other than xylanase.

The step of attaching a molecule of interest to a solid substrate is performed in the presence of $Ca^{2+}$. As provided herein, methods of the present invention enable attachment of proteins of solid substrates that is readily reversible under non-denaturing conditions. In one embodiment, said solid substrate is selected from the group consisting of a bead, a cell, an extracellular matrix, and a container. In another embodiment, said solid substrate comprises cellulose and said protein associated with said solid substrate further comprises a carbohydrate-binding module (CBM). The cohesin domain may be a Type I cohesin domain. The dockerin domain may be a Type I dockerin domain. Said truncated dockerin polypeptide may contain a 14-16 amino acid deletion N-terminal to the lysine residue at position 18 of the wild-type Type I dockerin domain sequence. Said truncated dockerin polypeptide may further comprise an N-terminal glycine residue, wherein the glycine residue is attached directly to the truncated dockerin polypeptide.

According to another aspect, the present invention provides a method of purifying a molecule of interest, the method comprising the steps of: a) providing a solid substrate associated with a protein comprising a cohesin domain; b) providing a molecule of interest covalently bound to a truncated dockerin polypeptide, wherein the truncated dockerin polypeptide comprises only one calcium binding motif; c) allowing the truncated dockerin molecule to bind to the cohesin domain; and d) eluting the molecule of interest of (b); thereby purifying a molecule of interest.

In one embodiment, the dockerin domain is a Type I dockerin domain. Said truncated dockerin polypeptide may contain a 14-16 amino acid deletion N-terminal to the lysine residue at position 18 of the wild-type Type I dockerin domain sequence. Said truncated dockerin polypeptide may further comprise an N-terminal glycine residue, wherein the glycine residue is attached directly to the truncated dockerin polypeptide. In one embodiment, the cohesin domain is a Type I cohesin domain. In another embodiment, said solid substrate is selected from the group consisting of a bead, a cell, an extracellular matrix, and a container. In another embodiment, said solid substrate comprises cellulose and said protein associated with said solid substrate further comprises a carbohydrate-binding module.

In another embodiment, the step of attaching a molecule of interest to a solid substrate is performed in the presence of $Ca^{2+}$. In another embodiment, the step of eluting the molecule of interest is performed with a chelator of a divalent cation. In another embodiment, the chelator is selected from the group consisting of EDTA and EGTA. Each possibility represents a separate embodiment of the present invention.

Said molecule of interest may be a molecule other than a peptide. Said molecule of interest may be a molecule other than xylanase. In one embodiment, the molecule of interest is a fusion peptide. In another embodiment, the molecule of interest is fused to the truncated dockerin polypeptide via a cleavable linker peptide, the method further comprising the step of cleaving said cleavable linker peptide. Said cleavable linker peptide may be self-cleavable.

The present invention may provide a method of purifying a molecule of interest, the method comprising the steps of: a) providing a solid substrate associated with a protein comprising a cohesin domain; b) providing a truncated dockerin polypeptide covalently bound to an antibody-binding domain bound to an antibody, the antibody recognizes the molecule of interest, wherein the truncated dockerin polypeptide comprises only one calcium binding motif; c) allowing the truncated dockerin molecule to bind to the cohesin domain; and d) eluting the molecule of interest; thereby purifying a molecule of interest.

The antibody-binding moiety may be selected from the group consisting of an anti-IgG antibody, protein A, a protein G, and a protein L.

A method of purifying a molecule of interest may be provided, the method comprising the steps of: (a) contacting a solid substrate with the molecule of interest; and (b) eluting the molecule of interest; wherein the solid substrate comprises (i) a first protein bound thereto, wherein the first protein comprises a cohesin domain; and (ii) a second protein or domain fused to the truncated dockerin polypeptide of the present invention, wherein the second protein or domain recognizes the molecule of interest, thereby purifying a molecule of interest. A method of engineering a molecule of interest to be readily purified using a solid substrate may be provided, the method comprising the step of fusing the molecule of interest to a truncated dockerin polypeptide, the truncated dockerin polypeptide is derived from a dockerin domain and comprises only one calcium binding motif, thereby engineering a molecule of interest to be purified using an affinity column.

The dockerin domain may be a Type-I dockerin domain. The molecule of interest may be a peptide. The truncated dockerin polypeptide of methods of the present invention may be linked to the C-terminus of the peptide. The truncated dockerin polypeptide of methods may be linked to the N-terminus of said peptide.

According to another aspect, the present invention provides an isolated truncated dockerin polypeptide derived from a dockerin domain, wherein the truncated dockerin polypeptide comprises only one calcium binding motif, wherein said truncated dockerin polypeptide contains a 14-16 amino acid deletion N-terminal to the lysine residue at position 18 or at position 50 of the wild-type Type I dockerin domain sequence The isolated truncated polypeptide may comprise from about 35 to about 70 amino acids. The isolated polypeptide may comprise from about 40 to about 55 amino acids.

According to some embodiments, the Type-I dockerin domain has the amino acid sequence as set forth in SEQ ID NO: 1 (GDVNDDGKVNSTDAVALKRYVLRSGISINTDNADLNEDGRVNSTDLGILKRYIL KEIDTLPYKN), or an analog thereof having at least 70% homology to SEQ ID NO: 1.

According to certain embodiments, the truncated dockerin polypeptide of the present invention comprises only one calcium binding motif, wherein the retained calcium binding motif is in the first segment of the dockerin domain. According to this embodiment, said truncation comprises the calcium-coordinating residues in the second segment of the dockerin domain. According to certain embodiments, the truncated dockerin polypeptide comprises the amino acid sequence as set forth in SEQ ID NO: 4. Each possibility represents a separate embodiment of the present invention.

According to other embodiments, the retained calcium binding motif of the isolated polypeptide is in the second segment of the dockerin domain. According to this embodiment, said truncation comprises the calcium-coordinating residues in the first segment of the dockerin domain. According to certain embodiments, the truncated dockerin polypeptide comprises the amino acid sequence as set forth in any one of SEQ ID NO: 2, SEQ ID NO: 3 and SEQ ID NO: 6. According to one embodiment, the truncated dockerin polypeptide consists of the amino acid sequence as set forth in SEQ ID NO: 2. According to another embodiment, the truncated dockerin polypeptide consists of the amino acid sequence as set forth in SEQ ID NO: 3. Each possibility represents a separate embodiment of the present invention.

An isolated polynucleotide sequence may be provided encoding the truncated dockerin polypeptide of the present invention. An expression vector may be provided comprising the isolated polynucleotide sequence encoding the truncated dockerin polypeptide of the present invention. A host cell may be provided comprising the expression vector.

Further embodiments and the full scope of applicability of the present invention will become apparent from the detailed description given hereinafter. However, it should be understood that the detailed description and specific examples, while indicating preferred embodiments of the invention, are given by way of illustration only.

## BRIEF DESCRIPTION OF THE FIGURES

**FIG. 1.** Schematic representation of the cohesin-dockerin-based affinity purification approach. CBM-Coh is first bound to the beaded cellulose resin. The dockerin-bearing target protein is then applied and eluted subsequently using increasing concentrations of EDTA.

**FIG. 2.** Affinity purification of wild-type Doc-Xyn on a CBM-Coh affinity column. **(A)** Performance of the affinity column. **(B)** SDS-PAGE analysis of the chromatogram, Cell lysate and CBM-Coh before and after the elution.

**FIG. 3.** Progressive truncation of dockerin derivatives borne at the N- terminus of the target protein. **(A)** Sequences of the wild type dockerin domain (SEQ ID NO: 1) and the truncated dockerin derivatives. **(B)** Comparative binding of the tructated dockerins to cohesin. **(C)** Changes in binding free energies ($\Delta\Delta G$) upon truncation, calculated as the ratio between the wild type and the mutant dockerins. **(D)** Comparison of eluted His- tagged Doc ($\Delta$16)- Xyn, purified using either IMAC or on a CBM- Coh column (Coh- Doc) . **(E)** Calcium- dependent binding properties of the truncated and the wild type Doc- Xyn measured using the ELISA- based assay. (■) and (▼) indicate wtDoc-Xyn supplemented with 1 mM CaC12 or 10 mM EDTA, respectively. (▲) and (♦) indicate Doc (Δ16)- Xyn supplemented with 1 mM CaCl2 or 10 mM EDTA, respectively.

**FIG. 4.** Affinity purification of truncated Doc (Δ16)- Xyn on a CBM- Coh affinity column. **(A)** Performance of the affinity column through repeated application (Ap) and elution (El) of the target protein. **(B)** SDS- PAGE analysis of the chromatogram. The protein profiles of the crude cell lysate and the CBM- Coh are also shown.

**FIG. 5. (A)** Determination of relative binding affinity by competitive ELISA. Microtiter plates were coated with a CBM- Coh and interacted with either wtDoc- GFP (■) or Doc (Δ16)- GFP (▲) in the presence of competitor wtDoc-Xyn. wtDoc- Xyn was used as a control (▼) . Measured OD values reflect the relative amount of wtDoc Xyn bound to the coating cohesin. **(B)** Repeated purification of Doc (Δ16)- GFP on a CBM- Coh affinity column. **(C 1- 3)** Consecutive elution fractions were analyzed by SDS- PAGE in order to evaluate protein purity. **(D)** Evaluation of the flow- through fraction after consecutive applications. **(E)** Boiled column beads after final wash. **(F)** SDS- PAGE analysis of Doc (Δ16)- GFP fusion protein purified on Ni- NTA. Gel visualization was done using coomassie brilliant blue staining.

**FIG. 6.** SDS-PAGE analysis of wtDoc-GFP fusion protein purified on CBM-Coh or Ni-NTA column. **(A)** Demonstration of the elution fraction by applying increasing amount of EDTA, and after boiling the column beads with SDS. The flow-through of the unbound cell lysate is shown as well. **(B)** Ni-NTA elution fractions.

**FIG. 7.** SDS- PAGE analysis of GFP with C- terminal ΔDoc or wtDoc affinity tag, purified on CBM- Coh. **(A- B)** Two consecutive elutions of GFP- Doc (Δ16) . **(C)** GFP- wtDoc elution followed by boiling the column beads in order to release attached proteins.

**FIG. 8.** Doc (Δ16)- ZZ- domain purification and binding activity. **(A)** SDS PAGE analysis of the CBM- Coh purified Doc (Δ16)- ZZ domain. **(B)** SDS PAGE analysis of the purified antibodies using immobilized Doc (Δ16)- ZZ.

**FIG. 9.** Doc (Δ16)- BglA purification and activity assessment. **(A)** SDS PAGE analysis of the CBM- Coh purified Doc (Δ16)- BglA. **(B)** Doc (Δ16)- BglA activity curve. **(C)** wild- type BglA activity curve

**FIG. 10.** Doc (Δ16)- TEP1 purification and activity assessment. **(A)** SDS- PAGE analysis of the CBM- Coh purified Doc (Δ16)- TEP1. **(B)** ΔDoc- TEP1 activity curve.

**FIG. 11.** Alignment of the amino acid sequences of dockerin domains of Aga27A, Cel8A and Cel48A of *C. josui* (Cj); and Xyn11A Xyn11B, Xyn10C, Cel5A, Cel8A, Cel9A, Cel26A-Cel5E (formerly CelH), Cel9D-Cel44A, Cel48A, and Lic16A (formerly LicB) of C. *thermocellum* (Ct). Asterisks indicate amino acid residue involved in calcium binding. Residues believed to serve as selectivity determinants are indicated by pound signs (#). Amino acids that have conserved similar chemical properties (I, L, M, V, K, R, S, and T) are presented in white on black or black on gray (Jindou, S. *et al.,* 2004). **(A)** Sequence alignment of the first dockerin segment. **(B)** Sequence alignment of the second dockerin segment.

**FIG. 12.** Sequence alignment of dockerin modules from *Clostridium thermocellum.*

## DETAILED DESCRIPTION OF THE INVENTION

The present invention is directed to truncated dockerin polypeptides, recombinant polypeptides and affinity columns comprising the truncated dockerin polypeptide, methods of generating same, and methods of using same to purify, isolate, and detect molecules of interest.

As exemplified herein below, a truncated dockerin polypeptide lacking one of the two $Ca^{2+}$ binding motifs retained relatively high binding capacities to a cohesion domain. The truncated dockerin polypeptide functioned as an effective affinity tag, and highly purified target proteins were obtained in a single step directly from crude cell extracts. Furthermore, the truncated dockerin affinity tag had no significant effect on the activity of the purified enzyme compared to the activity of the wild-type enzyme.

As disclosed herein below, the affinity column maintained high levels of capacity upon repeated rounds of loading and elution. Further, the coupling of the CBM-Cohesin to the matrix was not achieved by chemical activation of the protein, but by the advantageously innate property of the CBM to bind tenaciously to an inexpensive cellulose matrix.

According to one aspect, the present invention provides an affinity purification system, comprising a solid substrate, a bound protein comprising a cohesin domain and a recombinant or synthetic polypeptide comprising a molecule of interest and a truncated dockerin polypeptide derived from a dockerin domain, wherein the truncated dockerin polypeptide comprises only one calcium binding motif. Reference is made to FIG. 3A, which depicts the domain structure of the wild-type dockerin domains used to design the truncated dockerin polypeptides of the present invention. As shown, wild-type dockerin domains begin with a conserved glycine residue, which is designated residue 1 in the numbering used herein. The dockerin domains contain two segments, also known as conserved duplicated regions (CDRs), each of which contains a $Ca^{2+}$-binding loop and an alpha helix. An additional alpha helix intervenes between the two segments referred to in the figure as duplicated sequence 1 and duplicated sequence 2. Each duplicated sequence forms a single calcium binding motif. The alpha helix in each duplicated sequence contains a conserved KR or KK dipeptide. In other dockerin domains the conserved dipeptide may consist of KR, KK, KY, KM, KN, SR, RR or KG. It is exemplified herein below that deletions in the first or second segment of the dockerin domain confer highly advantageous properties in affinity purification systems. The terms "truncated dockerin" and "mutated dockerin domain" are used herein interchangeably and refer to any deletion removing one of the two calcium binding motifs, to provide a dockerin polypeptide comprising only one calcium binding motif.

"Deletion N-terminal to the lysine residue at position 18" may denote a deletion wherein 14-16 amino acids between residues 1-17 inclusive are removed. The 14-16 amino acids deletion may comprise the calcium-coordinating residues in the first segment of the dockerin domain. The calcium-coordinating residues in the second segment of the dockerin domain may be un-mutated. A truncated dockerin polypeptide of methods and compositions may begin with residue 17 of the Type I dockerin domain. The truncated dockerin polypeptide begins with residue 16 of the Type I dockerin domain. The truncated dockerin polypeptide may begin with residue 15 of the Type I dockerin domain. A truncated dockerin polypeptide may be defined as a dockerin domain containing a deletion of 14-16 amino acids between residues 2-17 thereof, inclusive. The deletion may be 15-16 amino acids. The deletion may be 16 amino acids.

The truncated dockerin of methods and compositions may comprise a 14-16 amino acid deletion N-terminal to the lysine residue at position 50 of the wild-type Type I dockerin domain sequence. "Deletion N-terminal to the lysine residue at position 50" is used herein to denote a deletion wherein 14-16 amino acids between residues 34-49 inclusive are removed. Said 14-16 amino acids deletion may comprise the calcium-coordinating residues in the second segment of the dockerin domain. The calcium-coordinating residues in the first segment of the dockerin domain may be un-mutated. A truncated dockerin polypeptide of methods and compositions may begin with residue 1 of the Type I dockerin domain. The truncated dockerin domain may begin with residue 2 of the Type I dockerin domain. A truncated dockerin polypeptide may be defined as a dockerin domain containing a deletion of 14-16 amino acids between residues 34-49 thereof, inclusive. The deletion may be 15-16 amino acids. The deletion may be 16 amino acids.

The wild-type Type I dockerin domain may have an amino acid sequence as set forth in SEQ ID NO: 1 (GDVNDDGKVNSTDAVALKRYVLRSGISINTDNADLNEDGRVNSTDLGILKRYIL KEIDTLPYKN); or an analog having at least 70% sequence homology to SEQ ID NO: 1. It will be apparent to those skilled in the art that dockerin deletions analogous to the dockerin deletions can be made in dockerin proteins other than Cel48S. FIG. 11 (SEQ ID NO:10 - SEQ ID NO:34), for example, provides an alignment of the N-terminal approximately 35 amino acids of dockerin domains of multiple species (starting from the -3 position, according the numbering utilized herein). The wild-type Type I dockerin domain may have an amino acid sequence as set forth in any one of SEQ ID NO: 35 - SEQ ID NO: 122. FIG. 12 depicts an alignment of additional dockerin domains, wherein the KR or KK dipeptide in the first segment of the dockerin domain is clearly delineated. A dipeptide selected from the group consisting of KN, KK, KM, and KG may be present instead of KR or KK. The lysine residue in this dipeptide is used instead of the first lysine in KR or KK in designing the truncated dockerin polypeptide. A dipeptide selected from the group consisting of SR, RR, and NR may be present instead of KR or KK. The first residue in this dipeptide is used instead of the first lysine in KR or KK in designing the truncated dockerin polypeptide. Corresponding deletions in other dockerin proteins (namely, 14-16 amino acid deletions N-terminal to but not including the KR, KK, KY, KM, KN, SR, RR, or KG dipeptide in the first or second segments of the dockerin domain) can thus readily be made. The deletion may be 14-16 amino acids, alternatively 15-16 amino acids, further alternatively 16 amino acids between but not including the conserved glycine and the KR, KK, KY, KM, KN, SR, RR, or KG dipeptide in the first segment of the dockerin domain. The deletion may be 15-17 amino acids, alternatively 16-17 amino acids, further alternatively 17 amino acids up to but not including the KR, KK, KY, KM, KN, SR, RR, or KG dipeptide in the first segment of the dockerin domain. The deletion may be 15-17 amino acids, alternatively 16-17 amino acids, further alternatively 17 amino acids up to but not including the KR, KK, KY, KM, KN, SR, RR, or KG dipeptide in the second segment of the dockerin domain.

According to a further aspect a recombinant or synthetic polypeptide may be provided comprising a molecule of interest covalently bound to a truncated dockerin polypeptide derived from a dockerin protein domain, the truncated dockerin polypeptide comprising only one calcium binding motif.

It will be apparent to those skilled in the art that the truncated dockerin domain may be linked to molecule of interest via a direct covalent bond or via a suitable linker. The molecule of interest may be a peptide or polypeptide and the truncated dockerin domain may be linked conveniently to either the N-terminus or the C-terminus, for example via a peptide bond. "Linked to" as utilized herein, refers to connection of two entities via a covalent bond. Linkages between peptide moieties may be via one or more peptide bonds within a polypeptide chain. The term encompasses embodiments wherein a linker peptide is present and wherein the molecule of interest is directly linked via a single peptide bond to a truncated dockerin domain. It will be understood by those of skill in the art that such linkage can be performed by engineering a nucleotide molecule to encode a fusion peptide or by chemical or other means of directly attaching peptides to one another. The linkage may be performed synthetically to yield non-peptide bonds.

A recombinant peptide may further comprise an N-terminal glycine residue attached directly to a truncated dockerin domain. "Attached directly" as used herein refers to a lack of intervening sequence between the N-terminal glycine residue and the C-terminal dockerin fragment. Truncated dockerin polypeptides thus may typically consist of the combination of an N-terminal glycine residue and a C-terminal dockerin domain fragment, wherein the glycine residue is attached directly to the dockerin domain fragment without an intervening, or linker, peptide. No N-terminal glycine residue may be also present.

"C- terminal Type I dockerin domain fragment" refers to a dockerin fragment that extends until the end of the wild- type dockerin domain. The C- terminus of the dockerin domain is often considered to be the C- terminus of the second segment of said dockerin domain thereof of the wild- type sequence. In the case of a dockerin domain located on the C- terminus of a protein of interest, the C- terminus of the dockerin domain may be considered to be the C- terminus of the molecule of interest. To illustrate this, the C- terminus of the truncated dockerin polypeptide utilized in the Examples, KRYVLRSGISINTDNADLNEDGRVNSTDLGILKRYILKEIDTLPYKN (SEQ ID NO: 5), is the C- terminus of the wild- type sequence SEQ ID NO: 1, which is also the C- terminus of the wild- type Cel48S protein (SEQ ID NO: 7; GenBank Accession No. L06942) . A C- terminal dockerin domain fragment extends at least until the conserved isoleucine residue occurring shortly after the alpha helix of the second segment of said dockerin domain. This conserved isoleucine residue is the first isoleucine residue after the alpha helix of the second segment and typically is located about two residues thereafter (residue 57 according to the numbering used herein) . In the case of the truncated dockerin domain utilized in the Examples, such a peptide would comprise the sequence GKRYVLRSGISINTDNADLNEDGRVNSTDLGILKRYILKEI (SEQ ID NO: 3) . A C- terminal Type I dockerin domain fragment extends until the end of a sequence selected from SEQ ID NO: 35- SEQ ID NO: 122 (FIG. 12) . Additional C- terminal residues from the wild- type dockerin domain sequence are included.

It is to be understood that affinity purification systems and methods for purifying a molecule of interest may be comprised utilizing the novel reversible interaction between a cohesion domain and a truncated dockerin polypeptide. Thus, affinity purification system may comprise, A recombinant polypeptide comprising a molecule of interest and a cohesion domain, and a bound protein comprising a truncated dockerin polypeptide. A method for purifying a molecule of interest may be provided, the method may comprise the steps of (a) contacting a solid substrate with a molecule of interest and (b) eluting said molecule of interest, wherein the solid substrate is associated with a protein comprising a truncated dockerin polypeptide , and the molecule of interest has been fused to cohesion domain.

**Affinity column apparatus**

Further an affinity column apparatus may be provided comprising an affinity column, a recombinant polypeptide and a bound protein comprising a cohesin domain. Preferably, the cohesin domain on the bound protein is capable of interacting with the recombinant polypeptide comprising a truncated dockerin polypeptide attached to the molecule of interest.

An affinity column may comprise cellulose, and the protein bound to the affinity column may further comprise a carbohydrate-binding module (CBM). The means of attachment of the protein to the affinity column is via interaction between the CBM and the cellulose.

An antibody-binding moiety may be attached to an affinity column via fusion of the antibody-binding moiety to a truncated dockerin polypeptide. The truncated dockerin polypeptide is preferably able to reversibly attach to a cohesin-containing protein bound to the affinity column. The antibody-binding moiety may be selected from the group consisting of an anti-IgG antibody, protein A, a protein G, and a protein L. The affinity column apparatus may further comprise an antibody that binds to the antibody-binding moiety. The affinity column can thus be used as a column for a ligand recognized by the bound antibody.

**Solid substrates useful in the present invention**

The solid substrate of methods and compositions may be a bead. The solid substrate may be a cell. The solid substrate may be an extracellular matrix. The solid substrate may be a fibrous matrix. The solid substrate may be a container. The container may be selected from the group consisting of a beaker, a flask, a cylinder, a test tube, a centrifugation tube, Petri dish, a culture dish and a multi-well plate. The solid substrate may be attached to or associated with an affinity column.

An antibody-binding moiety may be attached to a solid substrate via fusion of the antibody-binding moiety to a truncated dockerin polypeptide. The truncated dockerin polypeptide is able to reversibly attach to a cohesin-containing protein bound to the solid substrate. The antibody-binding moiety may be selected from the group consisting of an anti-IgG antibody, protein A, a protein G, and a protein L. The solid substrate apparatus may further comprise an antibody that binds to the antibody-binding moiety. The solid substrate can thus be used to immobilize or isolate a ligand recognized by the bound antibody.

A solid substrate of methods and compositions may comprise cellulose, and the protein bound to the solid substrate may further comprise a carbohydrate-binding module (CBM). The means of attachment of the protein to the solid substrate may be via interaction between the CBM and the cellulose.

### Molecules of interest that can be attached to truncated dockerin polypeptides

The molecule of interest of the methods and compositions may be any molecule that can be bound covalently, either directly or indirectly, to the truncated dockerin domain containing a single calcium binding motif as disclosed herein. The molecule of interest may be any type of molecule which it is desirable to purify or for which it is desirable to engineer an association with a solid substrate.

The molecule of interest may be a peptide. The molecule of interest may be a protein. The peptide may be an enzyme. The molecule may be a peptide hormone. The molecule may be a recombinant peptide. The molecule of interest may be any other type of peptide for which it is desirable to purify or to engineer an association with a solid substrate.

As provided herein, a variety of proteins can be successfully purified with high-efficiency under gentle conditions following fusion to truncated dockerin domains. As exemplified herein below, Xylanase (Xyn); green fluorescent protein (GFP), β-glucosidase, *BglA* and ZZ-domain have been successfully purified; maltose-binding protein (MBP), TEP and CprA have been utilized with equally successful results. It is to be understood that the fusion peptides of methods and compositions are not those fusion peptides that were disclosed to in Karpol *et al*., 2008.

### Elution steps

A method may further comprise the step of eluting the polypeptide comprising the molecule of interest or antibody-binding moiety from the affinity column. The step of eluting may be performed with a chelator of a divalent cation. As provided herein, truncated dockerin polypeptides are particularly suitable to efficient elution with divalent cations. A chelator of $Ca^{2+}$ may be utilized. The chelator may be selected from the group consisting of EDTA and EGTA. It should be understood that methods may further comprise one or more washing steps.

### Truncated dockerin polypeptides and wild-type dockerin domains useful in the design thereof

A dockerin domain of methods and compositions is, A mutated version of a Type I dockerin domain from a species selected from the group consisting of *Clostridium thermocellum, C. cellulolyticum,* and *C. cellulovorans.* The dockerin domain may be from a species selected from the group consisting of *Clostridium thermocellum, C. cellulolyticum, C. cellulovorans, C. papyrosolvens, C. josui, Acetivibrio cellulolyticus, Bacteroides cellulosolvens, R. flavefaciens, Ruminococcus albus,* and *Clostridium cellobioparum.*

The dockerin domain of methods and compositions may be from an *Archaeoglobus fulgidus* protein. An example of an *Archaeoglobus fulgidus* dockerin domain is the domain sequence set forth in GenBank Accession number NP_071198 (Bayer *et al.,* 1999).

The dockerin domain of methods and compositions may be from a thermophilic microbe. The thermophilic microbe may be selected from the group consisting of *C. thermocellum* and *Archaeoglobus fulgidus.*

One of the *C. thermocellum* dockerins as set forth in any one of SEQ ID NO: 35-SEQ ID NO: 122 (listed in FIG. 12 herein) may be utilized to design a truncated dockerin domain.

A cellulosomal dockerin domain may be utilized to design a truncated dockerin domain. A Type I dockerin domain from a cellulosomal protein may be used. A listing of Type I dockerin domains from cellulosomal proteins is provided in Gold and Martin, 2007. The sequences in this table are available from the United States Department of Energy Joint Genome Institute. The sequence of the complete genome is available as GenBank Accession Number CP000568.

The dockerin utilized to design the truncated dockerin polypeptide may be a dockerin disclosed in Table 1 of Zverlov VV *et al.,* 2005. Each of the sequences in this table is available from the United States Department of Energy Joint Genome Institute. The sequence of the complete genome is available as GenBank Accession Number CP000568.

A non-cellulosomal dockerin may be utilized to design a truncated dockerin domain. Non-cellulosomal dockerins are well-known and well-characterized in the art, and include, *inter alia,* glycoside hydrolases of family 2 (CpGH2), family 31 (CpGH31; GenBank Accession No. YP_695747), family 95 (CpGH95), and family 20 (GenBank Accession No. YP_696057), μ-toxin/NagH (GenBank Accession No. YP_694648), lacZ (GenBank Accession No. YP_695917), fibronectin type III domain-containing protein (GenBank Accession No. ABG82552 and YP_696557), calx-beta domain-containing protein (GenBank Accession No. ABG83106) and the dockerin domains set forth in GenBank Accession Numbers, YP_138060, AAV48354, AAG20133, YP_843398, YP_844001, ABK15364, YP_844005, AAM04927, AAM05172, YP_501678, NP_981324, AAP11768, ZP_00238756, ZP_00238791, AAO81591, YP_001089310, YP_210742, YP_269348, and ABM17463.

Methods for identification of dockerin domains are well known in the art, and are described, *inter alia*, in Zverlov *et al*., 2005. This publication describes identification of *C. thermocellum* dockerin domains; however, one of skill in the art could readily apply the same methods using dockerin domain sequences of other organisms to search the genome sequences of other organisms. A truncated dockerin polypeptide of methods and compositions may be an internally deleted version of the sequence: GDVNDDGKVNSTDAVALKRYVLRSGISINTDNADLNEDGRVNSTDLGILKRYIL KEIDTLPYKN (SEQ ID NO: 1), which is the C- terminus of the wild-type Cel48S protein (SEQ ID NO: 7; GenBank Accession No. L06942). The truncated dockerin domain is a internally deleted version of a sequence having at least 70% homology to SEQ ID NO: 1. The truncated dockerin domain may be a internally deleted version of a sequence having at least 80% homology to SEQ ID NO: 1. The truncated dockerin domain may be a internally deleted version of a sequence having at least 90% homology to SEQ ID NO: 1. The truncated dockerin domain may be a internally deleted version of a sequence having at least 92% homology to SEQ ID NO: 1. The truncated dockerin domain may be a internally deleted version of a sequence having at least 95% homology to SEQ ID NO: 1. The truncated dockerin polypeptide may be a internally deleted version of a sequence having at least 98% homology to SEQ ID NO: 1.

A truncated dockerin polypeptide of methods and compositions may have the sequence: GKRYVLRSGISINTDNADLNEDGRVNSTDLGILKRYILKEIDTLPYKN (SEQ ID NO: 2). The truncated dockerin polypeptide may have at least 70% homology to SEQ ID NO: 2. The truncated dockerin polypeptide may have at least 80% homology to SEQ ID NO: 2. The truncated dockerin polypeptide may have at least 90% homology to SEQ ID NO: 2. The truncated dockerin polypeptide may have at least 92% homology to SEQ ID NO: 2. The truncated dockerin polypeptide may have at least 95% homology to SEQ ID NO: 2. The truncated dockerin polypeptide may have at least 98% homology to SEQ ID NO: 2.

A truncated dockerin polypeptide of methods and compositions may have the sequence: MGSKRYVLRSGISINTDNAD LNEDGRVNST DLGILKRYIL KEIDTLPYKN (SEQ ID NO: 6).

It is understood in the art that a truncated dockerin polypeptide of methods and compositions may be a fragment of the aforementioned dockerin sequences. The term "fragment" as used herein refers to a portion of a polypeptide which retains the activity of the native polypeptide, i.e., use an affinity tag. The fragment may have between about 30 to about 60 amino acids, alternatively between about 35 to about 55 amino acids, further between about 40 to about 50 amino acids.

The $Ca^{2+}$-binding residues (Mechaly *et al,* 2000; also termed as "$Ca^{2+}$-coordinating residues") are preserved (i.e. are unmutated) in the second conserved duplicated region (i.e. segment) of a truncated dockerin polypeptide of methods and compositions. The DNDND motif at positions 1, 3, 5, 9, and 12 of the second conserved duplicated region is preserved. The residues DNDD motif at positions 1, 3, 5, and 12 of the second conserved duplicated region may be preserved. The $Ca^{2+}$-binding residues are preserved in the first conserved duplicated region (i.e. segment) of a truncated dockerin polypeptide of methods and compositions.

The cohesin recognition residues (Mechaly *et al*., 2000; and Pages *et al*., 1997) are preserved (i.e. are unmutated) in the second conserved duplicated region of a truncated dockerin polypeptide. Positions 10 and 11 may be preserved. The preserved residues at these positions are ST. The preserved residues at these positions are SS. The preserved residues at these positions are AL. The preserved residues at these positions are AI. The cohesin recognition residues are preserved (i.e. are unmutated) in the first conserved duplicated region of a truncated dockerin polypeptide .

**Dockerin-cohesin pairs that may be used in combination in the present invention**

The dockerin-cohesin pair utilized in methods and compositions may be from the same species. Dockerins have been shown to bind to each of the cohesins on the scaffoldin (Yaron *et al*., 1995; Pagès *et al*., 1997); thus, any cohesin from a given species is expected to bind any dockerin from that species. Cohesin-dockerin interactions are in some cases species-specific.

The dockerin and cohesin domains utilized are from two different species. As a non-limiting example, dockerin polypeptides of *C. thermocellum* Xyn11A bind to cohesin polypeptides from *C. josui* CipA (Jindou *et al*., 2004). The residues involved in determining specificity of the dockerin-cohesin have been publicized in scientific references. Methods for predicting and experimentally confirming whether a given dockerin-cohesin pair will bind to one another are well known in the art, and are described, for example, herein and in Pages *et al*., 1997; Nakar *et al*., 2004; Barak *et al.,* 2005; Haimovitz *et al*., 2008; Mechaly *et al.,* 2000; and Mechaly *et al*., 2001). As described in these references, the 11th and 12th residues of both segments, among other residues, are involved in determining the binding specificity of dockerin-cohesin pairs.

The suitability of a given dockerin-cohesin pair for affinity chromatography can be tested by performing affinity chromatography using, for example, xylanse fusion, as described herein. In such a system, the amount of interacting dockerin, indicative of the affinity of the dockerin-cohesin pair, can be determined immunochemically using anti-xylanase primary antibody and HRP-labeled secondary antibodies (Barak Y *et al*., 2005). Xylanase activity may be measured directly using an appropriate substrate (e.g. p-nitrophenyl derivatives of xylobiose or cellobiose), as described in Handelsman *et al.,* 2004.

The affinity of between the truncated dockerin polypeptide and the cohesin domain may be 2-fold less than the affinity between the wild-type dockerin domain and the cohesin domain. The cohesin-dockerin pair of methods and compositions may have a $K_a$, when the proteins are unmutated, of $10^9$-$10^{13}$ $M^{-1}$. The $K_a$, of the unmutated proteins may

be $10^8$-$10^{13}$ $M^{-1}$. The $K_a$, of the unmutated proteins may be 2 x $10^9$-$10^{13}$ $M^{-1}$. The $K_a$, of the unmutated proteins may be 5 x $10^9$-$10^{13}$ $M^{-1}$. The $K_a$, of the unmutated proteins may be $10^{10}$-$10^{13}$ $M^{-1}$. The $K_a$, of the unmutated proteins may be at least $10^8$ $M^{-1}$. The $K_a$, of the unmutated proteins may be at least 2 x $10^9$ $M^{-1}$. The $K_a$, of the unmutated proteins may be at least 5 x $10^9$ $M^{-1}$. The $K_a$, of the unmutated proteins may be at least $10^{10}$ $M^{-1}$.

The $K_a$ of the truncated dockerin domain with the wild-type cohesin, in the presence of EDTA may be low enough to act as a reversible affinity tag. The $K_a$ of this combination may be under $10^7$ $M^{-1}$. The $K_a$ of this combination may be under 3 x $10^6$ $M^{-1}$. The $K_a$ of this combination may be under $10^6$ $M^{-1}$. The $K_a$ of this combination may be under 3 x $10^5$ $M^{-1}$. The $K_a$ of this combination may be under $10^5$ $M^{-1}$. The $K_a$ of this combination may be under 3 x $10^4$ $M^{-1}$. The $K_a$ of this combination may be under $10^4$ $M^{-1}$. The $K_a$ of this combination may be under 5 x $10^3$ $M^{-1}$. The $K_a$ of this combination may be under 2 x $10^5$ $M^{-3}$. The $K_a$ of this combination may be under $10^3$ $M^{-1}$. The $K_a$ of this combination may be under 5 x $10^2$ $M^{-1}$. The $K_a$ of this combination may be under 2 x $10^2$ $M^{-1}$. The $K_a$ of this combination may be under $10^2$ $M^{-1}$. The $K_a$ of this combination may be under 5 x 10 $M^{-1}$. The $K_a$ of this combination may be under 2 x $10^1$ $M^{-1}$. The $K_a$ of this combination may be under $10^1$ $M^{-1}$.

## Suitable cohesin domains

The cohesin domain of methods and compositions is, A Type-I cohesin domain. The cohesin domain may be a Type-II cohesin domain. The cohesin domain may be any other type of cohesin domain known in the art.

The cohesin domain may be from a species selected from the group consisting of *Clostridium thermocellum, C. cellulolyticum,* and *C. cellulovorans.* The cohesin domain may be from a species selected from the group consisting of *Clostridium thermocellum, C. papyrosolvens,* and *Clostridium cellobioparum.* The cohesin domain may be from a species selected from the group consisting of *Clostridium thermocellum, C. cellulolyticum, C. cellulovorans, C. papyrosolvens, C. josui, Acetivibrio cellulolyticus, Bacteroides cellulosolvens, R. flavefaciens, Ruminococcus albus,* and *Clostridium cellobioparum.*

The cohesin domain may be a cohesin domain from a protein selected from CipA (or scaffoldin) of *C. thermocellum,* CipC of *C. cellulolyticum,* CbpA of *C. cellulovorans,* and CipA of *C. josui.*

The cohesin domain of methods and compositions may be from an *Archaeoglobus fulgidus* protein. Examples of *Archaeoglobus fulgidus* cohesin domains include the sequences set forth in GenBank Accession numbers NP_071198 and NP_071199. The cohesin domain of methods and compositions may be from a thermophilic bacterium. The thermophilic microbe may be selected from the group consisting of *C. thermocellum,* and *Archaeoglobus fulgidus.*

A cellulosomal cohesin domain may be utilized in methods and compositions. A Type I cohesin domain from a cellulosomal protein may be used.

A non-cellulosomal cohesin domain may be utilized in methods and compositions . Non-cellulosomal cohesins are well-known and well-characterized in the art, and include, *inter alia,* the X82 domains from NanJ (GenBank Accession No. YP_694986); the glycoside hydrolases of family 3, 31, 84, and 20 (CpGH3, CpGH31, CpGH84C, and CpGH20, respectively); and NagJ (GenBank Accession No. Q0TR53), all from *Clostridium perfringens* (Adams JJ et al., Structural basis of Clostridium perfringens toxin complex formation. Proc Natl Acad Sci U S A. 2008) and the dockerin domains set forth in GenBank Accession Numbers ABE51693, ABE51694, YP_001324319, YP_001324323, ZP_02077900, ZP_02077903, NP_691654, ZP_02845754, ZP_02846450, ZP_02848919, ZP_02849219, YP_695309, YP_210742, EAY29878, ZP_01693353, CAD71804, NP_864128, ABF40998, ABJ82058, ABB32088, ABQ26119, and ABG39560.

Examples of cohesin domains useful in methods and compositions are found in GenBank Accession numbers YP_001039469, L08665, NZ_ABVG01000001-NZ_ABVG01000046, AB004845, AB025362, AB011057, AY221113, AY221112, and AJ278969.

Other examples of *C. thermocellum* cohesin domains are found in the CipA protein comprising the amino acid sequence as set forth in SEQ ID NO: 8 (GenBank Accession number Q06851). The protein defined by this sequence contains 9 cohesin domains, in residues 29-182, 183-322, 560-704, 724-866, 889-1031, 1054-1196, 1219-1361, 1384-1526, and 1548-1690.

## Cleavable linkers

A recombinant polypeptide of methods and compositions may further comprise a cleavable linker peptide between the truncated dockerin polypeptide and the molecule of interest or antibody-binding moiety. The cleavable linker peptide may be self-cleavable.

Cleavable linkers are well known in the art, and are described, *inter alia*, in Wu WY *et al*., 2006 and in United States patent application 2005/0106700, which is incorporated herein by reference. The cleavable linker may be a chemical or enzymatic cleavage site between the target protein and the dockerin.

The term "peptide" as used herein encompasses native peptides (degradation products, synthetically synthesized peptides, or recombinant peptides), peptidomimetics (typically, synthetically synthesized peptides), and the peptide analogues peptoids and semipeptoids, and may have, for example, modifications rendering the peptides more stable while in a solution. Such modifications include, but are not limited to: N-terminus modifications; C-terminus modifications; peptide bond modifications, including but not limited to $CH_2$-NH, $CH_2$-S, $CH_2$-S=O, O=C-NH, $CH_2$-O, $CH_2$-$CH_2$, S=C-NH, CH=CH, and CF=CH; backbone modifications; and residue modifications. Methods for preparing peptidomimetic compounds are well known in the art and are specified, for example, in Ramsden, C. A., ed. (1992), Quantitative Drug Design, Chapter 17.2, F. Choplin Pergamon Press, which is incorporated by reference as if fully set forth herein.

Peptide bonds (- CO- NH- ) within the peptide may be substituted, for example, by N- methylated bonds (- N (CH3)- CO- ) ; ester bonds (- C (R) H- C- O- O- C (R)- N- ) ; ketomethylene bonds (- CO- CH2- ) ; α- aza bonds (- NH- N (R)- CO- ), wherein R is any alkyl group, e.g., methyl; carba bonds (- CH2- NH- ) ; hydroxyethylene bonds (- CH (OH)- CH2- ) ; thioamide bonds (- CS- NH- ) ; olefinic double bonds (- CH=CH- ) ; retro amide bonds (- NH- CO- ) ; and peptide derivatives (- N (R)- CH2- CO- ), wherein R is the "normal" side chain, naturally presented on the carbon atom. These modifications can occur at any of the bonds along the peptide chain and even at several (2- 3) at the same time.

Natural aromatic amino acids, Trp, Tyr, and Phe, may be substituted for synthetic non-natural acids such as, for instance, tetrahydroisoquinoline-3-carboxylic acid (TIC), naphthylelanine (Nol), ring-methylated derivatives of Phe, halogenated derivatives of Phe, and o-methyl-Tyr.

The term "amino acid" or "amino acids" is understood to include the 20 naturally occurring amino acids; those amino acids often modified post-translationally in vivo, including, for example, hydroxyproline, phosphoserine, and phosphothreonine; and other less common amino acids, including but not limited to 2-aminoadipic acid, hydroxylysine, isodesmosine, nor-valine, nor-leucine, and ornithine. Furthermore, the term "amino acid" includes both D- and L-amino acids. Conservative substitution of amino acids as known to those skilled in the art are within the scope. Conservative amino acid substitutions includes replacement of one amino acid with another having the same type of functional group or side chain e.g. aliphatic, aromatic, positively charged, negatively charged. These substitutions may enhance oral bioavailability, penetration into the central nervous system, targeting to specific cell populations and the like. One of skill will recognize that individual substitutions, deletions or additions to peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. The following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). (see, e.g., Creighton, Proteins (1984)).

As used herein, the term "mutation" carries its traditional connotation and refers to a change, inherited, naturally occurring, or introduced, in a nucleic acid or polypeptide sequence, and is used in its sense as generally known to those of skill in the art.

As used herein, the terms "isolated" refers to oligonucleotides substantially free of other nucleic acids, proteins, lipids, carbohydrates, or other materials with which they can be associated, such association being either in cellular material or in a synthesis medium. The term can also applies to polypeptides, in which case the polypeptide will be substantially free of nucleic acids, carbohydrates, lipids, and other undesired polypeptides.

The term "analogs" extends to any functional chemical or recombinant equivalent of the peptides, which may be characterised by their possession of at least one of the abovementioned activities. The term "analog" is also used herein to extend to any amino acid derivative of the peptides as described hereinabove. Generally, an analog may possess at least 70% sequence identity, at least 80% sequence identity, at least 90% sequence identity, at least 95% sequence identity, and at least 98% sequence identity with the native polypeptide. Percentage sequence identity can be determined, for example, by the Fitch *et al.* version of the algorithm (Fitch et al., Proc. Natl. Acad. Sci. U.S.A. 80: 1382-1386 (1983)) described by Needleman *et al*., (Needleman et al., J. Mol. Biol. 48: 443-453 (1970)), after aligning the sequences to provide for maximum homology. Other alignment techniques are disclosed herein below. Amino acid sequence analogs and variants of a polypeptide can be prepared by introducing appropriate nucleotide changes into DNA encoding the polypeptide, or by peptide synthesis. Such analogs include, for example, deletions from, and/or insertions into, and/or substitutions of, residues within the amino acid sequence of the polypeptide of interest. Any combination of deletion, insertion, and substitution is made to arrive at the final construct, provided that the final construct possesses the desired characteristics. The amino acid changes also can alter post-translational processes of the polypeptide, such as changing the number or position of glycosylation sites. Methods for generating amino acid sequence variants of polypeptides are described, for example, in U.S. Pat. No. 5,534,615.

As used herein, the term "recombinant polypeptide" refers to a polypeptide that has been produced in a host cell which has been transformed or transfected with a nucleic acid encoding the polypeptide, or produces the polypeptide as a result of homologous recombination.

A tagged target protein (e.s., the recombinant polypeptide) can be engineered by inserting a nucleic acid sequence encoding a target protein into a vector such that it is flanked on one side by a nucleic acid sequence encoding a tag (e.g. SEQ ID NOs: 2, 3 or 4). The vector may comprise the tag sequence and may be flanked on one or both sides by a multiple cloning region comprising one or more restriction sites. Factors to be considered when engineering a tagged target protein include, but are not limited to assuring that the nucleic acid sequence encoding a target protein is inserted so that it is contiguous with the nucleic acid sequence encoding a tag. Additionally, it is important to ensure that the sequences encoding the tag and the protein are inserted in frame, thereby assuring translation of the desired tagged protein. The nucleic acid sequence encoding the tag may further comprise a stop codon.

A tagged target synthetic protein (e.s., the synthetic polypeptide), and fragments thereof, can be chemically synthesized in whole or in part using techniques disclosed herein above. See also, Creighton, (1983) Proteins: Structures and Molecular Principles, W. H. Freeman & Co., New York, N.Y., United States of America, incorporated herein in its entirety.

Alternatively, in accordance with methods disclosed herein and known in the art, expression vectors containing a partial or the entire tag/ target protein coding sequence and appropriate transcriptional/ translational control signals are prepared. These methods include in vitro recombinant DNA techniques, synthetic techniques, and in vivo recombination/ genetic recombination. See e.g., the techniques described throughout Sambrook et al., (1989) Molecular Cloning: A Laboratory Manual, Cold Spring Harbor Laboratory, New York, N.Y., United States of America, and Ausubel et al., (1989) Current Protocols in Molecular Biology, Greene Publishing Associates and Wiley Interscience, New York, N.Y., United States of America, both incorporated herein in their entirety.

A variety of host-expression vector systems can be employed to express a tagged target protein coding sequence. These include, but are not limited to microorganisms such as bacteria transformed with recombinant bacteriophage DNA, plasmid DNA, or cosmid DNA expression vectors containing a truncated dockerin polypeptide coding sequence or a recombinant polypeptide comprising a truncated dockerin and a protein of interest coding sequence; yeast transformed with recombinant yeast expression vectors containing a coding sequence of the polypeptide of the present invention; insect cell systems infected with recombinant virus expression vectors (e.g., baculovirus) containing a coding sequence of the polypeptide of the present invention; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing a coding sequence of the polypeptide of the present invention; or animal cell systems. The expression elements of these systems vary in their strength and specificities.

Depending on the host/vector system utilized, any of a number of suitable transcription and translation elements, including constitutive and inducible promoters, can be used in the expression vector. For example, when cloning in bacterial systems, inducible promoters such as pL of bacteriophage .lamda., plac, ptrp, ptac (ptrp-lac hybrid promoter), and the like can be used. When cloning in insect cell systems, promoters such as the baculovirus polyhedrin promoter can be used. When cloning in plant cell systems, promoters derived from the genome of plant cells, such as heat shock promoters, the promoter for the small subunit of RUBISCO, the promoter for the chlorophyll a/b binding protein, or from plant viruses (e.g., the 35S RNA promoter of CaMV; the coat protein promoter of TMV) can be used. When cloning in mammalian cell systems, promoters derived from the genome of mammalian cells (e.g., metallothionein promoter) or from mammalian viruses (e.g., the adenovirus late promoter; the vaccinia virus 7.5 K promoter) can be used. When generating cell lines that contain multiple copies of the tyrosine kinase domain DNA, SV40-, BPV- and EBV-based vectors can be used with an appropriate selectable marker.

The protein to be purified using the method described herein may be produced using recombinant techniques. Methods for producing recombinant proteins are described, e.g., in US Pat No's 5,534,615 and 4,816,567, incorporated herein by reference. The protein of interest may be produced in a CHO cell (see, e.g. WO 94/11026). When using recombinant techniques, the protein can be produced intracellularly, in the periplasmic space, or directly secreted into the medium. If the protein is produced intracellularly, as a first step, the particulate debris, either host cells or lysed fragments, may be removed, for example, by centrifugation or ultrafiltration.

The eluted protein preparation may be subjected to additional purification steps either prior to, or after, the affinity chromatography step. Exemplary further purification steps include hydroxylapatite chromatography; dialysis; hydrophobic interaction chromatography (HIC); ammonium sulphate precipitation; anion or cation exchange chromatography; ethanol precipitation; reverse phase HPLC; chromatography on silica; chromatofocusing; and gel filtration.

The protein thus recovered may be formulated in a pharmaceutically acceptable carrier and may be used for various diagnostic, therapeutic or other uses known for such molecules.

The following examples are presented in order to more fully illustrate some embodiments of the invention. They should, in no way be construed, however, as limiting the broad scope of the invention.

## EXAMPLES

### Materials and methods

### Cloning of Constructs

#### *PCR*

Amplification of DNA fragments for cloning purposes was performed using the T-Gradient device (Biometra, Germany). PCR reaction mixtures contained: *PfuTurbo* (Stratagene, La Jolla, California) or *Extaq* (Takara Bio Inc. Otsu, Shiga, Japan) polymerase (4 units), dNTPs (2.5 mM each dNTP), reaction buffer, 0.5 μM of each primer (forward and reverse), DNA template, double distilled water (DDW) was added to complete the total volume to 50 μl. PCR was programmed as follows: 30 sec initial-denaturing at 95°C; followed by 28-35 cycles of: 30 sec of denaturing at 95°C, 30 sec annealing 50-61°C (mostly 58°C), 60-150 sec (depending on length of the amplified DNA) polymerization 72°C; after the last cycle 5 min of polymerization at 72°C (in order to finish polymerization). DNA samples were purified using a PCR purification kit (Real Biotech Corporation, RBC).

#### *Digestion*

PCR samples and plasmids were double-digested at 37°C for 1-2 hours with the appropriate digestion enzymes and buffers (New England Biolabs Inc. Baverly, Maryland). The required digested DNA fragments (PCR or plasmid) were run and isolated from agarose gel (0.8-1.2%) and purified using a DNA extraction kit (HiYield™ Gel/PCR DNA Extraction kit from RBC).

#### *Ligation and Selection.*

The digested DNA fragments were ligated into the appropriate linearized plasmid (original Novagen pET28a) using T4 DNA ligase according to manufacturer recommendation (Fermentas). Ligated samples were transformed into competent *E. coli* XL1-blue or DH5α strains.

#### *Bacterial Strains*

*E. coli* Strain K-12 DH5α. Genotype: $F^-$ end Al hsd R17 ($r_k^-$, $m_k^-$) sup E44 thi-1 $\lambda^-$rec A1 gyr A96 rel A1 Δ(arg $F^-$ lac ZYA) U 169 ψ80d lacZΔM15.

*E. coli* Strain K-12 XL1-blue. Genotype: rec A1 end A1 gyr A96 thi-1 hsd R17 ($r_k^-$, $m_k^-$) sup E44 rel A1 lac {F' pro AB lacI$^q$ZΔM15 Tn 10 (tet$^r$)}.

*E. coli* Strain B BL21(λDE3). $F^-$ ompT gal dcm lon hsd$S_B$($r_B^-$ $m_B^-$) λ(DE3 [lacI lacUV5-T7 gene 1 indl sam7 nin5])

#### *Transformation*

All the *E. coli* strains mentioned (XL- 1, DH5α, BL21 (λDE3) ) were transformed using the heat- shock technique. Ligation product or plasmid was added to 200 μl of competent cells and left to stand for 10 min on ice. Following one minute of heat- shock at 42°C the cells were transferred to ice for two additional minutes. One ml of Luria- Bertani (LB) medium was added to the cells for one hour recovery in a 37°C shaker. Next the cells were centrifuged (14000xg, 30 sec), resuspended in 100 μl LB and plated on 50 μg/ml kanamycin plates. Antibiotic- resistant colonies were isolated and further screened for positive clones trough the colony PCR procedure (similar to the previously described PCR using a bacterial colony as the PCR template) . Positive clones were amplified using a Plasmid DNA purification kit (iNtRON biotechnology Inc.) and verified by sequencing.

### Ligation Independent Cloning

#### *Restriction Free Cloning*

Restriction Free (RF) cloning was done according to van den Ent *et al.* The primers were ~50 bp long, the 28-bp 5' part was homologues to the insertion vector whereas the remaining- 20 bp were homologous to the fragment being inserted. In the first step regular PCR was done, as described above, in order to amplify the fragment to be inserted. This fragment contained flanking ends of 28- bp homologues to the vector. In the second step an additional PCR reaction was conducted. The reaction mixture included 100 ng of the PCR product from the previous step, vector DNA (pET28a) 10 ng, 10 mM dNTPs, *PfuTurbo* (4 units), the total volume was completed to 50μl with DDW. The PCR included the following steps: Initial denaturation 95°C 30 sec; followed by 35 cycles of: denaturation 95°C 30 sec; annealing 55- 61°C 1min; extension 68°C 2 min for every kilobase pair (12- 15min) . The final extension was done at 72°C for 10 min. At the end of the PCR 10 μl were removed and supplemented with 1 μl of the methylated DNA restricting endonuclease (DpnI), and its reaction buffer (NEBuffer 4) for 2 hr at 37°C. Subsequently, the 10μl were transformed to *E. coli* DH5α.

***In-Fusion™.***

The primers were designed as recommended in the user manual using the Clontech online tool (http://bioinfo.clontech.com/infusion/ convertPcrPrimersInit.do). The reaction was performed according to the user manual, and contained 200 ng PCR insert, 100 ng of linearized vector (previously restricted), and deionized water to a final volume of 10μl. The mixture was added to the In-Fusion Dry-Down pellet incubated for 15 min at 37°C, following 15 min at 50°C. Next, the mixture was transferred to ice, diluted with 40μl of TE buffer and transformed to *E. coli* DH5α.

**<u>Protein Expression and Purification:</u>**

***Protein Expression***

*E. coli* BL21 (λDE3) strain was used for over- expression of the recombinant proteins. The host cells were grown in 250- 500 ml LB medium, supplemented with 50 μg/ml kanamycin, at 37°C until culture reached $OD_{600}$ >0.6. IPTG (isopropyl- β- D- thiogalactopyranoside) was added at final concentration of 0.1- 1 mM, for induction of protein expression. Culture growth was continued for another 3 hr at 37°C, and 30°C or for overnight at 16°C, according to predetermined optimization experiments. Cells were harvested by centrifugation (6000 rpm, 20 min, 4°C) .

Immediately before purification, cells were resuspended in 10- 20 ml TBS (Tris- buffered saline- 25 mM Tris-HCl, 137 mM NaCl, 2.7 mM KCl, pH 7.4), supplemented with 1 mM $CaCl_2$ (TBS- $CaCl_2$) and lysed by sonication. Sonication was performed on ice, using pulses to avoid overheating of the solution. The lysate was centrifuged (15000 rpm, 30 min, 4°C), and the supernatant was used for protein purification.

***His-tagged Protein Purification***

Purification using an FPLC AKTA-prime System (Amersham Pharmacia Biotech): The protein-containing supernatant was loaded on a 3-5 ml Ni-NTA column, pre-equilibrated with TBS-$CaCl_2$, at 0.5 ml/min. The column was washed with ~40 ml TBS-$CaCl_2$ supplemented with 5 mM imidazole, 1 ml/min. Protein was eluted in TBS-$CaCl_2$ buffer and a linear gradient of 5-250 mM imidazole over ~30 ml, 1 ml/min..

Fractions (1-2 ml) were collected and analyzed on SDS-PAGE (10-15%), and visualized by coomassie brilliant blue (CBB) staining. Fractions containing relatively pure protein were pooled and dialyzed overnight at 4°C against TBS-$CaCl_2$.

***CBM-Containing-Protein Purification***

Batch purification: The supernatant was mixed with 10-15 ml of amorphous cellulose or five grams of Perloza MT 100 beaded cellulose (IONTOSORB, Usti nad Labem, Czech Republic), in a 50 ml tube, for one hr, on a rotator at 4°C. The amorphous/beaded cellulose was pelleted by centrifugation (4000 rpm, 5 min, 4°C). The pellet was washed 3 times with ~45 ml TBS, containing 1 M $NaCl_2$ and three times with 45 ml TBS. Each wash consisted of rapid vortexing (until the pellet was completely resuspended) and five min. rotation. Protein was eluted from the amorphous cellulose pellet by 12 ml (2-4 times) of 1% (v/v) triethylamine (TAE). Eluted fractions were quickly neutralized to pH~7 with 0.5-1.5 ml of 1 M MES at pH 5.5 and dialyzed against TBS-$CaCl_2$. When beaded cellulose was used no further elution steps were applied. The beaded cellulose was supplemented with sodium azide ($NaN_3$) to a final concentration of 0.05% and stored at 4°C. Purity of either the elution fractions or the beaded cellulose was estimated by SDS-PAGE (10-15%).

***Cohesin-Dockerin Based Affinity Chromatography***

The supporting matrix, comprising 2 g of Perloza MT 100 beaded cellulose (IONTOSORB, Usti nad Labem, Czech Republic), suspended in 5 ml TBS, was packed into a C10-series column (GE Healthcare, Pittsburgh, Pennsylvania). The column was then connected to an AKTA-Prime system (Amersham Pharmacia Biotech, Rehovot, Israel), and the flow rate was set at 1-3 ml/min throughout the experiment. The column was loaded with 54 μM of purified CBM-Coh, and then flushed with 30 ml of TBS-$CaCl_2$. A 5- to 20-ml cell extract of *E. coli* BL21 (λDE3) expressing the dockerin-tagged protein was applied to the column and washed with 30 ml of TBS-$CaCl_2$. The elution step was carried out under a gradient (0-250 mM) of EDTA in TBS, after which the system was equilibrated, for repeated applications, to its starting position with TBS-$CaCl_2$. The column flow-through and the elution fractions were collected and analyzed on SDS-PAGE.

When beaded cellulose on which CBM-Coh was directly purified was used, 1-2 ml were directly packed into a C10-series column. Except for the CBM-Coh loading step all of the following steps were the same as described above.

***Protein Concentration and Preservation***

Protein concentration was estimated by the absorbance at 280 nm and the extinction coefficient of the desired protein as calculated by Vector NTI program suite, (Invitrogen, Carlsbad, California.) from the known amino acid sequence. If needed, dilute protein solutions were concentrated using Vivaspin 2/6 ml, 5,000 MW cutoff - concentrators. Proteins were stored in 50% (v/v) glycerol at -20°C.

***Truncated dockerin-containing constructs***

A construct coding for *G. stearothermophilus* xylanase T-6 with an *EcoRI* site at the 5'-terminus and a *XhoI* site at the 3'-terminus was produced using PCR (Handelsman *et al.,* 2004). This construct was ligated at the *EcoRI* site with the PCR product of a *C. thermocellum* Ce148S dockerin gene (recombinant cellulosomal family-48 cellulase) (Wang *et al.,* 1993), containing tandem 5'-terminal *NcoI* and *BamHI* sites and a 3'-terminal *EcoRI* site. The latter two PCR products were then inserted in concert into the pET-28a vector at the *NcoI* and *XhoI* sites. The resulting plasmid (pNDoc1) allows facile replacement of the Cel48S dockerin (termed hereafter Doc) with any other desired dockerin by digestion with *EcoRI* and either *NcoI* or *BamHI.* The resulting expressed product constitutes a C-terminal His-tagged xylanase T-6 fusion protein, bearing a dockerin at the N-terminus (termed Doc-Xyn). The desired truncated dockerins were generated by PCR, with a sense primer that introduced an *NcoI* site and an anti-sense primer that introduced an *EcoRI* site, utilizing wild-type (WT) Doc-Xyn as a template.

In order to challenge the performance of the truncated dockerin (Doc (Δ16) ) as a purification tag, other candidates as diverse as possible were chosen, for example the jellyfish *Aequorea victoria* Green Fluorescent Protein (GFP), and the *E. coli* Maltose Binding Protein (MBP) . In order to examine if the purification process has any effect on the enzyme activity, two enzymatically active proteins were chosen: the *C. thermocellum* β- glucosidase, and the *E. coli* Thioesterase/ Protease I (TEP1) . In addition, two copies of the *Staphylococcus aureus* Fc binding B- domain of protein A (ZZ domain) were purified and additional aspects were applied to form a reusable antibody purification system.

Additional constructs were produced by replacement of Xyn with the above-mentioned enzymes, through digestion of the pET-28a vector with *EcoRI* and *XhoI* restriction enzymes and ligation of the respectively digested PCR products. The model proteins which contained the same restriction sites in the middle of their sequence were constructed through the Restriction Free (RF) method, as described above.

The resulting constructs are described and presented schematically in Table 1.

**Table 1. Fusion protein constructs used in the affinity purification studies.**

| Construct | Molecular Weight | Protein of Interest | Affinity Tag | Location of The Tag | SEQ ID NO: |
|---|---|---|---|---|---|
| wtDoc-Xyn | 53135 Da | Xyn | wtDoc | N-terminus | 123 |
| Doc(Δ16) - Xyn | 50785 Da | Xyn | ΔDoc | N-terminus | 124 |
| wtDoc-GFP | 35550 Da | GFP | wtDoc | N-terminus | 125 |
| Doc(Δ16)-GFP | 33880 Da | GFP | ΔDoc | N-terminus | 126 |
| GFP-wtDoc | 35270 Da | GFP | wtDoc | C-terminus | 127 |
| GFP-Doc(Δ16) | 33600 Da | GFP | ΔDoc | C-terminus | 128 |
| Doc(Δ16)-TEP1 | 27670 Da | TEP1 | ΔDoc | N-terminus | 129 |
| Doc(Δ16)-BglA | 58472-Da | β-glucosidase | ΔDoc | N-terminus | 130 |
| Doc(Δ16)-ZZ -domain | 22500 Da | ZZ-domain | ΔDoc | N-terminus | 131 |

All constructs had a C- terminal His tag except for the GFP- wtDoc and GFP- Doc (Δ16) constructs which included a His tag located at the N- terminus of GFP. It should be understood that the SEQ ID NOs listed in table 1 refer to the amino acid sequence of the corresponding constructs.

The full- length sequence of the dockerin- bearing protein encoded by the Cel48S dockerin, which contains a type I dockerin domain on its C- terminus, is set forth in SEQ ID NO: 7 (GenBank Accession # L06942) . The wild- type Type- I dockerin domain, utilized in the Examples herein, is set forth in SEQ ID NO: 1. The truncated dockerin domain (Doc (Δ16) ), utilized in the Examples herein, is set forth in SEQ ID NO: 6.

***CBM-Coh construct***

The gene encoding the protein construct (CBM-Coh), consisting of a carbohydrate-binding module (CBM) and a cohesin (Coh) from the *C. thermocellum* CipA (SEQ ID NO: 9; GenBank Accession # L08665), was cloned as previously described (Yaron *et al.,* 1995).

**Table 2. Primers used in cloning the constructs of the invention.**

| Construct | | Amino Acid sequence | SEQ ID NO: |
|---|---|---|---|
| Wt Doc XynT6 | 5' | CCCCATGGGATCCGGCGACGTCAATGATGACGG | 132 |
| | 3' | GGGGAATTCGTTCTTGTACGGCAATGTATC | 133 |
| Wt/ Doc (Δ16)-GFP | 5' | GATACATTGCCGTACAAGAACGAATTCATGAGTA AAGGAGAAGAAC | 134 |
| | 3' | CTCAGTGGTGGTGGTGGTGGTGCTCGAGTTTGTAG AGCTCATCCATGC | 135 |
| GFP wtDoc | 5' | ACCATGAGCCACCATCACCATCACCATATGAGTA AAGGAGAAGAACTT | 136 |
| | 3' | GTCATCATTGACGTCGCCAGGTACCACTTTGTAGA GCTCATCCATGCC | 137 |
| Doc (Δ16)-TEP1 | 5' | CCATCAGAATTCATGGCGGACACGTTATTGATTC | 138 |
| | 3' | CAGATACTCGAGTGAGTCATGATTTACTAAAG | 139 |
| Doc(Δ16)-BglA | 5' | GTATCCGAATTCATGTCAAAGATAACTTTCCC | 140 |
| | 3' | GCATAACTCGAGAAAACCGTTGTTTTTGATTAC | 141 |
| | 5' | GTACAAGAACGAATTCATGTCAAAGATAACTTTC C | 142 |
| | 3' | GGTGGTGGTGCTCGAGAAAACCGTTGTTTTTGATT AC | 143 |
| Doc(Δ16) ZZ -domain | 5' | CACGGTGAATTCCTGGTGCCACGCGGTTCCATG | 144 |
| | 3' | CCAATGCTCGAGTGCAAGCTTGTCATCGTCGTC | 145 |
| Doc (Δ16)-XynT6 | 5' | TATACCATGGGATCCAAGAGATATGTTTTGAGAT | 146 |
| | 3' | TCTTGAATTCGTTCTTGTACGGCAATGTATCTATTT CTTT | 147 |

***ELISA-based affinity assay***

The ELISA-based cohesin-dockerin binding assay was performed essentially according to Barak *et al* (Barak *et al.,* 2005) using a matching Coh-Doc fusion-protein system. The interaction of the test cohesin with the truncated dockerins was expressed as the function of change in Gibbs free energy (ΔΔG°), relative to the wild-type dockerin, calculated using equation 1:

Equation 1: $$\Delta\Delta G = \Delta G^{WT} - \Delta G^{mut} = -RT \ln (EC_{50}{}^{WT} / EC_{50}{}^{mut})$$

where *R* is the gas constant, *T* the absolute temperature (°K), and the $EC_{50}$ was determined from the binding curves of the truncated Doc-Xyn fusion proteins, compared with that of the wild-type Doc-Xyn (Reichmann *et al.,* 2007). To determine the $EC_{50}$ of the truncated dockerins, a nonlinear fit for the ELISA curves was calculated using the GraphPad Prism 4 program (GraphPad Software, Inc., La Jolla, CA).

In order to calculate the different $K_D$ values (e.g., for Doc (Δ16) ), $\Delta G^{WT}$ was first calculated by solving Equation 2 for $K_D=1.7x10^{-10}$ (*61*) and T=298 °K. Next, $\Delta G^{mut}$ was calculated by solving equation 1 with the previously calculated values of ΔΔG and $\Delta G^{WT}$. Finally, the different $K_D$ values were calculated by solving equation 2 once again with the calculated $\Delta G^{mut}$ value (and T= 298 °K) .

$$\text{Equation 2:} \qquad \Delta G = -RT \ln (K_D)$$

***Competitive ELISA***

This competitive assay was done in order to measure the relative binding affinity of different fusion proteins to which no primary antibody were available. It was done similarly to the above- mentioned ELISA procedure with a few changes. ELISA plates were initially coated with CBM- cohesin (50 nM), blocked and washed as described. Next, different concentrations (1 pM- 0.2 μM) of the dockerin- fused proteins (Doc (Δ16)- GFP, wtDoc- GFP) were mixed with a constant concentration of the wtDoc- Xyn (100 pM) . This mixture was then added in duplicate into the wells for interaction. Subsequently, washing and detection steps were conducted as mentioned above. The wtDoc- Xyn interaction with the cohesin was challenged by increasing concentrations of the dockerin- fused test protein, which resulted in the reduction of recognition by the primary antibody and consequently in the signal produced by the secondary antibody.

To determine the inhibition concentration ($IC_{50}$) of the Doc (Δ16) / wtDoc, a nonlinear fit for the ELISA curves was calculated using one- site binding competitive equation (Equation 3) of the GraphPad Prism 4 program, using (GraphPad Software, Inc., La Jolla, CA) . LogIC50 is the logarithm of the, IC50 (50% of the binding sites are occupied by the competitor) .

$$\text{Equation 3:} \qquad Y = Bottom + \frac{(Top - Bottom)}{1 + 10^{X - LogIC50}}$$

The changes in the Gibbs free energy (ΔΔG°) between the wild type and the truncated dockerin and their respective interactions with test cohesin were calculated using Equation 4.

$$\text{Equation 4:} \qquad \Delta\Delta G = \Delta G^{WT} - \Delta G^{mut} = -RT \ln (IC_{50}{}^{WT} / IC_{50}{}^{mut})$$

**EXAMPLE 1**

Cohesin-dockerin affinity purification using xylanase T-6

The aim of this study was to develop and optimize an efficient affinity-purification system, based on the CBM (carbohydrate-binding module) and cohesin-dockerin interaction. Beaded cellulose was used as the column support matrix for the immobilization of a type-I cohesin (Coh) module-containing CBM-Coh fusion protein, wherein the Coh module was from the same bacterium from which the cellulose was derived. This simple application step served as a non-covalent means for "activating" the column for subsequent purification of a matching dockerin-containing target protein. No leakage of the CBM-Coh fusion protein from the column was detected after extensive washing with buffer, and the column was then ready for protein purification.

The target protein destined for purification was fused to a truncated dockerin as an affinity tag and could be eluted effectively from the column by graded concentrations of EDTA. The regenerated cellulose:CBM-Coh column was available for subsequent use without significant reduction of its efficiency and capacity.

The first model target protein comprised *G. stearothermophilus* xylanase T-6, fused to a *C. thermocellum* dockerin (the affinity tag). A solution containing the dockerin-borne target protein was loaded onto the column, followed by extensive buffer washes in the presence of calcium. To elute the protein, a gradient of EDTA was applied, and protein elution was continuously monitored spectroscopically. The appropriate fractions were analyzed subsequently by SDS-PAGE. A schematic description of the approach is presented in FIG. 1.

***Cohesin-dockerin affinity chromatography***

Cell lysates of *E. coli,* expressing the wild-type dockerin-xylanase chimaera (Doc-Xyn) were applied onto the activated column (FIG. 2A, Ap) resulting in a large peak corresponding to unbound protein which immediately passed through the column; the column was washed with TBS-Ca and then subjected to a gradient of EDTA to elute the bound protein (FIG. 2A, E1). As seen from the chromatogram and the accompanying SDS-PAGE (FIG. 2B) of the fractions, very little protein was eluted from the column (FIG. 2B fractions 17-22). Most of the protein was retained on the beaded cellulose (FIG. 2B), suggesting that the cohesin-dockerin complex is too tight to dissociate using EDTA.

**EXAMPLE 2**

Truncation of residues 2-17 of the dockerin domain confers reversible binding to the cohesin-dockerin system and reusability of the affinity column

To overcome the tight cohesion-dockerin association, a homologous series of truncated dockerins were created and used to replace the dockerin tag in Doc-Xyn. FIG. 3A depicts the sequences of the wild type dockerin and truncated derivatives. The two dockerin segments (conserved duplicated regions) are indicated on top. Residues involved in $Ca^{2+}$ coordination are highlighted in gray. Black-highlighted (white font) residues represent those involved in direct hydrogen bonding to cohesin via the second duplicated repeat. Hydrogen-bonding residues in the alternative symmetry-related mode are shown in open boxes. Positions of the helices are marked *h1, h2* and *h3*, respectively. The first and second calcium-binding loops are marked as *$Ca^{2+}$ loop-1* and *$Ca^{2+}$ loop-2,* respectively.Binding affinities of the truncated dockerins to cohesin was assessed quantitatively by an ELISA-based method. Deletion of the N-terminal dockerin, from $Asp^2$ (residue 2 of SEQ ID NO:1; the first residue of the calcium-binding loop) up to $Lys^{18}$ (residue 18 of SEQ ID NO:1) in the middle of the first α-helix, yielded Doc(Δ16), which retained binding at a level close to that of the wild-type module (ΔΔG=0.4 kcal/mol) (FIG. 3B-C). Further incremental expansion of the dockerin deletion served to almost entirely abolish its binding to cohesin.

Thus, Doc (Δ16) (also designated as ΔDoc) was examined as a short affinity tag for the purification of the target protein on the CBM- Coh- immobilized beaded cellulose. A total of 24 nmol of Doc (Δ16)- Xyn was recovered from the column after elution, close to the total amount loaded onto the column (27 nmol) .

wtDoc- and Doc (Δ16)- Xyn were further evaluated for their binding to type- I cohesin not only in the presence of $Ca^{2+}$ but also in the presence of EDTA (FIG. 3E) . Cohesin- dockerin interactions were measured using the ELISA-based assay. 96- well plates (nunc™) were coated with CBM- Coh and interacted with either wtDoc- Xyn or Doc (Δ16)- Xyn in the presence of 1mM $CaCl_2$ or 10mM EDTA. The wtDoc- Xyn presented the strongest binding in the presence of $Ca^{2+}$, while in the presence of EDTA its binding was somewhat compromised (ΔΔG=2.01 kcal/mol) . In the presence of $Ca^{2+}$, Doc (Δ16)- Xyn interacted similarly to the wtDoc- Xyn supplemented with EDTA (ΔΔG=2.4 kcal/mol, between wild- type and truncated form supplemented with $Ca^{2+}$), while in the presence of EDTA the Doc (Δ16)- Xyn failed to present any significant binding. These results demonstrate that the truncated DocS although lacking its first α- helix and the first $Ca^{2+}$ binding loop retained relatively high binding capacities.

Furthermore, xylanase purified using the Doc (Δ16) in the cohesin- dockerin system exhibited enhanced purity compared to the purified by immobilized metal- ion affinity chromatography (IMAC) by virtue of the His tag (FIG. 3D) . Thus, cohesin- dockerin affinity purification using Doc (Δ16) is superior to both cohesin- dockerin affinity purification using wild- type dockerin and to IMAC.

**EXAMPLE 3**

Cohesin-dockerin affinity chromatography columns containing Doc(Δ16)-Xyn exhibit excellent yield, elution, and reusability

A critical feature for protein affinity purification is the ability to reuse the column several times without a decrease in performance. Four identical samples of the *E. coli* crude lysate (5 ml), containing the expressed Doc (Δ16)- Xyn, were applied (Ap) onto the column (2 ml of beaded cellulose) and eluted (El) using an EDTA gradient (FIG. 4A) . The eluted fractions in each cycle were highly enriched with Doc (Δ16)- Xyn (FIG. 4B) without any apparent contaminating proteins or CBM- Coh. Nearly identical amounts of protein were purified in the successive rounds, underscoring the robustness of the affinity tag.

**EXAMPLE 4**

Cohesin- dockerin affinity chromatography columns containing Doc (Δ16) fused to the N- terminus of the GFP exhibit excellent yield, elution, and reusability

The binding capacity of a truncated dockerin GFP (Doc (Δ16)- GFP) was evaluated relatively to the wild type Doc GFP (wtDoc- GFP) by a competitive ELISA assay. Thus, an ELISA plate was coated with CBM- Coh and rising amounts of either wtDoc- GFP or Doc (Δ16)- GFP fusion proteins were mixed together with a constant amount of wtDoc-Xyn. The reaction was done in the presence of 1mM $CaCl_2$. After appropriate incubation time the plate was washed and examined with anti- Xyn antibodies. As can be seen (FIG. 5A), the truncated dockerin had similar affinity to that of the wild- type module (ΔΔG=1.2 kcal/mol) . The competitive ELISA adds another parameter to the system thus making it more accurate. This experiment indicates that the Xyn fusion had but a minor effect on the interaction between the dockerin and the cohesin and further substantiates the dual- binding mode of the dockerin module.

In order to evaluate column durability, cell lysates of E. coli expressing Doc (Δ16) fused to the N- terminus of the GFP were applied and eluted consecutively using the immobilized cohesin column. Samples of the *E. coli* crude lysate (20 ml), containing the expressed Doc (Δ16)- GFP, were applied (Ap) onto the column (2 ml of beaded cellulose 2 mg CBM- Coh) and eluted (El) using an EDTA gradient. Following the first elution, two consecutive applications of the unbound protein (~5- 10 ml) were further applied and eluted. The different stages were monitored throughout the procedure by following the absorbance at 280 nm (FIG. 5B) . The eluted fractions were analyzed subsequently by SDS-PAGE (~40- 50 nmol purified protein per elution) (FIG. 5C 1- 3) .

The first elution (EL 1; FIG. 5B) indicates that the protein band corresponding to the calculated size of the Doc (Δ16)- GFP appears to be homogeneous and enriched after only one purification step. Thus, the washing step was insufficient and the first elution commenced before the entire unbound fraction was released, corresponding to the relatively high absorbance peak on the chromatogram, and to the minor impurities on the gel. In the following applications, the washing step was extended for longer periods of time, which resulted in relatively smaller absorbance peaks but highly homogenous protein bands.

Since the column is based on the direct binding between the cohesin and the Doc(Δ16), the maximum amount of obtained protein corresponds to the relatively small amount of pre-incubated CBM-Coh. Thus, it is not surprising to notice large amounts of proteins in the flow-through fractions (FIG. 5D). The protein found in the flow through was properly folded and bound to the column in the consecutive application as can be observed in the reduction in the amount of protein found in the unbound fractions. Each consecutive elution, not only retained the column capacity, but also achieved better results stemming from increased washing period. Even in the third elution single band in highly purified state is observed, serving as evidence to the robustness of the system (FIG. 5C 3).

In order to test the effectiveness of the elution step, some column beads were removed and subjected to SDS-PAGE after the final application was eluted and the column was extensively washed. The beads appear to contain only a single band corresponding to the molecular weight of the CBM- Coh, thus indicating that the Doc (Δ16)- GFP was completely eluted following the application of EDTA (FIG. 5E) .

In order to compare the new affinity system to the commonly used IMAC, Doc (Δ16)- GFP was purified through its His tag rather than the ΔDoc affinity tag (FIG. 5F) . Although high amounts of protein were received, a very close band was seen adjacent to the eluted protein. This band was absent from any of the elutions observed when using the Doc (Δ16) system, demonstrating its supremacy and its high specificity in protein purification. In order to examine whether this contaminating band may represent cleavage between the Doc (Δ16) and the GFP, a highly purified sample was sent to N- terminal sequencing. While the upper band was recognized as the beginning of the Doc (Δ16) the lower band could not be identified. This may imply that the truncation occurred in several close positions and not in one specific position or that it is an unrelated contamination of the host proteins with exposed histidine residues that were captured on the Ni beads.

## EXAMPLE 5

Cohesin-dockerin affinity chromatography columns wherein wild-type dockerin is fused to the N-terminus of GFP

Unlike the affinity system described by Craig *et al.,* GFP with wild- type dockerin at the N- terminus (wtDoc-GFP) binding to the CBM- Coh was too strong and impossible to elute the fusion protein even with concentrations as high as 500 mM EDTA (FIG. 6A) . It can be seen that the wtDoc- GFP retained on the column bound to the CBM- Coh and was separated after boiling the column beads (Perloza beaded cellulose) with SDS for 5 min. Only negligible amounts of wtDoc- GFP could be seen in the elution fraction. The CBM- Coh has a higher molecular weight (~36kDa), therefore it probably represents the upper band in the boiled beads fraction, whereas the wtDoc- GFP is the lower one.

In order to rule out the possibility that the wtDoc-GFP did not express well in the bacteria, a C-terminal His tag allowed an alternative affinity system (Ni-NTA) for purification of the protein (FIG. 6B). By applying increasing amounts of imidazole, large amounts of protein was obtained from the bacteria cell lysate, indicating that the strong binding of the intact dockerin, rather than any expression difficulties, was the reason for the poor protein elution using the CBM-Coh column.

**EXAMPLE 6**

Cohesin-dockerin affinity chromatography columns containing Doc(Δ16) fused to the C-terminus of the GFP exhibit excellent yield, elution, and reusability

A good affinity tag should exhibit similar qualities (specific attachment and efficient elution) when fused either to the N or the C- terminus of the protein of interest, in order to extend the purification options. However, the same affinity tag may have a very different effect on the expression, solubility and/or stability properties of a protein when fused to its N- rather than its C- terminus or vice versa. Thus, two additional versions of the GFP dockerin fusion proteins were constructed, wherein the truncated DocS (Doc (Δ16) ) or the wild type DocS (wtDoc) was positioned at the C- terminus of the GFP, and a His tag was positioned at the N- terminus. Under similar conditions as previously described, a relatively pure band corresponding in size to GFP- Doc (Δ16) was specifically purified in two consecutive applications of cell lysate (FIG. 7A- B), demonstrating the ability of the tag to be fused and remain active at the C- terminus of the protein.

Nevertheless, unlike in the previous section where no contaminations were seen in the CBM- Coh purified protein, an additional band appeared. This may imply that some cleavage events may have occurred at the N- terminus of the GFP- Doc (Δ16) during the expression in the bacterial host, while the C- terminally positioned Doc (Δ16) retained the ability to interact with the CBM- Coh. Alternatively, bond breakage may have occurred after the purification (during dialysis or storage) . On the other hand, this band may still represent some contamination.

Similar to the wtDoc-GFP the GFP-wtDoc did not elute from the column and could be seen together with CBM-Coh after boiling the column beads in the presence of SDS (FIG. 7C).

**EXAMPLE 7**

Cohesin-dockerin affinity chromatography columns containing Doc(Δ16) fused to the N-terminus of the ZZ domain

The ZZ-domain is a synthetic analogue of the B-domain of protein A from the bacteria *Staphylococcus aureus*. It is widely used in research and in the industry due to its ability to bind the heavy chain Fc region of immunoglobulins. Often the ZZ-domain is immobilized onto a solid support and used as a purification method of total IgG from blood serum. Attaching a detachable affinity tag to the ZZ-domain could make it reusable and more applicable not only for antibody purification but for other nanotechnological applications.

Therefore, Doc (Δ16) fused at the N- terminus of the ZZ- domain was cloned and expressed (FIG. 8A) . Due to its repetitive nature, ZZ- domain was cloned with an addition of 20 amino acids (7 upstream and 13 downstream), which enables the cloning of the two identical domains together. The resulting protein (Doc (Δ16)- ZZ) was immobilized onto beaded cellulose through CBM- Coh interaction. Subsequently, diluted mouse serum was applied onto the column, and, after extensive washes with TBS- $CaCl_2$, IgG's were eluted using glycine buffer (0.1M pH 2.8) . The Doc (Δ16)- ZZ specifically bound antibodies from mouse total blood serum (FIG. 8B) as can be deduced from molecular weight of the eluted proteins (50 kDa and 25 kDa) corresponding to the 150 kDa molecular weight of IgG's heavy and light chains.

**EXAMPLE 8**

Purification and activity assay of the BglA fusion protein

Many affinity tags usually have some influence on their adjacent proteins and therefore have to be removed. On the other hand, the His tag is considered to have a relatively minor effect on the protein when added, and is therefore a preferred affinity tag in this respect. Therefore, the *C. thermocellum* β-glucosidase (BglA) was purified, and the effect of the Doc(Δ16) tag on its fused protein function was examined.

The *C. thermocellum* β-glucosidase has a crucial role in the degradation of cellulose. It hydrolyzes the cellobiose - a strong inhibitor of the cellulase system, into fermentable glucose thus allowing the degradation of cellulose to continuously proceed. Enhancement of cellulose degradation was previously observed when free β-glucosidases from different sources were supplemented to cellulose-degrading enzymes. In addition, the thermophilic origin of the β-glucosidase makes it potentially useful in industrial saccharification of cellulosic substances even as the free enzyme, i.e., not supplemented to a cellulose-degrading system. Therefore, BglA was chosen as a model for enzymatic studies.

Truncated DocS was fused to the N-terminus of β-glucosidase (Table 1, Doc(Δ16) -BglA), expressed and purified on CBM-Coh bound to beaded cellulose. A single band of about ~58 kDa, corresponding to the calculated size of the fusion protein, can be seen (FIG. 9A). Two consecutive elutions were performed each produced a relatively pure single major band. The addition of the Doc(Δ16) tag had no significant effect on the activity of the enzyme compared to the activity of the wild-type enzyme (without the dockerin module, containing only a His tag; FIG. 9C), which was purified using a Ni-NTA affinity column. ΔDoc-BglA had kinetic parameters on the same order of magnitude as the wild-type BglA (with His tag) with slightly better values (Table 3) (FIG. 9B). The enzyme activity was tested at 50°C, similar to the environmental conditions of the thermophilic bacterium and temperature optimum of the enzyme. The curves were fitted using the Graphpad prism program.

**Table 3.** Kinetic parameters of different BglA dockerin derivatives

| | $V_{max}$ [$Ms^{-1}$] | $K_m$ [mM] | $k_{cat}$[$s^{-1}$] | $k_{cat}/K_m$ [$mM^{-1}$ $s^{-1}$] |
|---|---|---|---|---|
| Wt BglA | 2.2E-06 | 12.08 | 21.53 | 1.8 |
| ΔDoc BglA | 5.4E-07 | 4.9 | 53.8 | 10.9 |
| BglA Doc Cc | 7.7E-07 | 13.02 | 7.7 | 0.6 |

The addition of the free β-glucosidase to cellulosomal systems was proved to enhance their degradation activities by removing the inhibitory properties of the cellobiose produced by converting the disaccharide to the non-inhibitory glucose. In light of this, it is hypothesized that the addition of dockerin-fused β-glucosidase could enhance the degradation process even more than the addition of the free enzyme by creating a proximity effect.

**EXAMPLE 8**

Purification and activity assay of the TEP1 fusion protein

*E. coli* thioesterase I (TEP1) has been documented to execute diverse activities of thioesterase, esterase, arylesterase, protease, and lysophospholipase. TEP1 was first shown to catalyze hydrolytic cleavage of acyl- CoA thioesters. Subsequently, the same enzyme was isolated using different types of substrates and thus received alternative names, i.e., protease I for hydrolyzing *N*- acetyl- DL- phenylalanine- 2- naphthyl ester, and lysophospholipase $L_1$, for hydrolysing the 1- acyl group of lysophospholipid. Though the physiological role of TEP1 is unclear, it has been suggested to be potentially useful for the kinetic resolution of racemic mixtures of industrial chemicals.

From the wide variety of catalyzed reactions, the esterase ability of the TEP1 was examined, using the readily available substrate 4- nitrophenyl acetate (pNPA) . Truncated DocS was fused to the N- terminus of TEP1 (Doc (Δ16)-TEP1), expressed and purified on a CBM- Coh bound to beaded cellulose. A single band of about ~27 kDa, corresponding to the calculated size of the fusion protein, was observed in SDS- PAGE gels (FIG. 10A) . Two consecutive elutions were performed showing a similar single band displaying the high robustness of the purification system. The enzyme was active with a Vmax of 6.42 E- 06 $Ms^{-1}$ on pNPA and Km of 1.098 mM (FIG. 10B, curve fitted using the Graphpad prism program; table 4) .

**Table 4.** Kinetic parameters of the Doc(Δ16)-TEP1.

| | $V_{max}$ [M $s^{-1}$] | $K_m$ [mM] | $k_{cat}$ [$s^{-1}$] | $k_{cat}/K_m$ [$mM^{-1}$ $s^{-1}$] |
|---|---|---|---|---|
| ΔDoc-TEP1 | 6.42E-06 | 1.37 | 12.83 | 9.37 |

**References**

Barak, Y, Handelsman, T, Nakar, D, Mechaly, A, Lamed, R, Shoham, Y, Bayer, EA. 2005. J. Mol. Recogit. 18: 491-501.
Bayer, EA, Chanzy, H, Lamed, R, Shoham, Y. 1998. Curr. Opin. Struct. Biol. 8: 548-557.
Bayer, EA, Shimon, LJW, Lamed, R, Shoham, Y. 1998. J. Struct. Biol. 124: 221-234.
Bayer EA et al., FEBS Lett. 1999;463:277-80
Bayer, EA, Belaich, J-P, Shoham, Y, Lamed, R. 2004. Annu. Rev. Microbiol. 58: 521-554.
Craig, SJ, Foong, FC, Nordon, R. 2006. J Biotechnol 121: 165-173.
Ding S-Y, Bayer EA, Steiner D, Shoham Y, Lamed R. 1999. J. Bacteriol. 181: 6720-6729.
Ding S-Y, Bayer EA, Steiner D, Shoham Y, Lamed R. 2000. 182: 4915-4925.
Doi RH, Goldstein M, Hashida S, Park JS, Takagi M. Crit Rev Microbiol. 1994; 20 (2) : 87- 93
Fierobe HP, Pagès S, Bélaïch A, Champ S, Lexa D, Bélaïch JP. 1999 Sep 28; 38 (39) : 12822- 32
Gerngross UT, Romaniec MP, Kobayashi T, Huskisson NS, Demain AL. Mol Microbiol. 1993 Apr; 8 (2) : 325- 34. Erratum in: Mol Microbiol 1993 Dec; 10 (5) : 1155.
Gold and Martin. J Bacteriol. 2007; 189 (19) : 6787- 95.

Haimovitz, R, Barak, Y, Morag, E, Voronov-Goldman, M, Lamed, R, Bayer, EA. 2008. Proteomics 8: 968-979.
Handelsman, T, Barak, Y, Nakar, D, Mechaly, A, Lamed, R, Shoham, Y, Bayer, EA. 2004. FEBS Lett. 572: 195-200.
Jindou, S, Kajino, T, Inagaki, M, Karita, S, Beguin, P, Kimura, T, Sakka, K, Ohmiya, K. 2004. Biosci. Biotechnol. Biochem. 68: 924-926.
Kakiuchi M, Isui A, Suzuki K, Fujino T, Fujino E, Kimura T, Karita S, Sakka K, Ohmiya K. J Bacteriol. 1998, 180 (16) : 4303- 8
Karpol, A, Barak, Y, Lamed, R, Shoham, Y, Bayer, EA. 2008. Biochem. J. 410: 331-338.
Kirby J, Martin JC, Daniel AS, Flint HJ. 1997. FEMS Microbiol. Lett. 149: 213-219.
Lamed R, Setter E, Kenig R, Bayer EA. 1983. Biotechnol. Bioeng. Symp. 13: 163-181.
Lamed R, Naimark J, Morgenstern E, Bayer EA. J Bacteriol 1987, 169 (8) : 3792- 800
Mechaly, A, Yaron, S, Lamed, R, Fierobe, H-P, Belaich, A, Belaich, J-P, Shoham, Y, Bayer, EA. 2000. Proteins 39: 170-177.
Mechaly A, Fierobe HP, Belaich A, Belaich JP, Lamed R, Shoham Y, Bayer EA. J Biol Chem. 2001 Mar 30; 276 (13) : 9883- 8. Erratum in: J Biol Chem 2001 Jun 1; 276 (22) : 19678.
Nakar, D, Handelsman, T, Shoham, Y, Fierobe, H-P, Belaich, JP, Morag, E, Lamed, R, Bayer, EA. 2004. J Biol. Chem. 279: 42881-42888.
Ohara H, Karita S, Kimura T, Sakka K, Ohmiya K. Biosci Biotechnol Biochem. 2000, 64 (2) : 254- 60
Pagès, S, Belaich, A, Belaich, J-P, Morag, E, Lamed, R, Shoham, Y, Bayer, EA. 1997. Proteins 29: 517-527.
Pagès S, Belaich A, Tardif C, Reverbel-Leroy C, Gaudin C, Belaich JP. 1996. J Bacteriol 178: 2279-86
Pohlschröder M, Leschine SB, Canale- Parola E. J Bacteriol. 1994 Jan; 176 (1) : 70- 6.
Reichmann, D, Rahat, O, Cohen, M, Neuvirth, H, Schreiber, G. 2007. Curr. Opin. Struct. Biol. 17: 67-76.
Shoseyov O, Takagi M, Goldstein MA, Doi RH. Proc Natl Acad Sci USA. 1992 Apr 15; 89 (8) : 3483- 7.
Van den Ent, F., and Lowe, J., 2006, J Biochem Biophys Methods 67, 67-74.
Wang, WK, Kruus, K, Wu, JHD. 1993. J. Bacteriol. 175: 1293-1302.
Wu WY et al., Nat Protoc. 2006; 1 (5) : 2257- 62.
Yaron, S, Morag, E, Bayer, EA, Lamed, R, Shoham, Y. 1995. FEBS Lett. 360: 121-124.
Zverlov VV et al., Proteomics. 2005; 5:3646-53.

## Claims

1. An affinity purification system, comprising a solid substrate, a bound protein comprising a cohesin domain, a recombinant or synthetic polypeptide comprising a molecule of interest and a truncated dockerin polypeptide derived from a dockerin domain, wherein the truncated dockerin polypeptide comprises only one calcium binding motif.

**2.** The affinity purification system of claim 1,
wherein said solid substrate is selected from the group consisting of a bead, a cell, an extracellular matrix, and a container; or
wherein said solid substrate comprises cellulose and said protein bound to said solid substrate further comprises a carbohydrate-binding module (CBM); or
wherein said dockerin domain is a Type I dockerin domain; or
wherein said cohesin domain is a Type I cohesin domain; or
wherein said molecule of interest is a molecule other than a protein; or
wherein said molecule of interest is selected from the group consisting of a peptide, an enzyme, a hormone and an antibody, preferably wherein said molecule of interest is an enzyme other than xylanase; or
wherein said molecule of interest is an antibody-binding domain, and wherein said affinity purification system further comprises at least the antigen binding portion of an antibody bound to said antibody-binding domain; or
wherein said truncated dockerin polypeptide contains a 14-16 amino acid deletion N-terminal to the lysine residue at position 18 of the wild-type Type I dockerin domain sequence.

**3.** A recombinant or synthetic polypeptide comprising a molecule of interest and a truncated dockerin polypeptide derived from a dockerin protein domain, wherein the truncated dockerin polypeptide comprises only one calcium binding motif, wherein said truncated dockerin polypeptide contains a 14-16 amino acid deletion N-terminal to the lysine residue at position 18 or at position 50 of the wild-type Type I dockerin domain sequence.

**4.** The recombinant or synthetic polypeptide of claim 3,
wherein said molecule of interest is a molecule other than a protein; or
said truncated dockerin polypeptide further comprises an N-terminal glycine residue, wherein the glycine residue is attached directly to the truncated dockerin polypeptide; or
wherein the Type-I dockerin domain has the amino acid sequence as set forth in any one of SEQ ID NO:1, SEQ ID NO:35 - SEQ ID NO: 122, or an analog thereof having at least 70 % homology to SEQ ID NO: 1; or
wherein the calcium binding motif is in the first segment of the dockerin domain; or
wherein the calcium binding motif is in the second segment of the dockerin domain; or
wherein said truncated dockerin polypeptide is linked to said molecule of interest via a peptide bond; or
wherein said truncated dockerin polypeptide is linked to said molecule of interest via a linker peptide, preferably wherein the linker peptide is a cleavable linker peptide.

**5.** The recombinant or synthetic polypeptide of claim 3, wherein said molecule of interest is selected from the group consisting of a peptide, an enzyme, a hormone and an antibody.

**6.** The recombinant or synthetic polypeptide of claim 5,
wherein said molecule of interest is an enzyme other than xylanase; or
wherein said molecule of interest is a peptide, the truncated dockerin polypeptide is linked to the N-terminus of said peptide; or
wherein said molecule of interest is a peptide, the truncated dockerin polypeptide is linked to the C-terminus of said peptide.

**7.** The recombinant or synthetic polypeptide of claim 3, wherein the truncated dockerin polypeptide comprises the amino acid sequence as set forth in any one of SEQ ID NO: 2 - SEQ ID NO: 4 or an analog thereof having at least 70 % homology to SEQ ID NO: 1.

**8.** The recombinant or synthetic polypeptide of claim 7,
wherein the truncated dockerin polypeptide consists of the amino acid sequence as set forth in SEQ ID NO: 2; or
wherein the truncated dockerin polypeptide consists of the amino acid sequence as set forth in SEQ ID NO: 3; or
wherein the truncated dockerin polypeptide consists of the amino acid sequence as set forth in SEQ ID NO: 4.

**9.** A method of attaching a molecule of interest to a solid substrate, the method comprising the step of:

a) providing a solid substrate associated with a protein comprising a cohesin domain;
b) providing a molecule of interest covalently bound to a truncated dockerin polypeptide, wherein the truncated dockerin polypeptide comprises only one calcium binding motif;
c) allowing the truncated dockerin molecule to bind to the cohesin domain; thereby attaching a molecule of interest to a solid substrate.

**10.** The method of claim 9,
wherein said solid substrate is selected from the group consisting of a bead, a cell, an extracellular matrix and a container; or
wherein said solid substrate comprises cellulose and said protein associated with said solid substrate further comprises a carbohydrate- binding module (CBM); or
wherein said molecule of interest is a fusion peptide.

**11.** A method of purifying a molecule of interest, the method comprising the steps of:

a) providing a solid substrate associated with a protein comprising a cohesin domain;
b) providing a molecule of interest covalently bound to a truncated dockerin polypeptide, wherein the truncated dockerin polypeptide comprises only one calcium binding motif;
c) allowing the truncated dockerin molecule to bind to the cohesin domain; and
d) eluting the molecule of interest of (b); thereby purifying a molecule of interest.

**12.** The method of claim 11,
wherein said cohesin domain is a Type I cohesin domain; or
wherein said dockerin domain is a Type I dockerin domain; or
wherein said solid substrate is selected from the group consisting of a bead, a cell, an extracellular matrix and a container; or
wherein said solid substrate comprises cellulose and said protein associated with said solid substrate further comprises a carbohydrate- binding module (CBM); or
wherein the step of attaching a truncated dockerin molecule to the cohesin domain is performed in the presence of $Ca^{2+}$; or
wherein the step of eluting the molecule of interest is performed with a chelator of a divalent cation, preferably wherein the chelator is selected from the group consisting of EDTA and EGTA; or
wherein the molecule of interest is a fusion peptide, preferably wherein the molecule of interest is fused to the dockerin polypeptide via a cleavable linker peptide, the method further comprising the step of cleaving said cleavable linker peptide.

**13.** A method of purifying a molecule of interest, said method comprising the steps of contacting a solid substrate with the molecule of interest and
eluting said molecule of interest,
wherein said solid substrate comprises

(a) a protein bound thereto comprising a cohesin domain;
(b) an antibody-binding domain covalently bound to a truncated dockerin polypeptide comprising only one calcium binding motif; and
(c) at least the antigen binding portion of an antibody bound to the antibody- binding domain, wherein the antibody recognizes said molecule of interest.

**14.** A method of engineering a molecule of interest to be purified using a solid substrate, said method comprising the step of covalently binding said molecule of interest to a truncated dockerin polypeptide derived from a dockerin protein domain, wherein the truncated dockerin polypeptide comprises only one calcium binding motif , said truncated dockerin polypeptide contains a 14-16 amino acid deletion N-terminal to the lysine residue at position 18 or at position 50 of the wild-type Type I dockerin domain sequence, thereby engineering a molecule of interest to be purified using a affinity column.

**15.** An isolated truncated dockerin polypeptide derived from a dockerin domain, wherein the truncated dockerin polypeptide comprises only one calcium binding motif, said truncated dockerin polypeptide contains a 14-16 amino acid deletion N-terminal to the lysine residue at position 18 or at position 50 of the wild-type Type I dockerin domain sequence.

**16.** The isolated truncated dockerin polypeptide of claim 15,
wherein the Type-I dockerin domain comprises the amino acid sequence as set forth in any one of SEQ ID NO: 1, SEQ ID NO : 35 - SEQ ID NO: 122, or an analog thereof comprising at least 70% homology to SEQ ID NO: 1; or
wherein the calcium binding motif is in the first segment of the dockerin domain, preferably comprising the amino acid sequence as set forth in SEQ ID NO:4; or

wherein the dockerin domain is from a thermophilic microorganism; or
wherein the thermophilic microorganism is selected from a group consisting of *Clostridium thermocellum* and *Archaeoglobus fulgidus*; or
wherein the calcium binding motif is in the second segment of the dockerin domain, preferably comprising the amino acid sequence as set forth in any one of SEQ ID NO: 2 - SEQ ID NO: 3; or
wherein the calcium binding motif is in the second segment of the dockerin domain, consisting of the amino acid sequence as set forth in SEQ ID NO: 2; or
wherein the calcium binding motif is in the second segment of the dockerin domain, consisting of the amino acid sequence as set forth in SEQ ID NO: 3.

**Patentansprüche**

1. Affinitätsreinigungssystem umfassend ein festes Substrat, ein gebundenes Protein umfassend eine Cohesin-Domäne, ein rekombinantes oder synthetisches Polypeptid umfassend ein Molekül von Interesse und ein verkürztes Dockerin-Polypeptid abgeleitet von einer Dockerin-Domäne, worin das verkürzte Dockerin-Polypeptid lediglich ein Calcium-Bindemotiv umfasst.

2. Affinitätsreinigungssystem nach Anspruch 1,
worin das feste Substrat ausgewählt ist aus der Gruppe bestehend aus einem Bead, einer Zelle, einer extrazellulären Matrix und einem Container, oder
worin das feste Substrat Cellulose umfasst und das an das feste Substrat gebundene Protein ferner ein Kohlenwasserstoffbindungsmodul (CBM) umfasst, oder
worin die Dockerin-Domäne eine Typ-I Dockerin-Domäne ist, oder
worin die Cohesin-Domäne eine Typ-I Cohesin-Domäne ist, oder
worin das Molekül von Interesse ein von einem Protein verschiedenes Molekül ist, oder
worin das Molekül von Interesse ausgewählt ist aus der Gruppe bestehend aus einem Peptid, einem Enzym, einem Hormon und einem Antikörper, vorzugsweise worin das Molekül von Interesse ein von Xylanase verschiedenes Enzym ist, oder
worin das Molekül von Interesse eine Antikörper-Bindedomäne ist und worin das Affinitätsreinigungssystem ferner mindestens den Antigenbindebereich eines Antikörpers umfasst, der an der Antikörper-Bindedomäne gebunden vorliegt, oder
worin das verkürzte Dockerin-Polypeptid eine 14-16 Aminosäurendeletion N-terminal vom Lysinrest an Position 18 der Wildtyp Typ-I Dockerin-Domänensequenz enthält.

3. Rekombinantes oder synthetisches Polypeptid umfassend ein Molekül von Interesse und ein verkürztes Dockerin-Polypeptid abgeleitet von einer Dockerin-Proteindomäne, worin das verkürzte Dockerin-Polypeptid lediglich ein Calcium-Bindemotiv umfasst, worin das verkürzte Dockerin-Polypeptid eine 14-16 Aminosäurendeletion N-terminal vom Lysinrest an Position 18 oder an Position 50 der Wildtyp Typ-I Dockerin-Domänensequenz enthält.

4. Rekombinantes oder synthetisches Polypeptid nach Anspruch 3,
worin das Molekül von Interesse ein von einem Protein verschiedenes Molekül ist, oder
das verkürzte Dockerin-Polypeptid ferner einen N-terminalen Glycerinrest umfasst, worin der Glycerinrest direkt mit dem verkürzten Dockerin-Polypeptid verbunden ist, oder
worin die Typ-I Dockerin-Domäne die Aminosäuresequenz wie in einer von SEQ ID NO:1, SEQ ID NO:35 bis SEQ ID NO:122 aufgeführt oder wie in einem Analog davon mit wenigstens 70%iger Homologie zu SEQ ID NO:1 aufweist, oder
worin das Calcium-Bindemotiv in dem ersten Segment der Dockerin-Domäne vorliegt, oder
worin das Calcium-Bindemotiv in dem zweiten Segment der Dockerin-Domäne vorliegt, oder
worin das verkürzte Dockerin-Polypeptid mit dem Molekül von Interesse über eine Peptidbindung verbunden ist, oder
worin das verkürzte Dockerin-Polypeptid mit dem Molekül von Interesse über ein Linker-Peptid verbunden ist, vorzugsweise worin das Linker-Peptid ein spaltbares Linker-Peptid ist.

5. Rekombinantes oder synthetisches Polypeptid nach Anspruch 3, worin das Molekül von Interesse ausgewählt ist aus der Gruppe bestehend aus einem Peptid, einem Enzym, einem Hormon und einem Antikörper.

6. Rekombinantes oder synthetisches Polypeptid nach Anspruch 5,
worin das Molekül von Interesse ein von Xylanase verschiedenes Enzym ist, oder

worin das Molekül von Interesse ein Peptid ist und das verkürzte Dockerin-Polypeptid mit dem N-Terminus des Peptids verbunden ist, oder
worin das Molekül von Interesse ein Peptid ist und das verkürzte Dockerin-Polypeptid mit dem C-Terminus des Peptids verbunden ist.

**7.** Rekombinantes oder synthetisches Polypeptid nach Anspruch 3, worin das verkürzte Dockerin-Polypeptid die Aminosäuresequenz wie in einer von SEQ ID NO:2 bis SEQ ID NO:4 aufgeführt oder wie in einem Analog davon mit wenigstens 70%iger Homologie zu SEQ ID NO:1 aufweist.

**8.** Rekombinantes oder synthetisches Polypeptid nach Anspruch 7,
worin das verkürzte Dockerin-Polypeptid aus der Aminosäuresequenz wie in SEQ ID NO:2 aufgeführt besteht, oder
worin das verkürzte Dockerin-Polypeptid aus der Aminosäuresequenz wie in SEQ ID NO:3 aufgeführt besteht, oder
worin das verkürzte Dockerin-Polypeptid aus der Aminosäuresequenz wie in SEQ ID NO:4 aufgeführt besteht, oder

**9.** Verfahren zum Befestigen eines Moleküls von Interesse an einem festen Substrat, wobei das Verfahren die Schritte umfasst:

a) Bereitstellen eines festen Substrats, das mit einem Protein umfassend eine Cohesin-Domäne verbunden ist,
b) Bereitstellen eines Moleküls von Interesse, das mit einem verkürzten Dockerin-Polypeptid kovalent verbunden ist, wobei das verkürzte Dockerin-Polypeptid lediglich ein Calcium-Bindemotiv umfasst,
c) Ermöglichen einer Bindung des verkürzten Dockerin-Polypeptids an die Cohesin-Domäne, dabei Befestigen eines Moleküls von Interesse an einem festen Substrat.

**10.** Verfahren nach Anspruch 9,
wobei das feste Substrat ausgewählt ist aus der Gruppe bestehend aus einem Bead, einer Zelle, einer extrazellulären Matrix und einem Container, oder
wobei das feste Substrat Cellulose umfasst und das an das feste Substrat gebundene Protein ferner ein Kohlenwasserstoffbindungsmodul (CBM) umfasst, oder
wobei das Molekül von Interesse ein Fusionspeptid ist.

**11.** Verfahren zur Aufreinigung eines Moleküls von Interesse, wobei das Verfahren die Schritte umfasst:

a) Bereitstellen eines festen Substrats, das mit einem Protein umfassend eine Cohesin-Domäne verbunden ist,
b) Bereitstellen eines Moleküls von Interesse, das mit einem verkürzten Dockerin-Polypeptid kovalent verbunden ist, wobei das verkürzte Dockerin-Polypeptid lediglich ein Calcium-Bindemotiv umfasst,
c) Ermöglichen einer Bindung des verkürzten Dockerin-Moleküls an die Cohesin-Domäne, und
d) Eluieren des Moleküls von Interesse aus b), dabei Aufreinigen eines Moleküls von Interesse.

**12.** Verfahren nach Anspruch 11,
wobei die Cohesin-Domäne eine Typ-I Cohesin-Domäne ist, oder
wobei die Dockerin-Domäne eine Typ-I Dockerin-Domäne ist, oder
wobei das feste Substrat ausgewählt ist aus der Gruppe bestehend aus einem Bead, einer Zelle, einer extrazellulären Matrix und einem Container, oder
wobei das feste Substrat Cellulose umfasst und das an das feste Substrat gebundene Protein ferner ein Kohlenwasserstoffbindungsmodul (CBM) umfasst, oder
wobei der Schritt des Bindens eines verkürzten Dockerin-Moleküls an die Cohesin-Domäne in Gegenwart von $Ca^{2+}$ durchgeführt wird, oder
wobei der Schritt des Eluierens des Moleküls von Interesse mit einer chelatbildenden Verbindung eines zweiwertigen Kations durchgeführt wird, vorzugsweise wobei die chelatbildende Verbindung ausgewählt ist aus der Gruppe bestehend aus EDTA und EGTA, oder
wobei das Molekül von Interesse ein Fusionspeptid ist, vorzugsweise wobei das Molekül von Interesse an dem Dockerin-Polypeptid über ein spaltbares Linkerpeptid fusioniert ist, wobei das Verfahren ferner den Schritt des Spaltens des spaltbaren Linkerpeptids umfasst.

**13.** Verfahren zur Aufreinigung eines Moleküls von Interesse, wobei das Verfahren die Schritte umfasst:

Inkontaktbringen eines festen Substrats mit dem Molekül von Interesse und
Eluieren des Moleküls von Interesse,

wobei das feste Substrat umfasst:

(a) ein daran gebundenes Protein umfassend eine Cohesin-Domäne,
(b) eine an ein verkürztes Dockerin-Polypeptid umfassend lediglich ein Calcium-Bindemotiv kovalent gebundene Antikörperbindungsdomäne, und
(c) wenigstens den Antigenbindebereich eines Antikörpers, der an der Antikörper-Bindedomäne gebunden vorliegt, wobei der Antikörper das Molekül von Interesse erkennt.

**14.** Verfahren zur Herstellung eines Moleküls von Interesse, das unter Verwendung eines festen Substrats aufgereinigt werden soll, wobei das Verfahren den Schritt des kovalenten Bindens des Moleküls von Interesse an ein verkürztes Dockerin-Polypeptid abgeleitet von einer Dockerinprotein-Domäne umfasst, wobei das verkürzte Dockerin-Polypeptid lediglich ein Calcium-Bindemotiv umfasst, wobei das verkürzte Dockerin-Polypeptid eine 14-16 Aminosäurendeletion N-terminal von dem Lysinrest an Position 18 oder an Position 50 der Wildtyp Typ-I Dockerin-Domänensequenz enthält, dabei Erzeugen eines Moleküls von Interesse, das unter Verwendung einer Affinitätssäule aufgereinigt werden soll.

**15.** Isoliertes verkürztes Dockerin-Polypeptid abgeleitet von einer Dockerin-Domäne, worin das verkürzte Dockerin-Polypeptid lediglich ein Calcium-Bindemotiv umfasst, worin das verkürzte Dockerin-Polypeptid eine 14-16 Aminosäurendeletion N-terminal von dem Lysinrest an Position 18 oder an Position 50 der Wildtyp Typ-I Dockerin-Domänensequenz enthält.

**16.** Isoliertes verkürztes Dockerin-Polypeptid nach Anspruch 15,
worin die Typ-I Dockerin-Domäne die Aminosäuresequenz wie in einer von SEQ ID NO:1, SEQ ID NO:35 bis SEQ ID NO:122 aufgeführt oder wie in einem Analog davon mit wenigstens 70%iger Homologie zu SEQ ID NO:1 aufweist, oder
worin das Calcium-Bindemotiv in dem ersten Segment der Dockerin-Domäne vorliegt, vorzugsweise umfassend die in SEQ ID NO:4 aufgeführte Aminosäuresequenz, oder
worin die Dockerin-Domäne von einem thermophilen Mikroorganismus stammt, oder
worin der thermophile Mikroorganismus ausgewählt ist aus der Gruppe bestehend aus *Clostridium thermocellum* und *Archaeoglobus fulgidus*, oder
worin das Calcium-Bindemotiv in dem zweiten Segment der Dockerin-Domäne vorliegt, vorzugsweise umfassend die in einer von SEQ ID NO:2 bis SEQ ID NO:3 aufgeführte Aminosäuresequenz, oder
worin das Calcium-Bindemotiv in dem zweiten Segment der Dockerin-Domäne vorliegt, bestehend aus der in SEQ ID NO:2 aufgeführten Aminosäuresequenz, oder
worin das Calcium-Bindemotiv in dem zweiten Segment der Dockerin-Domäne vorliegt, bestehend aus der in SEQ ID NO:3 aufgeführten Aminosäuresequenz.

**Revendications**

**1.** Un système de purification par affinité comprenant un substrat solide, une protéine liée comprenant un domaine cohésine, un polypeptide recombinant ou synthétique comprenant une molécule d'intérêt et un polypeptide de dockerine tronquée dérivé d'un domaine dockerine, dans lequel le polypeptide de dockerine tronquée comprend un seul motif de liaison de calcium.

**2.** Le système de purification par affinité de la revendication 1,
dans lequel ledit substrat solide est choisi à partir du groupe consistant en une bille, une cellule, une matrice extracellulaire et un récipient. ; ou
dans lequel ledit substrat solide comprend de la cellulose et ladite protéine liée audit substrat solide comprend en outre un module de liaison d'hydrate de carbone(CBM) ; ou
dans lequel ledit domaine dockerine est un domaine dockerine de Type I ; ou
dans lequel ledit domaine cohésine est un domaine cohésine de Type I ; ou
dans lequel ladite molécule d'intérêt est une molécule autre qu'une protéine ; ou
dans lequel ladite molécule d'intérêt est choisie à partir du groupe consistant en un peptide, une enzyme, une hormone et un anticorps, de préférence dans lequel ladite molécule d'intérêt est une enzyme autre que la xylanase ; ou
dans lequel ladite molécule d'intérêt est un domaine de liaison anticorps et dans lequel ledit système de purification par affinité comprend en outre au moins la partie de liaison antigène d'un anticorps lié audit domaine de liaison aux anticorps ; ou
dans lequel ledit polypeptide de dockerine tronquée contient une délétion de 14 à 16 acides aminés N-terminaux du résidu lysine en position 18 de la séquence du domaine dockerine de type sauvage de Type I.

**3.** Un polypeptide recombinant ou synthétique comprenant une molécule d'intérêt et un polypeptide de dockerine tronquée dérivé d'un domaine protéique de dockerine, dans lequel le polypeptide de dockerine tronquée comprend un seul motif de liaison de calcium, dans lequel ledit polypeptide de dockerine tronquée contient une délétion de 14 à 16 acides aminés N-terminaux du résidu lysine en position 18 ou en position 50 de la séquence de domaine de dockerine de type sauvage de Type I.

**4.** Le polypeptide recombinant ou synthétique de la revendication 3,
dans lequel ladite molécule d'intérêt est une molécule autre qu'une protéine ; ou
ledit polypeptide de dockerine tronquée comprend en outre un résidu glycine N-terminal dans lequel le résidu de glycine est directement attaché au polypeptide de dockerine tronquée ; ou dans lequel le domaine dockerine de Type I comporte la séquence d'acides aminés telle qu'indiquée dans l'une de SEQ ID NO: 1, SEQ ID NO: 35 - SEQ ID NO: 122 ou un de leurs analogues ayant au moins 70 % d'homologie avec SEQ ID NO: 1 ; ou
dans lequel le motif de liaison de calcium est dans le premier segment du domaine dockerine ; ou dans lequel le motif de liaison de calcium est dans le second segment du domaine dockerine ; ou dans lequel ledit polypeptide de dockerine tronquée est lié à ladite molécule d'intérêt par l'intermédiaire d'une liaison peptidique ; ou
dans lequel ledit polypeptide de dockerine tronquée est lié à ladite molécule d'intérêt par l'intermédiaire d'une liaison peptidique, de préférence dans lequel le peptide de liaison est un peptide de liaison clivable.

**5.** Le polypeptide recombinant ou synthétique de la revendication 3, dans lequel ladite molécule d'intérêt est choisi à partir du groupe consistant en un peptide, une enzyme, une hormone et un anticorps.

**6.** Le polypeptide recombinant ou synthétique de la revendication 5,
dans lequel ladite molécule d'intérêt est une enzyme autre que la xylanase ; ou
dans lequel ladite molécule d'intérêt est un peptide, le polypeptide de dockerine tronquée est liée à l'extrémité N-terminale dudit peptide ; ou
dans lequel ladite molécule d'intérêt est un peptide, le polypeptide de dockerine tronquée est lié à l'extrémité C-terminale dudit peptide.

**7.** Le polypeptide recombinant ou synthétique de la revendication 3, dans lequel le polypeptide de dockerine tronquée comprend la séquence d'acides aminés telle qu'indiquée dans l'une de SEQ ID NO: 2 - SEQ ID NO: 4 ou un de leurs analogues ayant au moins 70 % d'homologie avec SEQ ID NO:1.

**8.** Le polypeptide recombinant ou synthétique de la revendication 7, dans lequel le polypeptide de dockerine tronquée consiste en la séquence d'acides aminés telle qu'indiquée dans SEQ ID NO:2 ; ou
dans lequel le polypeptide de dockerine tronquée consiste en la séquence d'acides aminés telle qu'indiquée dans SEQ ID NO: 3 ; ou
dans lequel le polypeptide de dockerine tronquée consiste en la séquence d'acides aminés telle qu'indiquée dans SEQ ID NO: 4.

**9.** Un procédé de fixation d'une molécule d'intérêt à un substrat solide, le procédé comprenant l'étape de:

a) fournir un substrat solide associé à une protéine comprenant un domaine cohésine ;
b) fournir une molécule d'intérêt liée de manière covalente à un polypeptide de dockerine tronquée, dans lequel le polypeptide de dockerine tronquée comprend un seul motif de liaison de calcium ;
c) laisser la molécule de dockerine tronquée de se lier au domaine cohésine, fixant ainsi une molécule d'intérêt à un substrat solide.

**10.** Le procédé de la revendication 9,
dans lequel ledit substrat solide est choisi à partir du groupe consistant en une bille, une cellule, une matrice extracellulaire et un récipient ; ou
dans lequel ledit substrat solide comprend de la cellulose et ladite protéine associée audit substrat solide comprend en outre un module de liaison d'hydrate de carbone (CBM) ; ou
dans lequel ladite molécule d'intérêt est un peptide de fusion.

**11.** Un procédé de purification d'une molécule d'intérêt, le procédé comprenant les étapes de:

a) fournir un substrat solide associé à une protéine comprenant un domaine cohésine ;
b) fournir une molécule d'intérêt liée par covalence à un polypeptide de dockerine tronquée dans lequel le polypeptide de dockerine tronquée comprend un seul motif de liaison de calcium ;
c) laisser la molécule de dockerine tronquée de se lier au domaine cohésine ; et
d) éluer la molécule d'intérêt de (b), purifiant de ce fait une molécule d'intérêt.

**12.** Le procédé de la revendication 11,
dans lequel ledit domaine cohésine est un domaine cohésine de Type I; ou
dans lequel ledit domaine dockerine est un domaine dockerine de Type I; ou
dans lequel ledit substrat solide est choisi à partir du groupe consistant en une bille, une cellule, une matrice extracellulaire et un récipient ; ou
dans lequel ledit substrat solide comprend de la cellulose et ladite protéine associée avec ledit substrat solide comprend en outre un module de liaison d'hydrate de carbone(CBM) ; ou
dans lequel l'étape de fixation d'une molécule de dockerine tronquée au domaine cohésine est effectuée en présence de $Ca^{2+}$ ; ou
dans lequel l'étape d'élution de la molécule d'intérêt est effectuée avec un agent de chélation d'un cation bivalent, de préférence dans lequel l'agent de chélation est choisi à partir du groupe consistant en EDTA et EGTA ; ou
dans lequel la molécule d'intérêt est un peptide de fusion, de préférence dans lequel la molécule d'intérêt est fusionnée au polypeptide par l'intermédiaire d'un peptide de liaison de dockerine clivable, le procédé comprenant en outre l'étape consistant à cliver ledit peptide de liaison clivable.

**13.** Procédé de purification d'une molécule d'intérêt, ledit procédé comprenant les étapes de mettre en contact un substrat solide avec la molécule d'intérêt et éluer ladite molécule d'intérêt, dans lequel ledit substrat solide comprend

a) une protéine liée à ce dernier comprenant un domaine cohésine ;
b) un domaine de liaison anticorps lié par covalence à un polypeptide de dockerine tronquée comprenant un seul motif de liaison calcium ; et
c) au moins la portion de liaison antigène d'un anticorps lié au domaine de liaison anticorps dans lequel l'anticorps reconnaît ladite molécule d'intérêt.

**14.** Procédé d'ingénierie d'une molécule d'intérêt à purifier à l'aide d'un substrat solide, ledit procédé comprenant l'étape consistant à lier par covalence ladite molécule d'intérêt à un polypeptide de dockerine tronquée dérivé d'un domaine protéique dockerine, dans lequel le polypeptide de dockerine tronquée comporte un seul motif liaison de calcium, ledit polypeptide de dockerine tronquée contient une délétion de 14 à 16 acides aminés N-terminaux du résidu lysine en position 18 ou en position 50 de la séquence de domaine de dockerine de type sauvage de Type I, façonnant de ce fait une molécule d'intérêt à purifier sur une colonne d'affinité.

**15.** Un polypeptide isolé de dockerine tronquée dérivé d'un domaine dockerine, dans lequel le polypeptide de dockerine tronquée comprend un seul motif de liaison de calcium, ledit polypeptide de dockerine tronquée contient une délétion de 14 à 16 acides aminés N-terminaux du résidu lysine en position 18 ou en position 50 de la séquence de domaine de dockerine de type sauvage de Type I.

**16.** Le polypeptide selon la revendication 15,
dans lequel le domaine dockerine de Type I comprend la séquence d'acides aminés telle qu'indiquée dans l'une de SEQ ID NO: 1, SEQ ID NO: 35 - SEQ ID NO: 122 ou un de ses analogues ayant au moins 70 % d'homologie avec SEQ ID NO :1 ; ou
dans lequel le motif de liaison de calcium est dans le premier segment du domaine dockerine comprenant de préférence la séquence d'acides aminés telle que figurant dans SEQ ID NO : 4; ou dans lequel le domaine de dockerine provient d'un microorganisme thermophile ; ou
dans lequel le microorganisme thermophile est choisi à partir du groupe consistant en *Clostridium thermocellum et Archaeoglobusfulgidus* ; ou
dans lequel le motif de liaison calcium est dans le second segment du domaine dockerine, comprenant de préférence la séquence d'acides aminés telle qu'indiquée dans SEQ ID NO :2 - SEQ ID NO :3; ou
dans lequel le motif de liaison de calcium de dockerine tronquée est dans le second segment du domaine de dockerine consistant en la séquence d'acides aminés telle qu'indiquée dans SEQ ID NO : 2 ; ou
dans lequel le motif de liaison calcium de dockerine tronquée est dans le second segment du domaine de dockerine consistant en la séquence d'acides aminés telle qu'indiquée dans SEQ ID NO : 3.

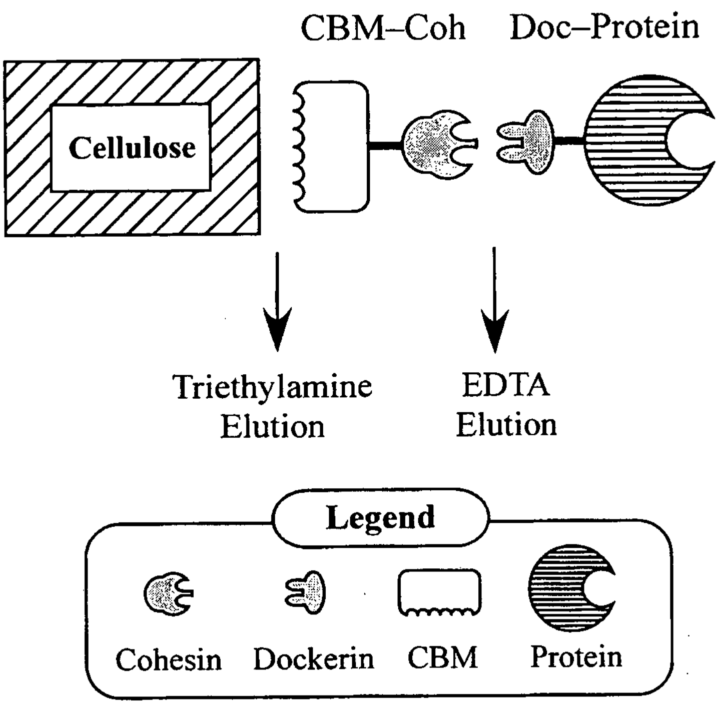
CBM–Coh
Doc–Protein
Cellulose
Triethylamine Elution
EDTA Elution
Legend
Cohesin
Dockerin
CBM
Protein

FIG. 1

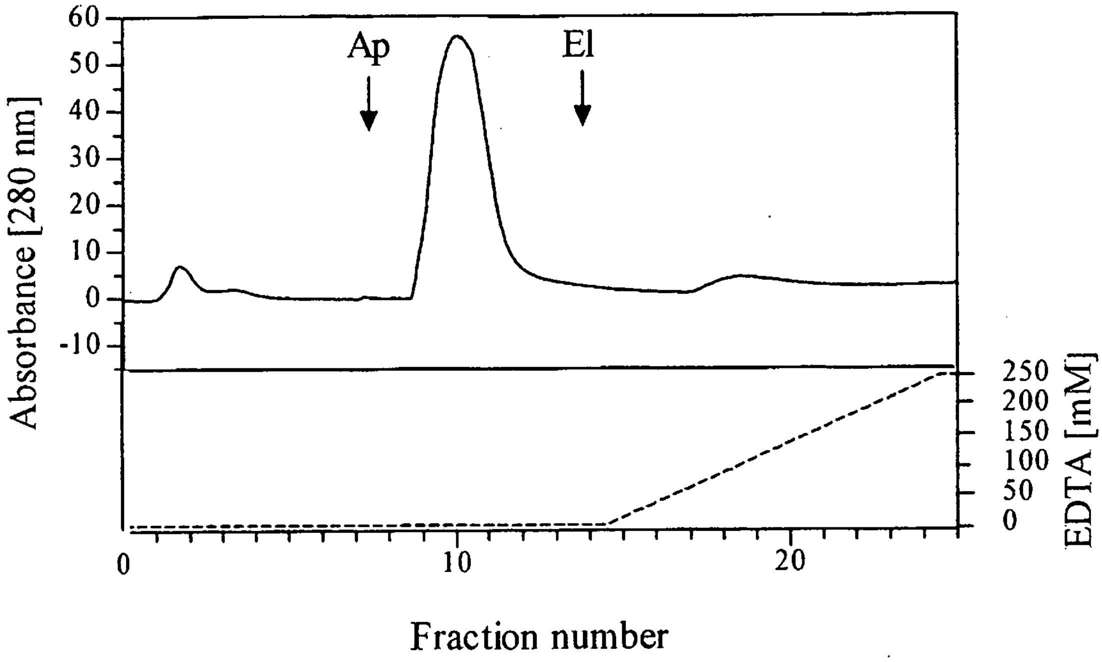
Absorbance [280 nm]
60
50
40
30
20
10
0
-10
Ap
El
250
200
150
100
50
0
EDTA [mM]
0
10
20
Fraction number

FIG. 2A

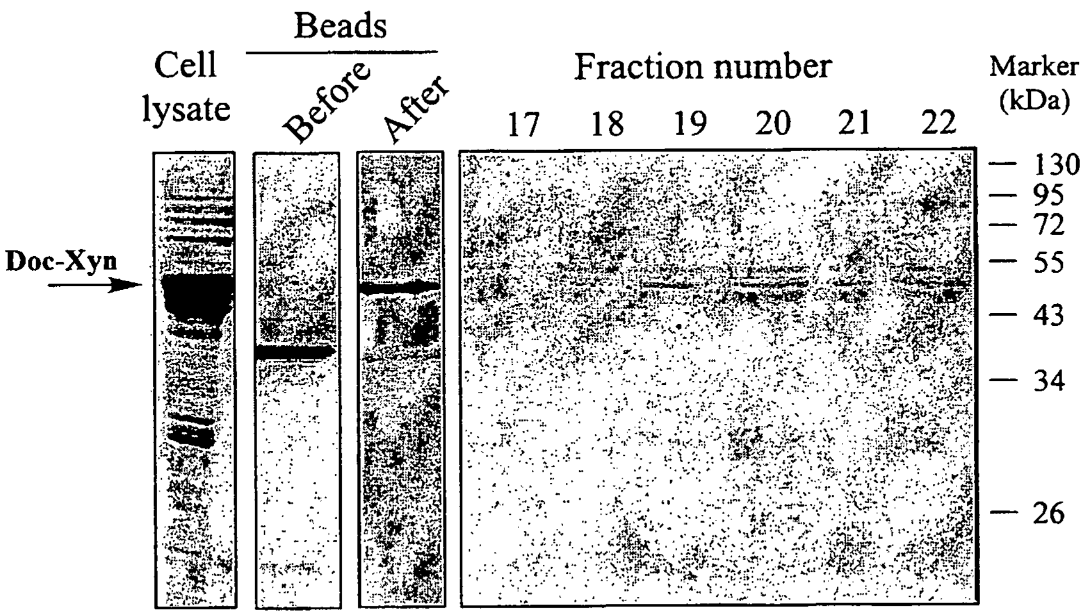
Beads
Cell lysate
Before
After
Fraction number
17
18
19
20
21
22
Marker (kDa)
Doc-Xyn
130
95
72
55
43
34
26

FIG. 2B

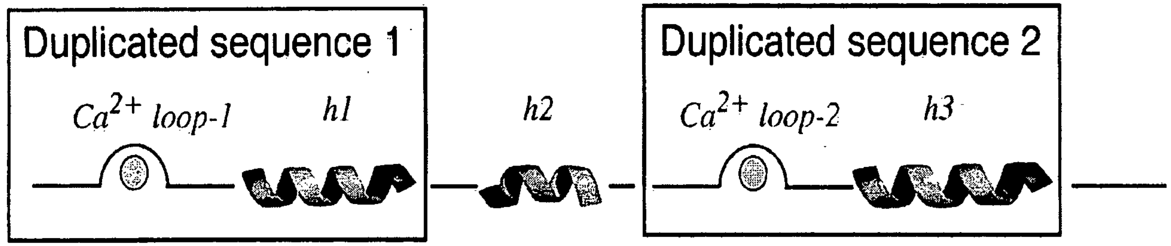


```
                1         2         3         4         5         6
         1234567890123456789012345678901234567890123456789012345678901234
         ................................................................
Doc WT   GDVNDDGKVNSTDAVALKRYVLRSGISINTDNADLNEDGRVNSTDLGILKRYILKEIDTLPYKN SEQ ID NO:1
```

**Deletions into N-terminus**

```
Doc (Δ16)  G---------------KRYVLRSGISINTDNADLNEDGRVNSTDLGILKRYILKEIDTLPYKN SEQ ID NO:2
Doc (Δ18)  G-----------------YVLRSGISINTDNADLNEDGRVNSTDLGILKRYILKEIDTLPYKN SEQ ID NO:148
Doc (Δ20)  G-------------------LRSGISINTDNADLNEDGRVNSTDLGILKRYILKEIDTLPYKN SEQ ID NO:149
Doc (Δ22)  G---------------------SGISINTDNADLNEDGRVNSTDLGILKRYILKEIDTLPYKN SEQ ID NO:150
Doc (Δ24)  G-----------------------ISINTDNADLNEDGRVNSTDLGILKRYILKEIDTLPYKN SEQ ID NO:151
Doc (Δ26)  G-------------------------INTDNADLNEDGRVNSTDLGILKRYILKEIDTLPYKN SEQ ID NO:152
```

FIG. 3A

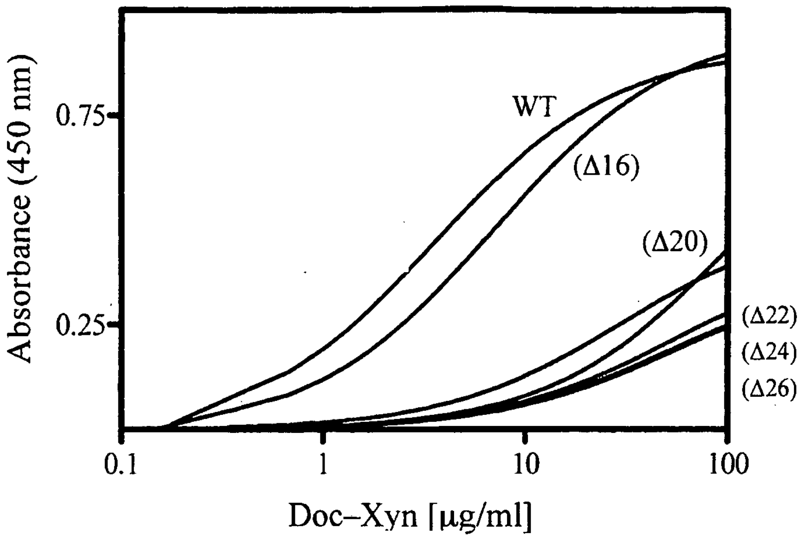


FIG. 3B

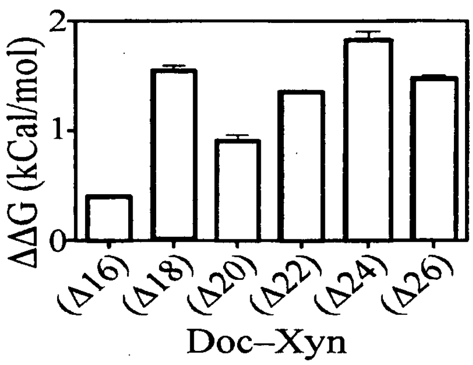


FIG. 3C

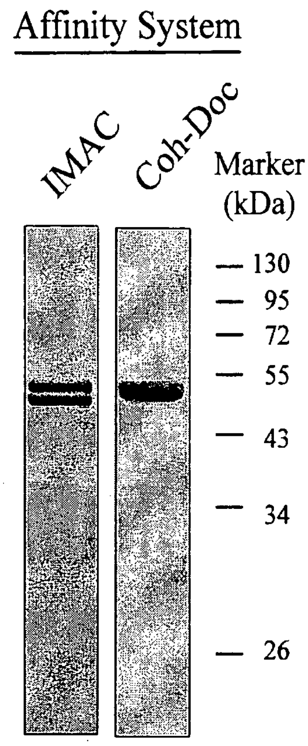


FIG. 3D

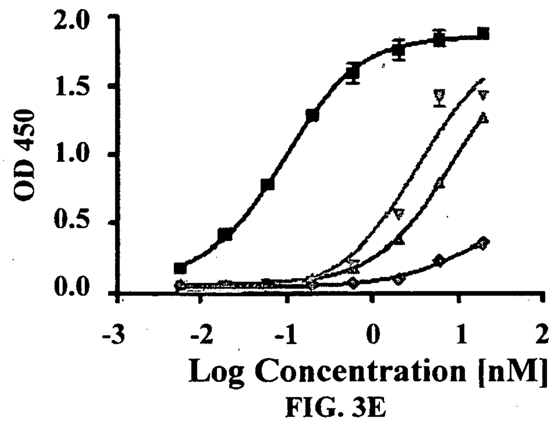


FIG. 3E

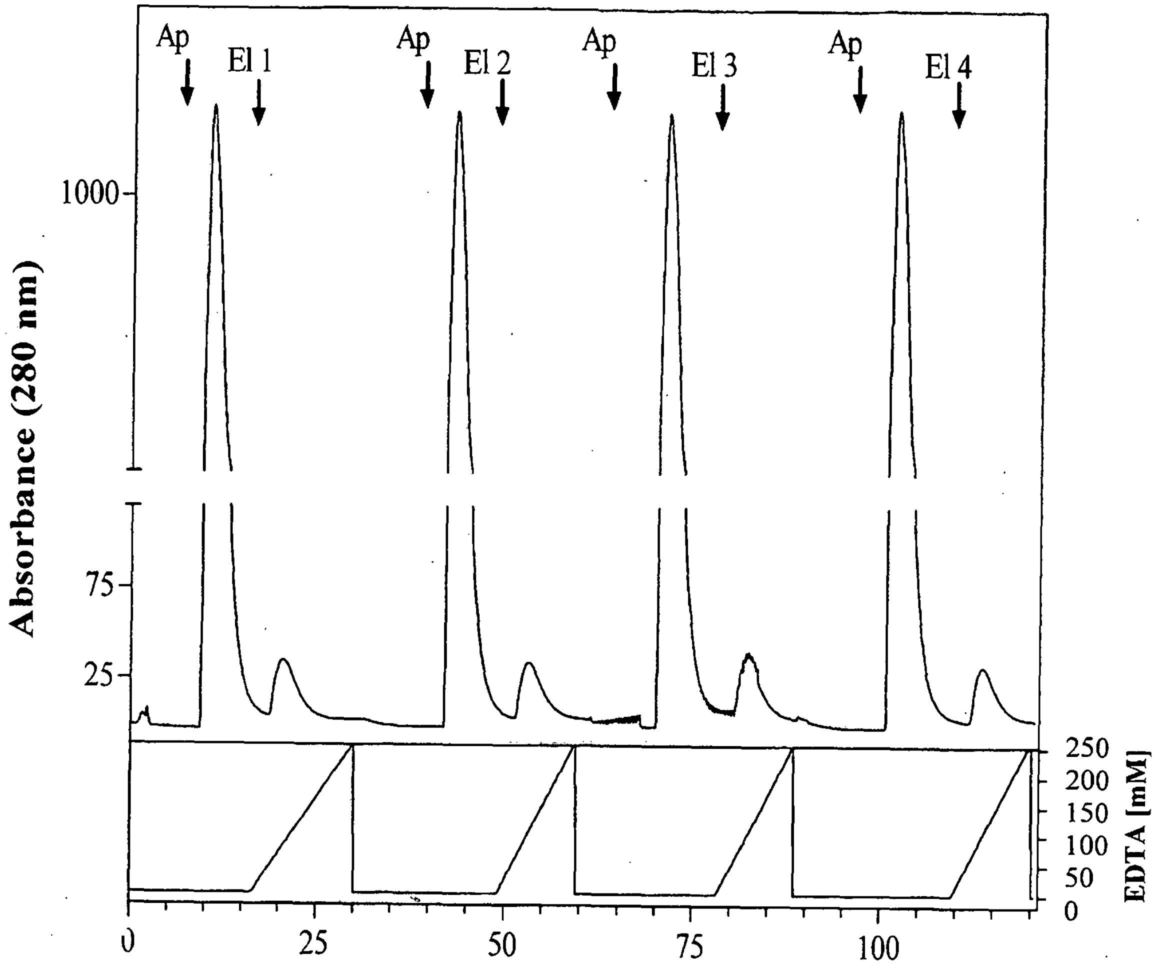
Ap
El 1
Ap
El 2
Ap
El 3
Ap
El 4
Absorbance (280 nm)
1000
75
25
250
200
150
100
50
0
EDTA [mM]
0
25
50
75
100

FIG. 4A

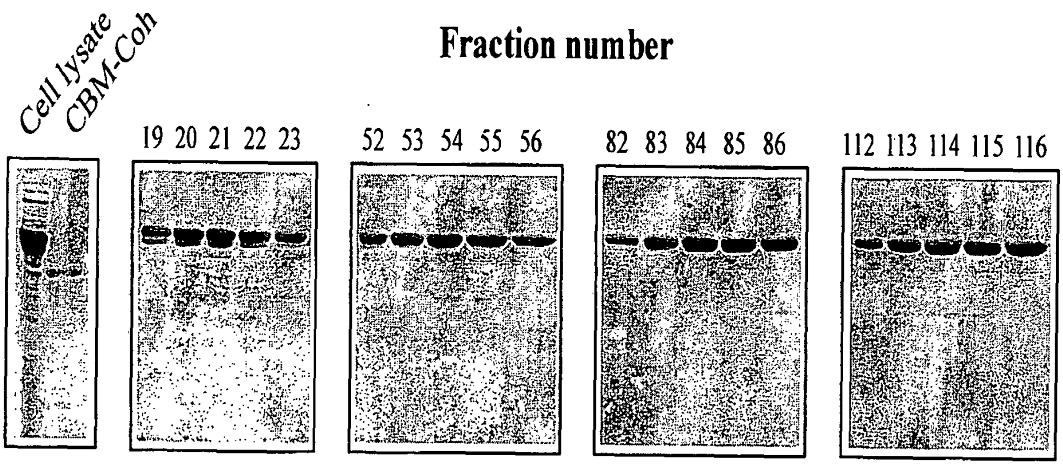
Fraction number
Cell lysate
CBM-Coh
19 20 21 22 23
52 53 54 55 56
82 83 84 85 86
112 113 114 115 116

FIG. 4B

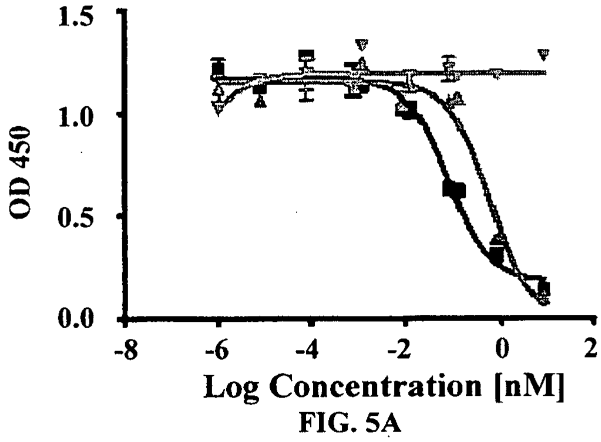
1.5
1.0
0.5
0.0
OD 450
-8
-6
-4
-2
0
2
Log Concentration [nM]
FIG. 5A

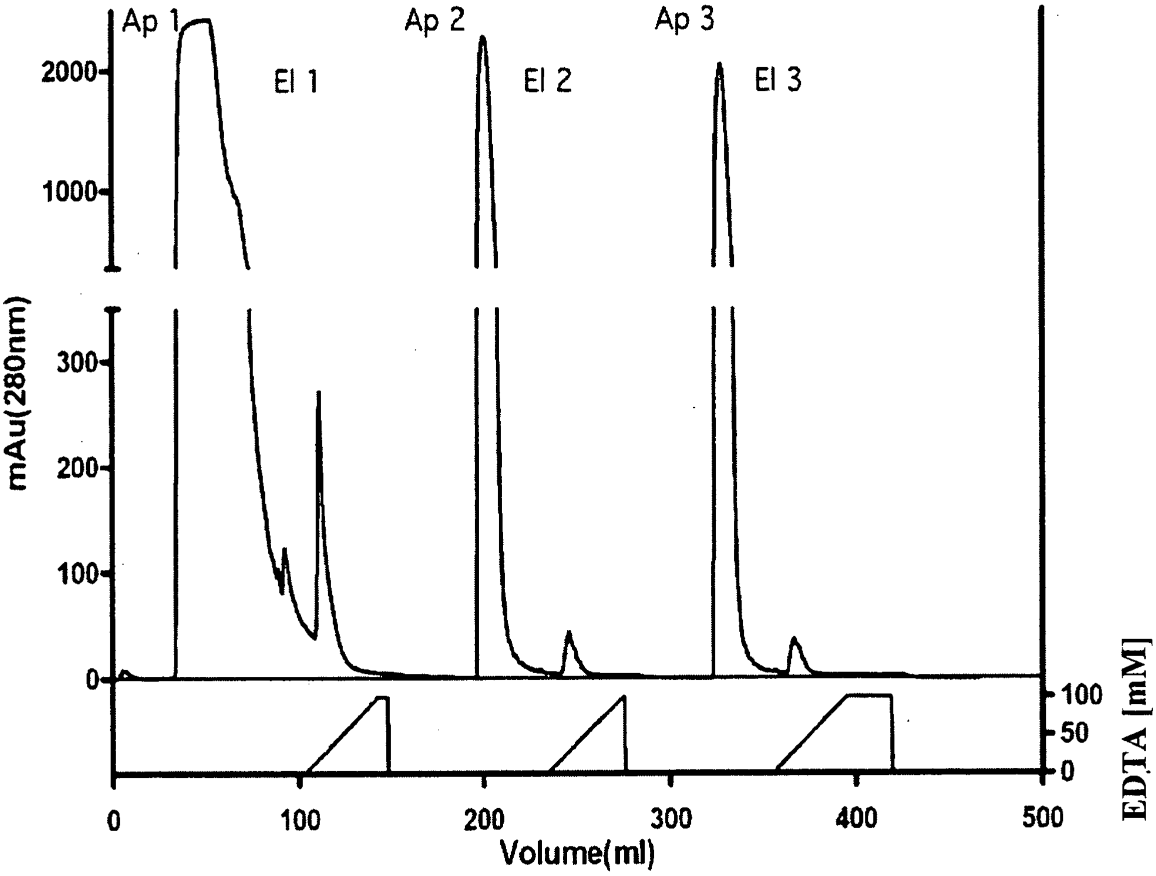
Ap 1
Ap 2
Ap 3
El 1
El 2
El 3
2000
1000
300
200
100
0
mAu(280nm)
100
50
0
EDTA [mM]
0
100
200
300
400
500
Volume(ml)
FIG. 5B

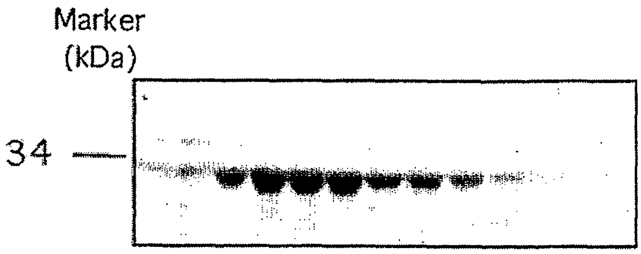


FIG. 5C 1

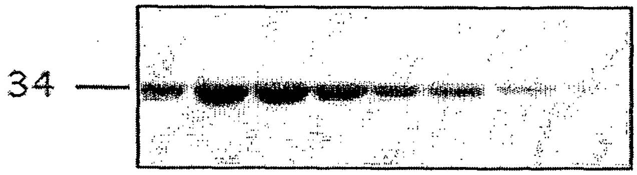


FIG. 5C 2

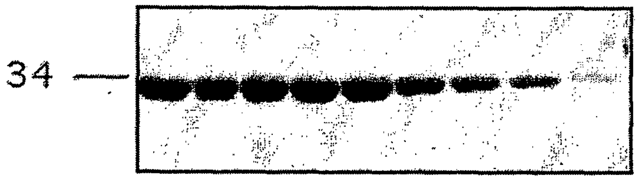


FIG. 5C 3

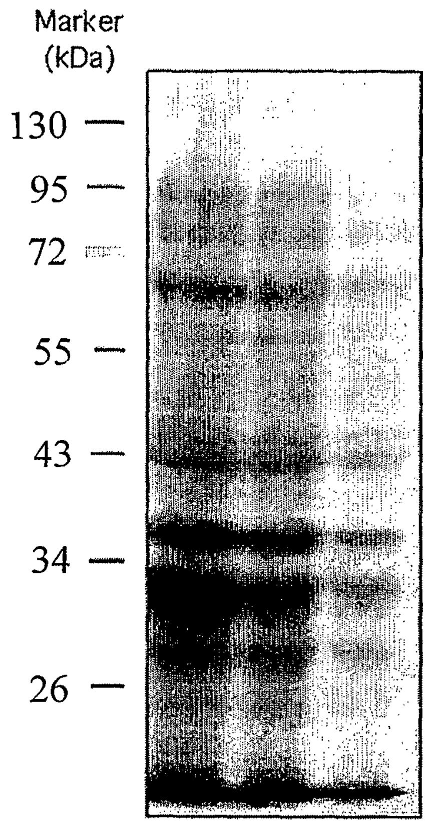


FIG. 5D

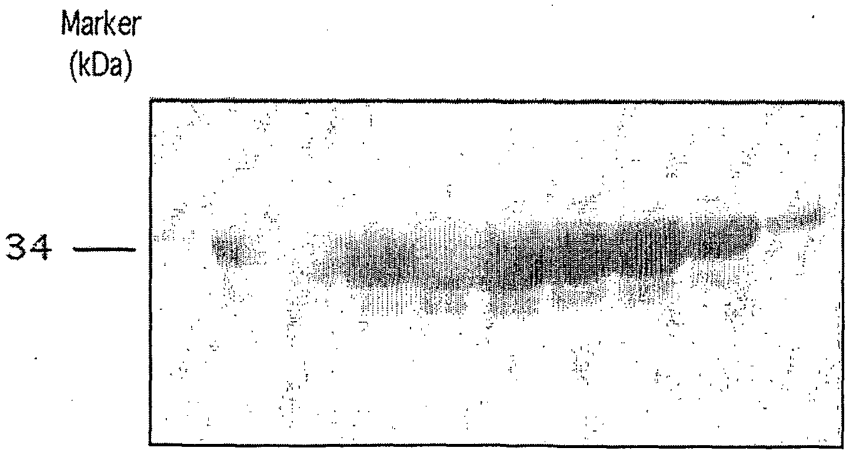


FIG. 5F

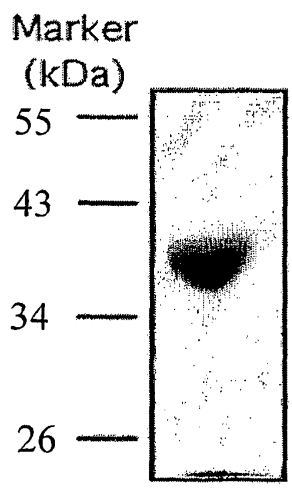


FIG. 5E

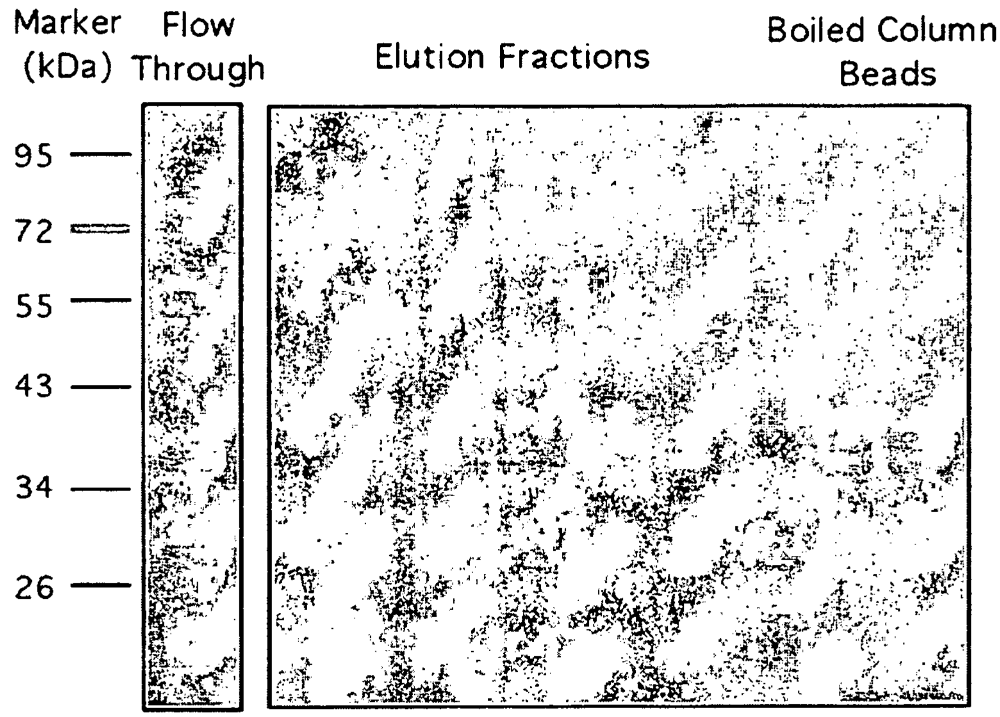
Marker (kDa)
Flow Through
Elution Fractions
Boiled Column Beads
95
72
55
43
34
26

FIG. 6A

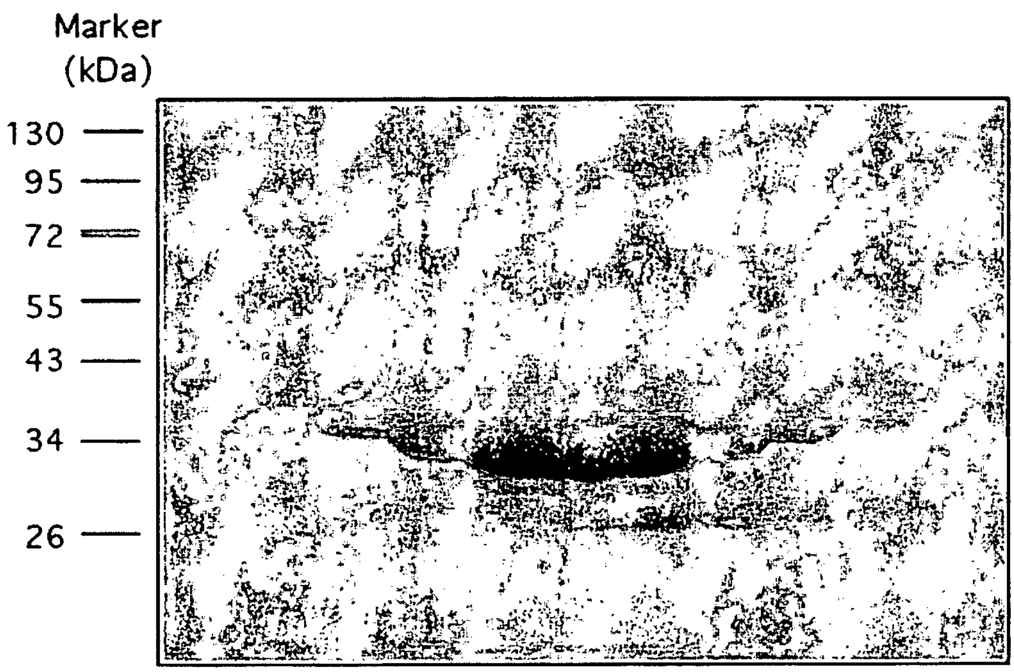
Marker (kDa)
130
95
72
55
43
34
26

FIG. 6B

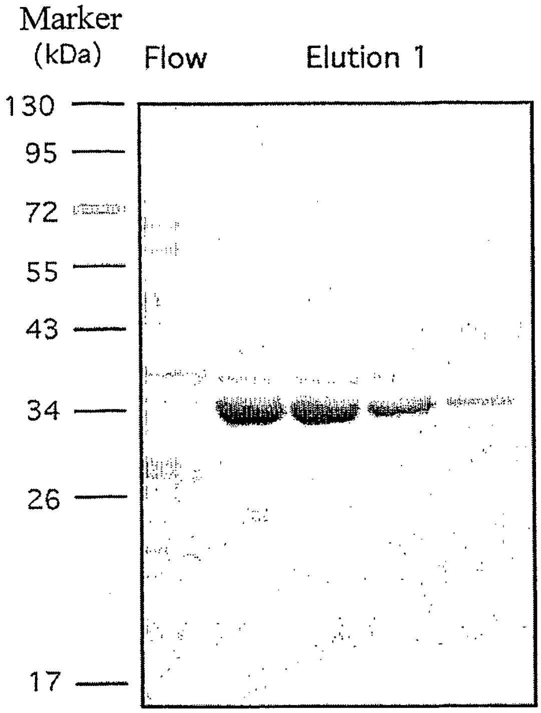


FIG. 7A

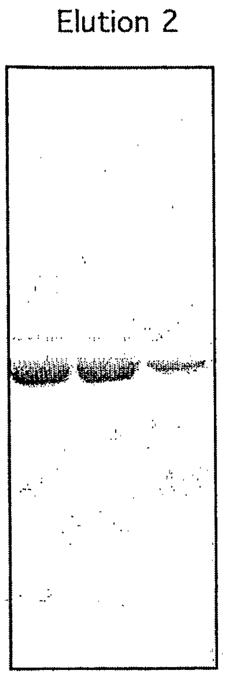


FIG. 7B

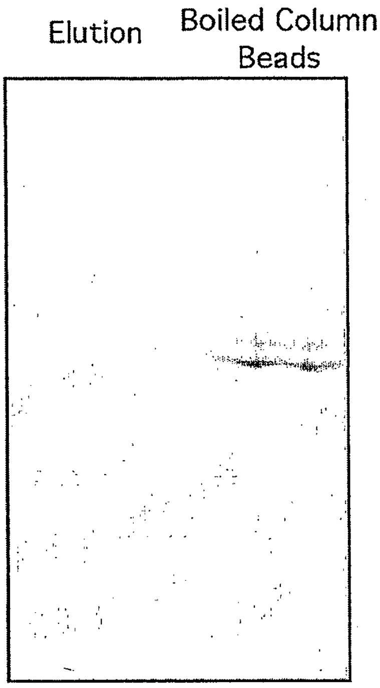


FIG. 7C

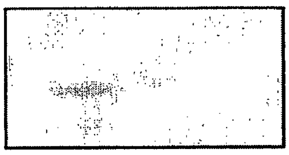

FIG. 8A

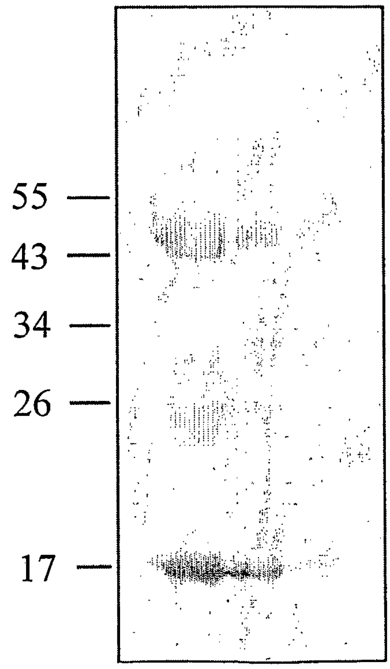


FIG. 8B

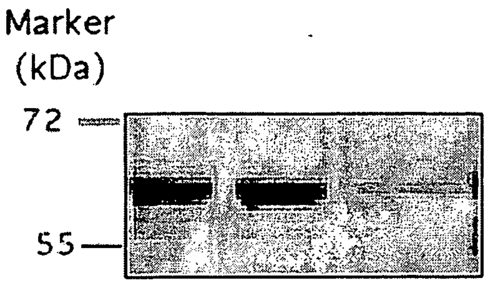


FIG. 9A

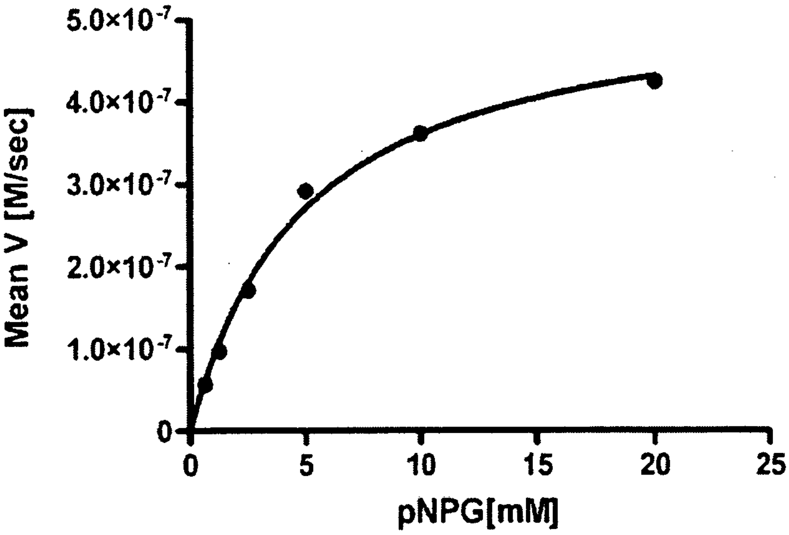


FIG. 9B

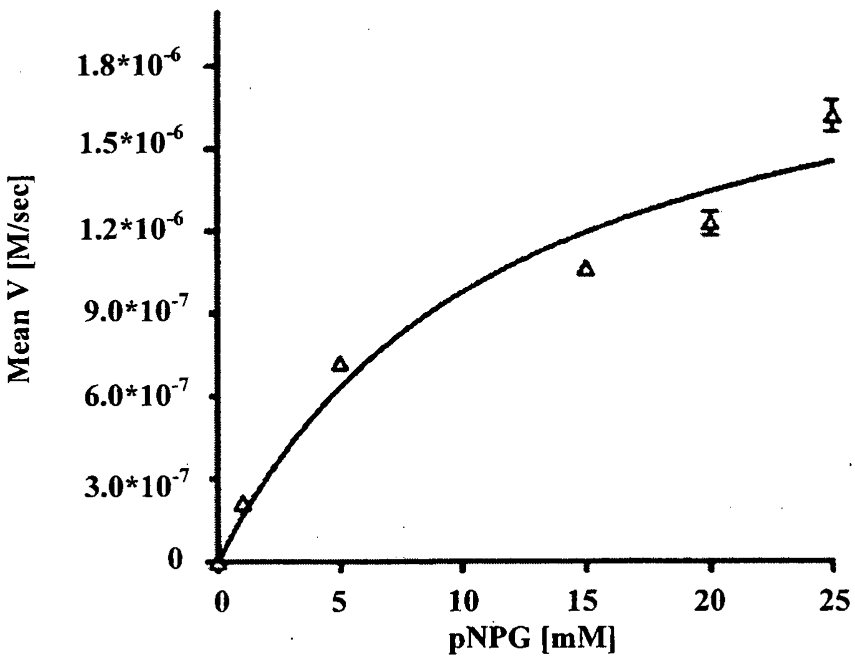


FIG. 9C

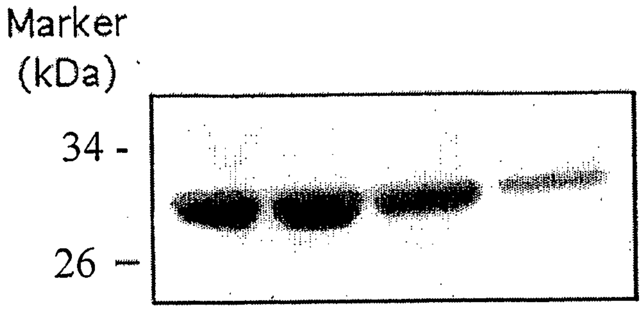


FIG. 10A

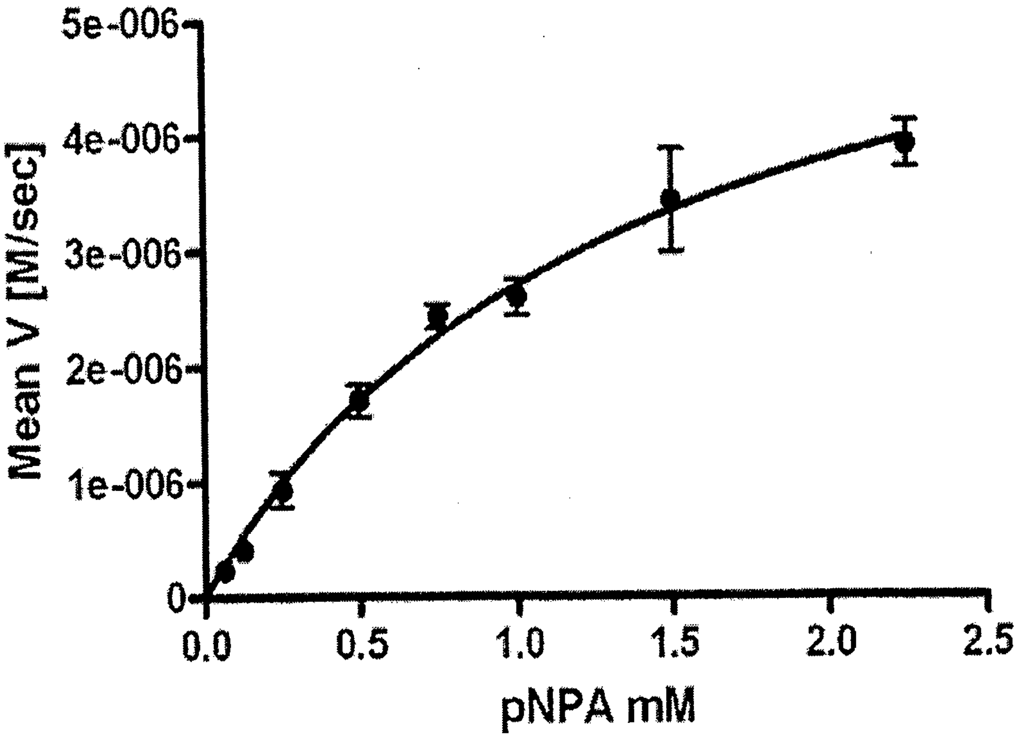


FIG. 10B

| | | |
|---|---|---|
| | | * * * *##* |
| Cj Aga27A | 402 | IEFGDVDGNGMIDALDYSLVRKYLLGQISDCPDSKGKLA |
| Cj Cel8A | 396 | GLKGDVNNDGAIDALDIAALKKAILTQSTSNINLTN |
| Cj Cel48A | 654 | SNLGDVNGDETVDAIDLAMLKKYLLNSSTSIVAGN |
| Ct Xyn11A | 384 | QVLGDLNGDKQVNSTDYTALKRHLLNITRLSGTALAN |
| Ct Xyn11B | 386 | QVLGDLNGDKQVNSTDYTALKRHLLNITRLSGTALAN |
| Ct Xyn10C | 551 | VIPGDVNGDGRVNSSDLTLMKRYLLKSIDFDFPTPEGKIA |
| Ct Cel8A | 407 | VVTGDVNGDGNVNSTDLTILKRYLLKSVTNINREA |
| Ct Cel5A | 492 | VTYGDVNGDGRVNSSDLALLKRYLLGLVENINKEAA |
| Ct Cel9A | 575 | VLYGDVNDDGKVNSTDLTLLKRYVLKAVSTLPSSKAEKA |
| Ct Cel26A-Cel5E | 882 | IKHGDLNFDNAVNSTDLLMLKRYILKSLELGTSEHEEK |
| Ct Cel9D-Cel44A | 1282 | VVYGDLNNDSKVNAVDIMMLKRYILGIIDNINLTA |
| Ct Cel48A | 669 | KLYGDVNDDGKVNSTDAVALKRYVLRSGISINTDN |
| Ct Lic16A | 263 | PLKGDVNGDGHV SSDYSLFKRYLLRVIDRFPVGDQSV |

**FIG. 11 A**

| | | |
|---|---|---|
| | | * * * *##* |
| Cj Aga27A | 441 | ADVDGDQQITALDFSLIKQYLLGTINKFPAQTASKIKP |
| Cj Cel8A | 437 | ADMNNDGNIDAIDFAQLKVKLLN-COOH |
| Cj Cel48A | 694 | ADMNGDGAIDAIDYALLKKALLANQ-COOH |
| Ct Xyn11A | 427 | ADLNGDGKVDSTDLMILHRYLLGIISSFPR |
| Ct Xyn11B | 429 | ADVNRDGKVDSTDLMMLHRYLLRIISKLG-COOH |
| Ct Xyn10C | 601 | ADLNEDGKVNSTDLLALKKLVLREL-COOH |
| Ct Cel8A | 449 | ADVNRDGAINSSDMTILKRYLIKSIPHLPY-COOH |
| Ct Cel5A | 534 | ADVNVSGTVNSTDLAIMKRYVLRSISELPY-COOH |
| Ct Cel9A | 621 | ADVNRDGRVNSSDVTILSRYLIRVIEKLPI-COOH |
| Ct Cel26A-Cel5E | 871 | ADLNRDNKVDSTDLTILKRYLLYAISEIPI-COOH |
| Ct Cel9D-Cel44A | 1323 | ADIYFDGVVNSSDYNIMKRYLLKAIEDIPY |
| Ct Cel48A | 711 | ADLNEDGRVNSTDLGILKRYILKEIDTLPKN-COOH |
| Ct Lic16A | 308 | ADVNRDGRIDSTDLTMLKRYLIRAVPSL-COOH |

**FIG. 11 B**

```
1OHZ_B       GDVNGDGTINSTDLTMLKRSVLRAITLT--------DDAKARADVDKNGSINSTDVLLLSRYLLRVI
1DAV_A       GDVNDDGKVNSTDAVALKRYVLRSGISI----------NTDNADLNEDGRVNSTDLGILKRYILKEIDTLPYKNG
A47063       GDVNDDGKVNSTDAVALKRYVLRSGISIN----------TDNADLNEDGRVNSTDLGILKRYILKEIDTLPYKN
AAB27404     GDVNDDGKVNSTDAVALKRYVLRSGIS----------INTDNADLNEDGRVNSTDLGILKRYILKEIDTLPYKN
AAC04578     GDLNGDKQVNSTDYTALKRHLLNITRL------S-GTALANADVNRDGKVDSTDLMMLHRYLLRIISKLG
AAC04579     GDLNGDKQVNSTDYTALKRHLLNITRLSG--------TALANADLNGDGKVDSTDLMILHRYLLGIISSFPRSNPQPSSNPQ
AAC06139     GDCNGDGKVNSTDAVALKRYILRSGIS----------INTDNADVNADGRVNSTDLAILKRYILKEIDVLPHK
AAO74890     GDVNGDGHVNSSDYSLFKRYLLRVIDRF-------PVGDQSVADVNRDGRIDSTDLTMLKRYLIRAIPSL
BAA12070     GDLNNDSKVNAVDIMMLKRYILGII----------DNINLTAADIYFDGVVNSSDYNIMKRYLLKAIEDIPYVPENQAPKAI
BAA33542     GDLNGDKQVNSTDYTALKRHLLNITRL--------SGTALANADVNRDGKVDSTDLMMLHRYLLRIISKLG
BAA33543     GDLNGDKQVNSTDYTALKRHLLNITRLS--------GTALANADLNGDGKVDSTDLMILHRYLLGIISSFPRSNPQPSSNPQ
BAB19050     GDLNGDGKVNSTDLTIMKRYILKNFDK--------LAVPEEAADLNGDGRINSTDLSILHRYLRRIITSFPVEQQ
CAA06692     GDCNGDGKVNSTDAVALKRYILRSGISI----------NTDNADVNADGRVNSTDLAILKRYILKEIDVLPHK
CAA27266     GDVNGDGRVNSSDVALLKRYLLGLVENI----------NKEAADVNVSGTVNSTDLAIMKRYVLRSISELPYK
CAA28255     GDVNDDGKVNSTDLTLLKRYVLKAVSTLP------SSKAEKNADVNRDGRVNSSDVTILSRYLIRVIEKLPI
CAA41281     GDVNGDGHVNSSDYSLFKRYLLRVIDRFPVGDP--------QDGCGRHDRVVDSGSK
CAA44959     GDVNGDGHVNSSDYSLFKRYLLRVIDR-------FPVGDQSVADVNRDGRIDSTDLTMLKRYLIRAIPSL
CAA56918     GDCNDDGKVNSTDVAVMKRYLKKEN----------VNINLDNADVNADGKVNSTDFSILKRYVMKNIEELPYR
CAA58242     GDVNGDGTINSTDLTMLKRSVLRAIT--------LTDDAKARADVDKNGSINSTDVLLLSRYLLRVIDKFPVAENPSSSFKY
CAA93150     GDLNGDGKVNSSDLAILKRYMLRAI------SDFPIPEGRKLADLNRDGNVNSTDYSILKRYILKAIDNIPVDD
CAB52403     GDLNGDGKVNSTDLTIMKRYILKNFD--------KLAVPEEAADLNGDGRINSTDLSILHRYLLRIITSFPVEQQ
CAB76935     GDLNGDGNINSTDFTMLKRAILGNPAP---------GTNLAAGDLNRDGNTNSTDLMILRRYLLKLIGSLPI
CAB76936     GDINLDGKINSTDLSALKRHILRITTLS--------GKQLENADVNNDGSVNSTDASILKKYIAKAIPSL
CAB76938     GDVNGDFAVNSNDLTLIKRYVLKNID------EFPSPHGLKAADVDGNEKITSSDAALVKRYVLRAITSFPVEENQNE
CAE51306     GDLNGDNRINSTDLTLMKRYILKSIED------LPVEDDLWAADINGDGKINSTDYTYLKKYLLQAIPELPKK
CAE51307     GDLNQDGQVSSTDLVAMKRYLLKNFEL--------SGVGLEAADLNSDGKVNSTDLVALKRFLLKEIDELPLKR
CAE51308     GDINLDGKINSSDVTLLKRYIVKSIDV---FPTADPERSLIASDVNGDGRVNSTDYSYLKRYVLKIIPTIPGNS
CZCLDM       GDVNDDGKVNSTDLTLLKRYVLKAVSTL------PSSKAEKNADVNRDGRVNSSDVTILSRYLIRVIEKLPI
CZCLEM       GDVNGDGKINSTDCTMLKRYILRGIE------EFPSPSGIIAADVNADLKINSTDLVLMKKYLLRSIDKFPAEDSQTPDEDN
ZP_00311428  GDVNADGVINSSDIMVLKRFLLRTITLTE--------EMLLNADTNGDGAVNSSDFTLLKRYILRSIDSFPV
ZP_00311429  GDINGDNSVNSTDLTILKRYLLGSTVPT-------APNWRLAADLNLDGNINSTDFTILKRYILGRIEAPPWVNQT
ZP_00311430  GDVDGNGTVNSTDVNYMKRYLLRQIEEFPYEK------ALMAGDVDGNGNINSTDLSYLKKYILKLISAFPAETN
```

FIG. 12

```
ZP_00311446    GDVNGDFAVNSNDLTLIKRYVLKNID------EFPSSHGLKAADVDGDEKITSSDAALVKRYVLRAITSFPVEENQNE
ZP_00311454    GDLNRNGIVNDEDYILLKNYLLRGNKL---------VIDLNVADVNKDGKVNSTDCLFLKKYILGLITI
ZP_00311690    GDLNFDNAVNSTDLLMLKRYILKSLELG---TSEQEEKFKKAADLNRDNKVDSTDLTILKRYLLKAISEIPI
ZP_00311743    GDINDDGNINSTDLQMLKRHLLRSIRLT--------EKQLLNADTNRDGRVDSTDLALLKRYILRVITTL
ZP_00311744    GDLNGDGNINSTDLQILKKHLLRITLL--------TGKELSNADVTKDGKVDSTDLTLLKRYILRFVTNF
ZP_00311745    GDLNDDGKVNSTDFQILKKHLLRITL--------LTGKNLSNADLNKDGKVDSSDLSLMKRYLLQIIPTF
ZP_00311746    GDLNNDGKVNSTDFQLLKMHVLR--------QELPAGTDLSNADVNRDGKVDSSDCTLLKRYILRVISDFPQN
ZP_00311747    GDLNGDGKVNSTDLQLMKMHVLRQR--------QLTGTSLLNADVNRDGKVDSTDVALLKRYILRQISSFDDYARS
ZP_00311957    GDINNDKTVNSTDVTYLKRFLLKQI---------NSLPNQKAADVNLDGNINSTDLVILKRYVLRGISKLPYAP
ZP_00311980    GDYNGDGAVNSTDLLACKRYLLYALK--------PEQINVIAGDLDGNGKINSTDYAYLKRYLLKQIDKFPVQLK
ZP_00312042    GDLNGDGNVNSTDSTLMSRYLLGIIT--------TLPAGEKAADLNGDGKVNSTDYNILKRYLLKYIDKFPVES
ZP_00312096    GDLNGDNNVNSTDLTLLKRYLTRVI------NDFPHPDGSVNADVNGDGKINSTDYSAMIRYILRIIDKFPAEKS
ZP_00312204    GDLNGDGLVNSSDYSLLKRYILKQIDL--------TEEKLKAADLNRNGSVDSVDYSILKRFLLKTITQLPV
ZP_00312205    GDLNNDGRTNSTDYSLMKRYLLGSISFT--------NEQLKAADVNLDGKVNSSDYTVLRRFLLGSIDLLPYNGTATYQAED
ZP_00312306    GDLNGDGNINSSDLQALKRHLLGISPLTG--------EALLRADVNRSGKVDSTDYSVLKRYILRIITEFPGQGDVQTPNPS
ZP_00312390    GDVNGDGTINSTDLTMLKRSVLRAITL--------TDDAKARADVDKNGSINSTDVLLLSRYLLRVIDKFPVAENPSSSFKY
ZP_00312443    GDLNGDGRVNSTDYTLLKRYLLG------AIQTFPYERGIKAADLNLDGRINSTDYTVLKRYLLNAIPSLPVK
ZP_00312445    GDLNGDNRINSTDLTLMKRYILKS------IEDLPVEDDLWAADINGDGKINSTDYTYLKKYLLQAIPELPKK
ZP_00312458    GDLNGDGKINSTDISLMKRYLLKQI------VDLPVEDDIKAADINKDGKVNSTDMSILKRVILRNYPL
ZP_00312459    GDVNGDLKVNSTDFSMLRRYLLKT------IDNFPTENGKQAADLNGDGRINSSDLTMLKRYLLMEVDL
ZP_00312553    GDVNLDGSVDSIDLALLYNTTYYAVPLP-------NRLQYIAADVNYDSSCTMLDFYMLEDYLLGRISSFPAGQTYTVYYGD
ZP_00312745    GDLNGDKQVNSTDYTALKRHLLNITR--------LSGTALANADLNGDGKVDSTDLMILHRYLLGIISSFPRSNPQPSSNPQ
ZP_00312800    GDLNYDGKVNSTDYLVLKRYLLGTIDKE------SDPNFLKAADLNRDGRVNSTDMSLMKRYLLGIITSF
ZP_00312801    GDVNGDGKVNSTDCSIVKRYLLK------NIEDFPYEYGKEAGDVNGDGKVNSTDYSLLKRFVLRNIDKFPVEQ
ZP_00312831    GDINSDGNVNSTDLGILKRIIVKN---------PPASANMDAADVNADGKVNSTDYTVLKRYLLRSIDKLPHTT
ZP_00312867    GDVNGDGKINSTDCTMLKRYIL------RGIEEFPSPSGIIAADVNADLKINSTDLVLMKKYLLRSIDKFPAEDSQTPDEDN
ZP_00312868    GDVNLDGQVNSTDFSLLKRYILKVVDI--------NSINVTNADMNNDGNINSTDISILKRILLRN
ZP_00312891    GDINRDGKINSTDLGMLNRHILKLVI-------LDDNLKLAAADIDGNGNINSTDYSWLKKYILKVISEFPGGDTRIVTP
ZP_00312895    GDVNDDGKVNSTDLTLLKRYVLKAV------STLPSSKAEKNADVNRDGRVNSSDVTILSRYLIRVIEKLPI
ZP_00312979    GDLNGDGRVNSTDLAVMKRYLLKQV----------QISDIRPADLNGDGKANSTDYQLLKRYILKTIDIFPVEK
ZP_00313008    GDVNADGKIDSTDLTLLKRYLLRSATLT--------EEKILNADTDGNGTVNSTDLNYLKKYILRVISVFPAEGNKPPTPTP
ZP_00313118    GDVNADGVVNISDYVLMKRYILRIIA------DFPADDDMWVGDVNGDNVINDIDCNYLKRYLLHMIREFPKNSYNSAPTFT
```

FIG. 12 (CONT. 1)

```
ZP_00313120   GDLNGDGRVNSSDLALMKRYVVKQIEKL--------NVPVKAADLNGDDKVNSTDYSVLKRYLLRSIEVIPIK
ZP_00313140   GDCNDDGKVNSTDVAVMKRYLKKENV----------NINLDNADVNADGKVNSTDFSILKRYVMKNIEELPYR
ZP_00313141   GDCNGDGKVNSTDAVALKRYILR----------SGISINTDNADVNADGRVNSTDLAILKRYILKEIDVLPHK
ZP_00313148   GDVNGDGNVNSTDVVWLRRFLLKLVE------DFPVPSGKQAADMNDDGNINSTDMIALKRKVLKIPI
ZP_00313234   GDLNGDGKVNSTDLTIMKRYILKNFD--------KLAVPEEAADLNGDGRINSTDLSILHRYLLRIITSFPVEQQ
ZP_00313235   GDLNGDQKVTSTDYTMLKRYLMKSID--------RFNTSEQAADLNRDGKINSTDLTILKRYLLYSIPSLPI
ZP_00313301   GDLNGDGVVNSTDSVILKRHIIKF-------SEITDPVKLKAADLNGDGNINSSDVSLMKRYLLRIIDKFPVE
ZP_00313302   GDLNNDSKVNAVDIMMLKRYILGI----------IDNINLTAADIYFDGVVNSSDYNIMKRYLLKAIEDIPYVPENQAPKAI
ZP_00313369   GDIVLDGNINSLDMMKLKKYLIRETQF--------NYDELLRADVNSDGEVNSTDYAYLKRYILRIIDAFPQ
ZP_00313420   GDVNDDGKVNSTDAVALKRYVLRSGI----------SINTDNADLNEDGRVNSTDLGILKRYILKEIDTLPYKN
ZP_00313489   GDVNGDGRVNSSDLTLMKRYLLKSISDF------PTPEGKIAADLNEDGKVNSTDLLALKKLVLREL
ZP_00313565   GDLNGDGRVNSTDLLLMKKRIIREIDK--------FNVPDENADLNLDGKINSSDYTILKRYVLKSIEKLPVK
ZP_00313593   GDVNGDGRVNSSDVALLKRYLLGL----------VENINKEAADVNVSGTVNSTDLAIMKRYVLRSISELPYK
ZP_00313600   GDVNFDGRINSTDYSRLKRYVIKSLEFT---DPEEHQKFIAAADVDGNGRINSTDLYVLNRYILKLIEKFPAEQ
ZP_00313635   GDINLDGKINSSDVTLLKRYIVKSIDVFPT---ADPERSLIASDVNGDGRVNSTDYSYLKRYVLKIIPTIPGNS
ZP_00314034   GDINLDGKINSTDLSALKRHILRITTLS--------GKQLENADVNNDGSVNSTDASILKKYIAKAIPSL
ZP_00314035   GDLNGDGNINSTDFTMLKRAILGNPAP---------GTNLAAGDLNRDGNTNSTDLMILRRYLLKLIGSLPI
ZP_00314046   GDLNNDGNINSTDYMILKKYILKVLERMN--------VPEKAADLNGDGSINSTDLTILKRFIMKAITKFPVTQK
ZP_00314080   GDVNKDGRINSTDIMYLKGYLLRNSAFNLD------EYGLMAADVDGNGSVSSLDLTYLKRYILRRISDFPANKK
ZP_00314122   GDLNQDGQVSSTDLVAMKRYLLKNFE--------LSGVGLEAADLNSDGKVNSTDLVALKRFLLKEIDELPLKR
ZP_00314291   GDTNSDGKINSTDVTALKRHLLRVTQ--------LTGDNLANADVNGDGNVNSTDLLLLKRYILGEIENFPI
ZP_00314354   GDLNVDGSINSVDITYMKRYLLRS-ISVLPYQENERIRIPAADTNGDGAINSSDMVLLKRYVLRSISEFPVKYDIYGNIIN
ZP_00314358   GDLNGDGKVNSSDLAILKRYMLRAIS------DFPIPEGRKLADLNRDGNVNSTDYSILKRYILKAIDNIPVDD
ZP_00314359   GDVNGDGNVNSTDLTMLKRYLLK----------SVTNINREAADVNRDGAINSSDMTILKRYLIKSIPHLPY
ZP_00314391   GDVNGDGHVNSSDYSLFKRYLLRV-------IDRFPVGDQSVADVNRDGRIDSTDLTMLKRYLIRAIPSL
```

FIG. 12 (CONT. 2)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050106700 A
- WO 2009028532 A
- EP 1441030 A
- US 5534615 A
- US 4816567 A
- WO 9411026 A
- US 61086813 B
- US 61093371 B


### Non-patent literature cited in the description


- **KARPOL et al.** *Biochem. J.,* 2008, vol. 410, 331-8
- **ADAMS JJ et al.** Structural basis of Clostridium perfringens toxin complex formation. *Proc Natl Acad Sci U S A.,* 2008
- Quantitative Drug Design. Pergamon Press, 1992
- **CREIGHTON.** *Proteins,* 1984
- **FITCH et al.** *Proc. Natl. Acad. Sci. U.S.A.,* 1983, vol. 80, 1382-1386
- **NEEDLEMAN et al.** *J. Mol. Biol.,* 1970, vol. 48, 443-453
- **CREIGHTON.** Proteins: Structures and Molecular Principles. W. H. Freeman & Co, 1983
- **SAMBROOK et al.** Molecular Cloning: A Laboratory Manual. Cold Spring Harbor Laboratory, 1989
- **AUSUBEL et al.** Current Protocols in Molecular Biology. Greene Publishing Associates and Wiley Interscience, 1989
- **BARAK, Y ; HANDELSMAN, T ; NAKAR, D ; MECHALY, A ; LAMED, R ; SHOHAM, Y ; BAYER, EA.** *J. Mol. Recogit.,* 2005, vol. 18, 491-501
- **BAYER, EA ; CHANZY, H ; LAMED, R ; SHOHAM, Y.** *Curr. Opin. Struct. Biol.,* 1998, vol. 8, 548-557
- **BAYER, EA ; SHIMON, LJW ; LAMED, R ; SHOHAM, Y.** *J. Struct. Biol.,* 1998, vol. 124, 221-234
- **BAYER EA et al.** *FEBS Lett.,* 1999, vol. 463, 277-80
- **BAYER, EA ; BELAICH, J-P ; SHOHAM, Y ; LAMED, R.** *Annu. Rev. Microbiol.,* 2004, vol. 58, 521-554
- **CRAIG, SJ ; FOONG, FC ; NORDON, R.** *J Biotechnol,* 2006, vol. 121, 165-173
- **DING S-Y ; BAYER EA ; STEINER D ; SHOHAM Y ; LAMED R.** *J. Bacteriol.,* 1999, vol. 181, 6720-6729
- **DOI RH ; GOLDSTEIN M ; HASHIDA S ; PARK JS ; TAKAGI M.** *Crit Rev Microbiol.,* 1994, vol. 20 (2), 87-93
- **GERNGROSS UT ; ROMANIEC MP ; KOBAYASHI T ; HUSKISSON NS ; DEMAIN AL.** *Mol Microbiol.,* April 1993, vol. 8 (2), 325-34
- *Mol Microbiol,* December 1993, vol. 10 (5), 1155
- **GOLD ; MARTIN.** *J Bacteriol.,* 2007, vol. 189 (19), 6787-95
- **HAIMOVITZ, R ; BARAK, Y ; MORAG, E ; VORONOV-GOLDMAN, M ; LAMED, R ; BAYER, EA.** *Proteomics,* 2008, vol. 8, 968-979
- **HANDELSMAN, T ; BARAK, Y ; NAKAR, D ; MECHALY, A ; LAMED, R ; SHOHAM, Y ; BAYER, EA.** *FEBS Lett.,* 2004, vol. 572, 195-200
- **JINDOU, S ; KAJINO, T ; INAGAKI, M ; KARITA, S ; BEGUIN, P ; KIMURA, T ; SAKKA, K ; OHMIYA, K.** *Biosci. Biotechnol. Biochem.,* 2004, vol. 68, 924-926
- **KAKIUCHI M ; ISUI A ; SUZUKI K ; FUJINO T ; FUJINO E ; KIMURA T ; KARITA S ; SAKKA K ; OHMIYA K.** *J Bacteriol.,* 1998, vol. 180 (16), 4303-8
- **KARPOL, A ; BARAK, Y ; LAMED, R ; SHOHAM, Y ; BAYER, EA.** *Biochem. J.,* 2008, vol. 410, 331-338
- **KIRBY J ; MARTIN JC ; DANIEL AS ; FLINT HJ.** *FEMS Microbiol. Lett.,* 1997, vol. 149, 213-219
- **LAMED R ; SETTER E ; KENIG R ; BAYER EA.** *Biotechnol. Bioeng. Symp.,* 1983, vol. 13, 163-181
- **LAMED R ; NAIMARK J ; MORGENSTERN E ; BAYER EA.** *J Bacteriol,* 1987, vol. 169 (8), 3792-800
- **MECHALY, A ; YARON, S ; LAMED, R ; FIEROBE, H-P ; BELAICH, A ; BELAICH, J-P ; SHOHAM, Y ; BAYER, EA.** *Proteins,* 2000, vol. 39, 170-177
- **MECHALY A ; FIEROBE HP ; BELAICH A ; BELAICH JP ; LAMED R ; SHOHAM Y ; BAYER EA.** *J Biol Chem.,* 30 March 2001, vol. 276 (13), 9883-8
- *J Biol Chem,* 01 June 2001, vol. 276 (22), 19678

- **NAKAR, D ; HANDELSMAN, T ; SHOHAM, Y ; FIEROBE, H-P ; BELAICH, JP ; MORAG, E ; LAMED, R ; BAYER, EA.** *J Biol. Chem.,* 2004, vol. 279, 42881-42888
- **OHARA H ; KARITA S ; KIMURA T ; SAKKA K ; OHMIYA K.** *Biosci Biotechnol Biochem.,* 2000, vol. 64 (2), 254-60
- **PAGÈS, S ; BELAICH, A ; BELAICH, J-P ; MORAG, E ; LAMED, R ; SHOHAM, Y ; BAYER, EA.** *Proteins,* 1997, vol. 29, 517-527
- **PAGÈS S ; BELAICH A ; TARDIF C ; REVERBEL-LEROY C ; GAUDIN C ; BELAICH JP.** *J Bacteriol,* 1996, vol. 178, 2279-86
- **POHLSCHRÖDER M ; LESCHINE SB ; CANALE-PAROLA E.** *J Bacteriol.,* January 1994, vol. 176 (1), 70-6
- **REICHMANN, D ; RAHAT, O ; COHEN, M ; NEUVIRTH, H ; SCHREIBER, G.** *Curr. Opin. Struct. Biol.,* 2007, vol. 17, 67-76
- **SHOSEYOV O ; TAKAGI M ; GOLDSTEIN MA ; DOI RH.** *Proc Natl Acad Sci USA.,* 15 April 1992, vol. 89 (8), 3483-7
- **VAN DEN ENT, F. ; LOWE, J.** *J Biochem Biophys Methods,* 2006, vol. 67, 67-74
- **WANG, WK ; KRUUS, K ; WU, JHD.** *J. Bacteriol.,* 1993, vol. 175, 1293-1302
- **WU WY et al.** *Nat Protoc.,* 2006, vol. 1 (5), 2257-62
- **YARON, S ; MORAG, E ; BAYER, EA ; LAMED, R ; SHOHAM, Y.** *FEBS Lett.,* 1995, vol. 360, 121-124
- **ZVERLOV VV et al.** *Proteomics,* 2005, vol. 5, 3646-53

SEQUENCE LISTING

<110> YEDA RESEARCH AND DEVELOPMENT CO. LTD. RAMOT at Tel Aviv University Ltd.

<120> AFFINITY PURIFICATION BY COHESIN DOCKERIN INTERACTION

<130> YEDA/080 PCT

<150> US Prov 61/086813
<151> 2008-08-07
<150> US Prov 61/093371

<151> 2008-09-01

<160> 152

<170> PatentIn version 3.5

<210> 1
<211> 64
<212> PRT
<213> Artificial

<220>
<223> Synthetic peptide

<400> 1

```
Gly Asp Val Asn Asp Asp Gly Lys Val Asn Ser Thr Asp Ala Val Ala
1               5                   10                  15

Leu Lys Arg Tyr Val Leu Arg Ser Gly Ile Ser Ile Asn Thr Asp Asn
            20                  25                  30

Ala Asp Leu Asn Glu Asp Gly Arg Val Asn Ser Thr Asp Leu Gly Ile
        35                  40                  45

Leu Lys Arg Tyr Ile Leu Lys Glu Ile Asp Thr Leu Pro Tyr Lys Asn
    50                  55                  60
```

<210> 2
<211> 48
<212> PRT
<213> Artificial

<220>
<223> Synthetic peptide

<400> 2

```
Gly Lys Arg Tyr Val Leu Arg Ser Gly Ile Ser Ile Asn Thr Asp Asn
1               5                   10                  15

Ala Asp Leu Asn Glu Asp Gly Arg Val Asn Ser Thr Asp Leu Gly Ile
            20                  25                  30

Leu Lys Arg Tyr Ile Leu Lys Glu Ile Asp Thr Leu Pro Tyr Lys Asn
        35                  40                  45
```

<210> 3
<211> 41
<212> PRT
<213> Artificial

<220>
<223> Synthetic peptide

<400> 3

```
Gly Lys Arg Tyr Val Leu Arg Ser Gly Ile Ser Ile Asn Thr Asp Asn
1               5                   10                  15

Ala Asp Leu Asn Glu Asp Gly Arg Val Asn Ser Thr Asp Leu Gly Ile
            20                  25                  30

Leu Lys Arg Tyr Ile Leu Lys Glu Ile
        35                  40
```

<210> 4
<211> 48
<212> PRT
<213> Artificial

<220>
<223> Synthetic peptide

<400> 4

```
Gly Asp Val Asn Asp Asp Gly Lys Val Asn Ser Thr Asp Ala Val Ala
1               5                   10                  15

Leu Lys Arg Tyr Val Leu Arg Ser Gly Ile Ser Ile Asn Thr Asp Asn
            20                  25                  30

Ala Lys Arg Tyr Ile Leu Lys Glu Ile Asp Thr Leu Pro Tyr Lys Asn
        35                  40                  45
```

<210> 5
<211> 47
<212> PRT
<213> Artificial

<220>
<223> synthetic peptide

<400> 5

```
Lys Arg Tyr Val Leu Arg Ser Gly Ile Ser Ile Asn Thr Asp Asn Ala
1               5                   10                  15

Asp Leu Asn Glu Asp Gly Arg Val Asn Ser Thr Asp Leu Gly Ile Leu
            20                  25                  30

Lys Arg Tyr Ile Leu Lys Glu Ile Asp Thr Leu Pro Tyr Lys Asn
        35                  40                  45
```

<210> 6
<211> 50
<212> PRT
<213> Artificial

<220>
<223> Synthetic peptide

<400> 6

```
Met Gly Ser Lys Arg Tyr Val Leu Arg Ser Gly Ile Ser Ile Asn Thr
1               5                   10                  15

Asp Asn Ala Asp Leu Asn Glu Asp Gly Arg Val Asn Ser Thr Asp Leu
            20                  25                  30

Gly Ile Leu Lys Arg Tyr Ile Leu Lys Glu Ile Asp Thr Leu Pro Tyr
        35                  40                  45

Lys Asn
    50
```

<210> 7
<211> 741
<212> PRT
<213> Clostridium thermocellum

<400> 7

```
Met Val Lys Ser Arg Lys Ile Ser Ile Leu Leu Ala Val Ala Met Leu
1               5                   10                  15

Val Ser Ile Met Ile Pro Thr Thr Ala Phe Ala Gly Pro Thr Lys Ala
            20                  25                  30

Pro Thr Lys Asp Gly Thr Ser Tyr Lys Asp Leu Phe Leu Glu Leu Tyr
        35                  40                  45

Gly Lys Ile Lys Asp Pro Lys Asn Gly Tyr Phe Ser Pro Asp Glu Gly
    50                  55                  60

Ile Pro Tyr His Ser Ile Glu Thr Leu Ile Val Glu Ala Pro Asp Tyr
65                  70                  75                  80

Gly His Val Thr Thr Ser Glu Ala Phe Ser Tyr Tyr Val Trp Leu Glu
                85                  90                  95

Ala Met Tyr Gly Asn Leu Thr Gly Asn Trp Ser Gly Val Glu Thr Ala
            100                 105                 110

Trp Lys Val Met Glu Asp Trp Ile Ile Pro Asp Ser Thr Glu Gln Pro
        115                 120                 125

Gly Met Ser Ser Tyr Asn Pro Asn Ser Pro Ala Thr Tyr Ala Asp Glu
    130                 135                 140

Tyr Glu Asp Pro Ser Tyr Tyr Pro Ser Glu Leu Lys Phe Asp Thr Val
145                 150                 155                 160
```

Arg Val Gly Ser Asp Pro Val His Asn Asp Leu Val Ser Ala Tyr Gly
165 170 175

Pro Asn Met Tyr Leu Met His Trp Leu Met Asp Val Asp Asn Trp Tyr
180 185 190

Gly Phe Gly Thr Gly Thr Arg Ala Thr Phe Ile Asn Thr Phe Gln Arg
195 200 205

Gly Glu Gln Glu Ser Thr Trp Glu Thr Ile Pro His Pro Ser Ile Glu
210 215 220

Glu Phe Lys Tyr Gly Gly Pro Asn Gly Phe Leu Asp Leu Phe Thr Lys
225 230 235 240

Asp Arg Ser Tyr Ala Lys Gln Trp Arg Tyr Thr Asn Ala Pro Asp Ala
245 250 255

Glu Gly Arg Ala Ile Gln Ala Val Tyr Trp Ala Asn Lys Trp Ala Lys
260 265 270

Glu Gln Gly Lys Gly Ser Ala Val Ala Ser Val Val Ser Lys Ala Ala
275 280 285

Lys Met Gly Asp Phe Leu Arg Asn Asp Met Phe Asp Lys Tyr Phe Met
290 295 300

Lys Ile Gly Ala Gln Asp Lys Thr Pro Ala Thr Gly Tyr Asp Ser Ala
305 310 315 320

His Tyr Leu Met Ala Trp Tyr Thr Ala Trp Gly Gly Gly Ile Gly Ala
325 330 335

Ser Trp Ala Trp Lys Ile Gly Cys Ser His Ala His Phe Gly Tyr Gln
340 345 350

Asn Pro Phe Gln Gly Trp Val Ser Ala Thr Gln Ser Asp Phe Ala Pro
355 360 365

Lys Ser Ser Asn Gly Lys Arg Asp Trp Thr Thr Ser Tyr Lys Arg Gln
370 375 380

Leu Glu Phe Tyr Gln Trp Leu Gln Ser Ala Glu Gly Gly Ile Ala Gly
385 390 395 400

Gly Ala Thr Asn Ser Trp Asn Gly Arg Tyr Glu Lys Tyr Pro Ala Gly
405 410 415

Thr Ser Thr Phe Tyr Gly Met Ala Tyr Val Pro His Pro Val Tyr Ala
420 425 430

Asp Pro Gly Ser Asn Gln Trp Phe Gly Phe Gln Ala Trp Ser Met Gln
435 440 445

Arg Val Met Glu Tyr Tyr Leu Glu Thr Gly Asp Ser Ser Val Lys Asn
450 455 460

Leu Ile Lys Lys Trp Val Asp Trp Val Met Ser Glu Ile Lys Leu Tyr
465 470 475 480

Asp Asp Gly Thr Phe Ala Ile Pro Ser Asp Leu Glu Trp Ser Gly Gln
485 490 495

Pro Asp Thr Trp Thr Gly Thr Tyr Thr Gly Asn Pro Asn Leu His Val
500 505 510

Arg Val Thr Ser Tyr Gly Thr Asp Leu Gly Val Ala Gly Ser Leu Ala
515 520 525

Asn Ala Leu Ala Thr Tyr Ala Ala Ala Thr Glu Arg Trp Glu Gly Lys
530 535 540

Leu Asp Thr Lys Ala Arg Asp Met Ala Ala Glu Leu Val Asn Arg Ala
545 550 555 560

Trp Tyr Asn Phe Tyr Cys Ser Glu Gly Lys Gly Val Val Thr Glu Glu
565 570 575

Ala Arg Ala Asp Tyr Lys Arg Phe Phe Glu Gln Glu Val Tyr Val Pro
580 585 590

Ala Gly Trp Ser Gly Thr Met Pro Asn Gly Asp Lys Ile Gln Pro Gly
595 600 605

Ile Lys Phe Ile Asp Ile Arg Thr Lys Tyr Arg Gln Asp Pro Tyr Tyr
610 615 620

Asp Ile Val Tyr Gln Ala Tyr Leu Arg Gly Glu Ala Pro Val Leu Asn
625 630 635 640

Tyr His Arg Phe Trp His Glu Val Asp Leu Ala Val Ala Met Gly Val
645 650 655

Leu Ala Thr Tyr Phe Pro Asp Met Thr Tyr Lys Val Pro Gly Thr Pro
660 665 670

Ser Thr Lys Leu Tyr Gly Asp Val Asn Asp Asp Gly Lys Val Asn Ser
675 680 685

Thr Asp Ala Val Ala Leu Lys Arg Tyr Val Leu Arg Ser Gly Ile Ser
690 695 700

Ile Asn Thr Asp Asn Ala Asp Leu Asn Glu Asp Gly Arg Val Asn Ser
705 710 715 720

Thr Asp Leu Gly Ile Leu Lys Arg Tyr Ile Leu Lys Glu Ile Asp Thr
725 730 735

Leu Pro Tyr Lys Asn
740

<210> 8
<211> 1853
<212> PRT
<213> Clostridium thermocellum

<400> 8

Met Arg Lys Val Ile Ser Met Leu Leu Val Val Ala Met Leu Thr Thr
1 5 10 15

Ile Phe Ala Ala Met Ile Pro Gln Thr Val Ser Ala Ala Thr Met Thr
20 25 30

Val Glu Ile Gly Lys Val Thr Ala Ala Val Gly Ser Lys Val Glu Ile
35 40 45

Pro Ile Thr Leu Lys Gly Val Pro Ser Lys Gly Met Ala Asn Cys Asp
50 55 60

Phe Val Leu Gly Tyr Asp Pro Asn Val Leu Glu Val Thr Glu Val Lys
65 70 75 80

Pro Gly Ser Ile Ile Lys Asp Pro Asp Pro Ser Lys Ser Phe Asp Ser
85 90 95

Ala Ile Tyr Pro Asp Arg Lys Met Ile Val Phe Leu Phe Ala Glu Asp
100 105 110

Ser Gly Arg Gly Thr Tyr Ala Ile Thr Gln Asp Gly Val Phe Ala Thr
115 120 125

Ile Val Ala Thr Val Lys Ser Ala Ala Ala Ala Pro Ile Thr Leu Leu
130 135 140

Glu Val Gly Ala Phe Ala Asp Asn Asp Leu Val Glu Ile Ser Thr Thr
145 150 155 160

Phe Val Ala Gly Gly Val Asn Leu Gly Ser Ser Val Pro Thr Thr Gln
165 170 175

Pro Asn Val Pro Ser Asp Gly Val Val Val Glu Ile Gly Lys Val Thr
180 185 190

Gly Ser Val Gly Thr Thr Val Glu Ile Pro Val Tyr Phe Arg Gly Val
195 200 205

Pro Ser Lys Gly Ile Ala Asn Cys Asp Phe Val Phe Arg Tyr Asp Pro
210 215 220

Asn Val Leu Glu Ile Ile Gly Ile Asp Pro Gly Asp Ile Ile Val Asp
225 230 235 240

Pro Asn Pro Thr Lys Ser Phe Asp Thr Ala Ile Tyr Pro Asp Arg Lys
245 250 255

Ile Ile Val Phe Leu Phe Ala Glu Asp Ser Gly Thr Gly Ala Tyr Ala
260 265 270

Ile Thr Lys Asp Gly Val Phe Ala Lys Ile Arg Ala Thr Val Lys Ser
275 280 285

Ser Ala Pro Gly Tyr Ile Thr Phe Asp Glu Val Gly Gly Phe Ala Asp
290 295 300

Asn Asp Leu Val Glu Gln Lys Val Ser Phe Ile Asp Gly Gly Val Asn
305 310 315 320

Val Gly Asn Ala Thr Pro Thr Lys Gly Ala Thr Pro Thr Asn Thr Ala
325 330 335

Thr Pro Thr Lys Ser Ala Thr Ala Thr Pro Thr Arg Pro Ser Val Pro
340 345 350

Thr Asn Thr Pro Thr Asn Thr Pro Ala Asn Thr Pro Val Ser Gly Asn
355 360 365

Leu Lys Val Glu Phe Tyr Asn Ser Asn Pro Ser Asp Thr Thr Asn Ser
370 375 380

Ile Asn Pro Gln Phe Lys Val Thr Asn Thr Gly Ser Ser Ala Ile Asp
385 390 395 400

Leu Ser Lys Leu Thr Leu Arg Tyr Tyr Tyr Thr Val Asp Gly Gln Lys
405 410 415

Asp Gln Thr Phe Trp Cys Asp His Ala Ala Ile Ile Gly Ser Asn Gly
420 425 430

Ser Tyr Asn Gly Ile Thr Ser Asn Val Lys Gly Thr Phe Val Lys Met
435 440 445

Ser Ser Ser Thr Asn Asn Ala Asp Thr Tyr Leu Glu Ile Ser Phe Thr
450 455 460

Gly Gly Thr Leu Glu Pro Gly Ala His Val Gln Ile Gln Gly Arg Phe
465 470 475 480

Ala Lys Asn Asp Trp Ser Asn Tyr Thr Gln Ser Asn Asp Tyr Ser Phe
485 490 495

Lys Ser Ala Ser Gln Phe Val Glu Trp Asp Gln Val Thr Ala Tyr Leu
500 505 510

Asn Gly Val Leu Val Trp Gly Lys Glu Pro Gly Gly Ser Val Val Pro
515 520 525

Ser Thr Gln Pro Val Thr Thr Pro Pro Ala Thr Thr Lys Pro Pro Ala
530 535 540

Thr Thr Lys Pro Pro Ala Thr Thr Ile Pro Pro Ser Asp Asp Pro Asn
545 550 555 560

Ala Ile Lys Ile Lys Val Asp Thr Val Asn Ala Lys Pro Gly Asp Thr
565 570 575

Val Asn Ile Pro Val Arg Phe Ser Gly Ile Pro Ser Lys Gly Ile Ala
580 585 590

Asn Cys Asp Phe Val Tyr Ser Tyr Asp Pro Asn Val Leu Glu Ile Ile
595 600 605

Glu Ile Lys Pro Gly Glu Leu Ile Val Asp Pro Asn Pro Asp Lys Ser
610 615 620

Phe Asp Thr Ala Val Tyr Pro Asp Arg Lys Ile Ile Val Phe Leu Phe
625 630 635 640

Ala Glu Asp Ser Gly Thr Gly Ala Tyr Ala Ile Thr Lys Asp Gly Val
645 650 655

Phe Ala Thr Ile Val Ala Lys Val Lys Ser Gly Ala Pro Asn Gly Leu
660 665 670

Ser Val Ile Lys Phe Val Glu Val Gly Gly Phe Ala Asn Asn Asp Leu
675 680 685

Val Glu Gln Arg Thr Gln Phe Phe Asp Gly Gly Val Asn Val Gly Asp
690 695 700

Thr Thr Val Pro Thr Thr Pro Thr Thr Pro Val Thr Thr Pro Thr Asp
705 710 715 720

Asp Ser Asn Ala Val Arg Ile Lys Val Asp Thr Val Asn Ala Lys Pro
725 730 735

Gly Asp Thr Val Arg Ile Pro Val Arg Phe Ser Gly Ile Pro Ser Lys
            740                 745                 750

Gly Ile Ala Asn Cys Asp Phe Val Tyr Ser Tyr Asp Pro Asn Val Leu
        755                 760                 765

Glu Ile Ile Glu Ile Glu Pro Gly Asp Ile Ile Val Asp Pro Asn Pro
    770                 775                 780

Asp Lys Ser Phe Asp Thr Ala Val Tyr Pro Asp Arg Lys Ile Ile Val
785                 790                 795                 800

Phe Leu Phe Ala Glu Asp Ser Gly Thr Gly Ala Tyr Ala Ile Thr Lys
                805                 810                 815

Asp Gly Val Phe Ala Thr Ile Val Ala Lys Val Lys Ser Gly Ala Pro
            820                 825                 830

Asn Gly Leu Ser Val Ile Lys Phe Val Glu Val Gly Gly Phe Ala Asn
        835                 840                 845

Asn Asp Leu Val Glu Gln Lys Thr Gln Phe Phe Asp Gly Gly Val Asn
    850                 855                 860

Val Gly Asp Thr Thr Glu Pro Ala Thr Pro Thr Thr Pro Val Thr Thr
865                 870                 875                 880

Pro Thr Thr Thr Asp Asp Leu Asp Ala Val Arg Ile Lys Val Asp Thr
                885                 890                 895

Val Asn Ala Lys Pro Gly Asp Thr Val Arg Ile Pro Val Arg Phe Ser
            900                 905                 910

Gly Ile Pro Ser Lys Gly Ile Ala Asn Cys Asp Phe Val Tyr Ser Tyr
        915                 920                 925

Asp Pro Asn Val Leu Glu Ile Ile Glu Ile Glu Pro Gly Asp Ile Ile
    930                 935                 940

Val Asp Pro Asn Pro Asp Lys Ser Phe Asp Thr Ala Val Tyr Pro Asp
945                 950                 955                 960

Arg Lys Ile Ile Val Phe Leu Phe Ala Glu Asp Ser Gly Thr Gly Ala
                965                 970                 975

Tyr Ala Ile Thr Lys Asp Gly Val Phe Ala Thr Ile Val Ala Lys Val
            980                 985                 990

Lys Ser Gly Ala Pro Asn Gly Leu Ser Val Ile Lys Phe Val Glu Val
        995                 1000                1005

Gly Gly Phe Ala Asn Asn Asp Leu Val Glu Gln Lys Thr Gln Phe
1010 1015 1020

Phe Asp Gly Gly Val Asn Val Gly Asp Thr Thr Glu Pro Ala Thr
1025 1030 1035

Pro Thr Thr Pro Val Thr Thr Pro Thr Thr Thr Asp Asp Leu Asp
1040 1045 1050

Ala Val Arg Ile Lys Val Asp Thr Val Asn Ala Lys Pro Gly Asp
1055 1060 1065

Thr Val Arg Ile Pro Val Arg Phe Ser Gly Ile Pro Ser Lys Gly
1070 1075 1080

Ile Ala Asn Cys Asp Phe Val Tyr Ser Tyr Asp Pro Asn Val Leu
1085 1090 1095

Glu Ile Ile Glu Ile Glu Pro Gly Asp Ile Ile Val Asp Pro Asn
1100 1105 1110

Pro Asp Lys Ser Phe Asp Thr Ala Val Tyr Pro Asp Arg Lys Ile
1115 1120 1125

Ile Val Phe Leu Phe Ala Glu Asp Ser Gly Thr Gly Ala Tyr Ala
1130 1135 1140

Ile Thr Lys Asp Gly Val Phe Ala Thr Ile Val Ala Lys Val Lys
1145 1150 1155

Glu Gly Ala Pro Asn Gly Leu Ser Val Ile Lys Phe Val Glu Val
1160 1165 1170

Gly Gly Phe Ala Asn Asn Asp Leu Val Glu Gln Lys Thr Gln Phe
1175 1180 1185

Phe Asp Gly Gly Val Asn Val Gly Asp Thr Thr Glu Pro Ala Thr
1190 1195 1200

Pro Thr Thr Pro Val Thr Thr Pro Thr Thr Thr Asp Asp Leu Asp
1205 1210 1215

Ala Val Arg Ile Lys Val Asp Thr Val Asn Ala Lys Pro Gly Asp
1220 1225 1230

Thr Val Arg Ile Pro Val Arg Phe Ser Gly Ile Pro Ser Lys Gly
1235 1240 1245

Ile Ala Asn Cys Asp Phe Val Tyr Ser Tyr Asp Pro Asn Val Leu
1250 1255 1260

Glu Ile Ile Glu Ile Glu Pro Gly Glu Leu Ile Val Asp Pro Asn
1265 1270 1275

Pro Thr Lys Ser Phe Asp Thr Ala Val Tyr Pro Asp Arg Lys Met
1280 1285 1290

Ile Val Phe Leu Phe Ala Glu Asp Ser Gly Thr Gly Ala Tyr Ala
1295 1300 1305

Ile Thr Glu Asp Gly Val Phe Ala Thr Ile Val Ala Lys Val Lys
1310 1315 1320

Ser Gly Ala Pro Asn Gly Leu Ser Val Ile Lys Phe Val Glu Val
1325 1330 1335

Gly Gly Phe Ala Asn Asn Asp Leu Val Glu Gln Lys Thr Gln Phe
1340 1345 1350

Phe Asp Gly Gly Val Asn Val Gly Asp Thr Thr Glu Pro Ala Thr
1355 1360 1365

Pro Thr Thr Pro Val Thr Thr Pro Thr Thr Thr Asp Asp Leu Asp
1370 1375 1380

Ala Val Arg Ile Lys Val Asp Thr Val Asn Ala Lys Pro Gly Asp
1385 1390 1395

Thr Val Arg Ile Pro Val Arg Phe Ser Gly Ile Pro Ser Lys Gly
1400 1405 1410

Ile Ala Asn Cys Asp Phe Val Tyr Ser Tyr Asp Pro Asn Val Leu
1415 1420 1425

Glu Ile Ile Glu Ile Glu Pro Gly Asp Ile Ile Val Asp Pro Asn
1430 1435 1440

Pro Asp Lys Ser Phe Asp Thr Ala Val Tyr Pro Asp Arg Lys Ile
1445 1450 1455

Ile Val Phe Leu Phe Ala Glu Asp Ser Gly Thr Gly Ala Tyr Ala
1460 1465 1470

Ile Thr Lys Asp Gly Val Phe Ala Thr Ile Val Ala Lys Val Lys
1475 1480 1485

Glu Gly Ala Pro Asn Gly Leu Ser Val Ile Lys Phe Val Glu Val
1490 1495 1500

Gly Gly Phe Ala Asn Asn Asp Leu Val Glu Gln Lys Thr Gln Phe
1505 1510 1515

Phe Asp Gly Gly Val Asn Val Gly Asp Thr Thr Val Pro Thr Thr
1520 1525 1530

Ser Pro Thr Thr Thr Pro Pro Glu Pro Thr Ile Thr Pro Asn Lys
1535 1540 1545

Leu Thr Leu Lys Ile Gly Arg Ala Glu Gly Arg Pro Gly Asp Thr
1550 1555 1560

Val Glu Ile Pro Val Asn Leu Tyr Gly Val Pro Gln Lys Gly Ile
1565 1570 1575

Ala Ser Gly Asp Phe Val Val Ser Tyr Asp Pro Asn Val Leu Glu
1580 1585 1590

Ile Ile Glu Ile Glu Pro Gly Glu Leu Ile Val Asp Pro Asn Pro
1595 1600 1605

Thr Lys Ser Phe Asp Thr Ala Val Tyr Pro Asp Arg Lys Met Ile
1610 1615 1620

Val Phe Leu Phe Ala Glu Asp Ser Gly Thr Gly Ala Tyr Ala Ile
1625 1630 1635

Thr Glu Asp Gly Val Phe Ala Thr Ile Val Ala Lys Val Lys Glu
1640 1645 1650

Gly Ala Pro Glu Gly Phe Ser Ala Ile Glu Ile Ser Glu Phe Gly
1655 1660 1665

Ala Phe Ala Asp Asn Asp Leu Val Glu Val Glu Thr Asp Leu Ile
1670 1675 1680

Asn Gly Gly Val Leu Val Thr Asn Lys Pro Val Ile Glu Gly Tyr
1685 1690 1695

Lys Val Ser Gly Tyr Ile Leu Pro Asp Phe Ser Phe Asp Ala Thr
1700 1705 1710

Val Ala Pro Leu Val Lys Ala Gly Phe Lys Val Glu Ile Val Gly
1715 1720 1725

Thr Glu Leu Tyr Ala Val Thr Asp Ala Asn Gly Tyr Phe Glu Ile
1730 1735 1740

Thr Gly Val Pro Ala Asn Ala Ser Gly Tyr Thr Leu Lys Ile Ser
1745 1750 1755

Arg Ala Thr Tyr Leu Asp Arg Val Ile Ala Asn Val Val Val Thr
1760 1765 1770

Gly Asp Thr Ser Val Ser Thr Ser Gln Ala Pro Ile Met Met Trp
1775 1780 1785

Val Gly Asp Ile Val Lys Asp Asn Ser Ile Asn Leu Leu Asp Val
1790 1795 1800

Ala Glu Val Ile Arg Cys Phe Asn Ala Thr Lys Gly Ser Ala Asn
1805 1810 1815

Tyr Val Glu Glu Leu Asp Ile Asn Arg Asn Gly Ala Ile Asn Met
1820 1825 1830

Gln Asp Ile Met Ile Val His Lys His Phe Gly Ala Thr Ser Ser
1835 1840 1845

Asp Tyr Asp Ala Gln
1850

<210> 9
<211> 5573
<212> DNA
<213> Clostridium thermocellum

<400> 9

atgagaaaag tcatcagtat gctcttagtt gtggctatgc tgacgacgat ttttgcggcg 60
atgataccgc agacagtatc ggcggccaca atgacagtcg agatcggcaa agttacagca 120
gccgttggat caaaagtaga aatacctata accctgaaag gagtgccatc caaaggaatg 180
gccaattgcg acttcgtatt gggttatgat ccaaatgtgc tggaagtaac agaagtaaaa 240
ccaggaagca taataaaaga tccggatcct agcaagagct ttgatagcgc aatatatccg 300
gatcgaaaga tgattgtatt tctgtttgca gaagacagtg gaagaggaac gtatgcaata 360
actcaggatg gagtatttgc aacaattgta gccactgtca aatcagctgc agcggcaccg 420
attactttgc ttgaagtagg tgcatttgcg gacaacgatt tagtagaaat aagcacaact 480
tttgtcgcgg gcggagtaaa tcttggtagt tccgtaccga caacacagcc aaatgttccg 540
tcagacggtg tggtagtaga aattggcaaa gttacgggat ctgttggaac tacagttgaa 600
atacctgtat atttcagagg agttccatcc aaaggaatag caaactgcga ctttgtgttc 660
agatatgatc cgaatgtatt ggaaattata gggatagatc ccggagacat aatagttgac 720
ccgaatccta ccaagagctt tgatactgca atatatcctg acagaaagat aatagtattc 780
ctgtttgcgg aagacagcgg aacaggagcg tatgcaataa ctaaagacgg agtatttgca 840
aaaataagag caactgtaaa atcaagtgct ccgggctata ttactttcga cgaagtaggt 900
ggatttgcag ataatgacct ggtagaacag aaggtatcat ttatagacgg tggtgttaac 960
gttggcaatg caacaccgac caagggagca acaccaacaa atacagctac gccgacaaaa 1020
tcagctacgg ctacgcccac caggccatcg gtaccgacaa acacaccgac aaacacaccg 1080
gcaaatacac cggtatcagg caatttgaag gttgaattct acaacagcaa tccttcagat 1140

actactaact caatcaatcc tcagttcaag gttactaata ccggaagcag tgcaattgat 1200
ttgtccaaac tcacattgag atattattat acagtagacg gacagaaaga tcagaccttc 1260
tggtgtgacc atgctgcaat aatcggcagt aacggcagct acaacggaat tacttcaaat 1320
gtaaaaggaa catttgtaaa aatgagttcc tcaacaaata acgcagacac ctaccttgaa 1380
ataagcttta caggcggaac tcttgaaccg ggtgcacatg ttcagataca aggtagattt 1440
gcaaagaatg actggagtaa ctatacacag tcaaatgact actcattcaa gtctgcttca 1500
cagtttgttg aatgggatca ggtaacagca tacttgaacg gtgttcttgt atggggtaaa 1560
gaacccggtg gcagtgtagt accatcaaca cagcctgtaa caacaccacc tgcaacaaca 1620
aaaccacctg caacaacaaa accacctgca acaacaatac cgccgtcaga tgatccgaat 1680
gcaataaaga ttaaggtgga cacagtaaat gcaaaaccgg gagacacagt aaatatacct 1740
gtaagattca gtggtatacc atccaaggga atagcaaact gtgactttgt atacagctat 1800
gacccgaatg tacttgagat aatagagata aaaccgggag aattgatagt tgacccgaat 1860
cctgacaaga gctttgatac tgcagtatat cctgacagaa agataatagt attcctgttt 1920
gcagaagaca gcggaacagg agcgtatgca ataactaaag acggagtatt tgctacgata 1980
gtagcgaaag taaaatccgg agcacctaac ggactcagtg taatcaaatt tgtagaagta 2040
ggcggatttg cgaacaatga ccttgtagaa cagaggacac agttctttga cggtggagta 2100
aatgttggag atacaacagt acctacaaca cctacaacac ctgtaacaac accgacagat 2160
gattcgaatg cagtaaggat taaggtggac acagtaaatg caaaaccggg agacacagta 2220
agaatacctg taagattcag cggtatacca tccaagggaa tagcaaactg tgactttgta 2280
tacagctatg acccgaatgt acttgagata atagagatag aaccgggaga cataatagtt 2340
gacccgaatc ctgacaagag ctttgatact gcagtatatc ctgacagaaa gataatagta 2400
ttcctgtttg cggaagacag cggaacagga gcgtatgcaa taactaaaga cggagtattt 2460
gctacgatag tagcgaaagt aaaatccgga gcacctaacg gactcagtgt aatcaaattt 2520
gtagaagtag gcggatttgc gaacaatgac cttgtagaac agaagacaca gttctttgac 2580
ggtggagtaa atgttggaga tacaacagaa cctgcaacac ctacaacacc tgtaacaaca 2640
ccgacaacaa cagatgatct ggatgcagta aggattaaag tggacacagt aaatgcaaaa 2700
ccgggagaca cagtaagaat acctgtaaga ttcagcggta taccatccaa gggaatagca 2760
aactgtgact ttgtatacag ctatgacccg aatgtacttg agataataga gatagaaccg 2820
ggagacataa tagttgaccc gaatcctgac aagagctttg atactgcagt atatcctgac 2880
agaaagataa tagtattcct gtttgcggaa gacagcggaa caggagcgta tgcaataact 2940
aaagacggag tatttgctac gatagtagcg aaagtaaaat ccggagcacc taacggactc 3000
agtgtaatca aatttgtaga agtaggcgga tttgcgaaca atgaccttgt agaacagaag 3060
acacagttct ttgacggtgg agtaaatgtt ggagatacaa cagaacctgc aacacctaca 3120
acacctgtaa caacaccgac aacaacagat gatctggatg cagtaaggat taaagtggac 3180

acagtaaatg caaaaccggg agacacagta agaatacctg taagattcag cggtatacca 3240
tccaagggaa tagcaaactg tgactttgta tacagctatg acccgaatgt acttgagata 3300
atagagatag aaccgggaga cataatagtt gacccgaatc ctgacaagag ctttgatact 3360
gcagtatatc ctgacagaaa gataatagta ttcctgtttg cagaagacag cggaacagga 3420
gcgtatgcaa taactaaaga cggagtattt gctacgatag tagcgaaagt aaaagaagga 3480
gcacctaacg gactcagtgt aatcaaattt gtagaagtag gcggatttgc gaacaatgac 3540
cttgtagaac agaagacaca gttctttgac ggtggagtaa atgttggaga tacaacagaa 3600
cctgcaacac ctacaacacc tgtaacaaca ccgacaacaa cagatgatct ggatgcagta 3660
aggattaaag tggacacagt aaatgcaaaa ccgggagaca cagtaagaat acctgtaaga 3720
ttcagcggta taccatccaa gggaatagca aactgtgact ttgtatacag ctatgacccg 3780
aatgtacttg agataataga gatagaaccg ggagaattga tagttgaccc gaatcctacc 3840
aagagctttg atactgcagt atatcctgac agaaagatga tagtattcct gtttgcggaa 3900
gacagcggaa caggagcgta tgcaataact gaagatggag tatttgctac gatagtagcg 3960
aaagtaaaat ccggagcacc taacggactc agtgtaatca aatttgtaga agtaggcgga 4020
tttgcgaaca atgaccttgt agaacagaag acacagttct ttgacggtgg agtaaatgtt 4080
ggagatacaa cagaacctgc aacacctaca acacctgtaa caacaccgac aacaacagat 4140
gatctggatg cagtaaggat taaagtggac acagtaaatg caaaaccggg agacacagta 4200
agaatacctg taagattcag cggtatacca tccaagggaa tagcaaactg tgactttgta 4260
tacagctatg acccgaatgt acttgagata atagagatag aaccgggaga cataatagtt 4320
gacccgaatc ctgacaagag ctttgatact gcagtatatc ctgacagaaa gataatagta 4380
ttcctgtttg cagaagacag cggaacggga gcgtatgcaa taactaaaga cggagtattt 4440
gctacgatag tagcgaaagt aaaagaagga gcacctaacg gactcagtgt aatcaaattt 4500
gtagaagtag gcggatttgc gaacaatgac cttgtagaac agaagacaca gttctttgac 4560
ggtggagtaa atgttggaga tacaacagta cctacaacat cgccgacaac aacaccgcca 4620
gagccgacga taactccgaa caagttgaca cttaagatag gcagagcaga aggaagacct 4680
ggagacacgg tggaaatacc ggttaacttg tatggagtac ctcaaaaagg aatagcaagc 4740
ggtgacttcg tagtaagcta tgacccgaat gtacttgaga taatagagat agaaccggga 4800
gaattgatag ttgacccgaa tcctaccaag agctttgata ctgctgcagt atatcctgac 4860
agaaagatga tagtattcct gtttgcggaa gacagcggaa caggagcgta tgcaataact 4920
gaagatggag tatttgctac gatagtagcg aaagtaaaag aaggagcacc tgaaggattc 4980
agtgcaatag aaatttctga gtttggtgca tttgcagata atgatctggt agaagtggaa 5040
actgacctta tcaatggtgg agtacttgta actaataaac ctgtaataga aggatataaa 5100
gtatccggat acattttgcc agacttctcc ttcgacgcta ctgttgcacc acttgtaaag 5160
gccggattca aagttgaaat agtaggaaca gaattgtatg cagtaacaga tgcaaacgga 5220

tactttgaaa taaccggagt acctgcaaat gcaagcggat atacattgaa gatttcaaga 5280
gcaacttact tggacagagt aattgcaaat gttgtagtaa cgggagatac ttcagtttca 5340
acttcacagg ctccaataat gatgtgggta ggagacatag tgaaagacaa ttctatcaac 5400
ctgttggacg ttgcagaagt tatccgttgc ttcaacgcta ctaaaggaag cgcaaactac 5460
gtagaagaac ttgacattaa tagaaacggc gcaattaaca tgcaagacat aatgattgtt 5520
cataagcact ttggagctac atcaagtgat tacgacgcac agtaaatatt aaa 5573

<210> 10
<211> 39
<212> PRT
<213> Artificial

<220>
<223> Synthetic peptide

<400> 10

Ile Glu Phe Gly Asp Val Asp Gly Asn Gly Met Ile Asp Ala Leu Asp
1 5 10 15

Tyr Ser Leu Val Arg Lys Tyr Leu Leu Gly Gln Ile Ser Asp Cys Pro
20 25 30

Asp Ser Lys Gly Lys Leu Ala
35

<210> 11
<211> 36
<212> PRT
<213> Artificial

<220>
<223> synthetic peptide

<400> 11

Gly Leu Lys Gly Asp Val Asn Asn Asp Gly Ala Ile Asp Ala Leu Asp
1 5 10 15

Ile Ala Ala Leu Lys Lys Ala Ile Leu Thr Gln Ser Thr Ser Asn Ile
20 25 30

Asn Leu Thr Asn
35

<210> 12
<211> 35
<212> PRT
<213> Artificial

<220>
<223> Synthetic peptide

<400> 12

Ser Asn Leu Gly Asp Val Asn Gly Asp Glu Thr Val Asp Ala Ile Asp
1               5                   10                  15

Leu Ala Met Leu Lys Lys Tyr Leu Leu Asn Ser Ser Thr Ser Ile Val
            20                  25                  30

Ala Gly Asn
        35

<210> 13
<211> 37
<212> PRT
<213> Artificial

<220>
<223> synthetic peptide

<400> 13

Gln Val Leu Gly Asp Leu Asn Gly Asp Lys Gln Val Asn Ser Thr Asp
1               5                   10                  15

Tyr Thr Ala Leu Lys Arg His Leu Leu Asn Ile Thr Arg Leu Ser Gly
            20                  25                  30

Thr Ala Leu Ala Asn
        35

<210> 14
<211> 40
<212> PRT
<213> Artificial

<220>
<223> Synthetic peptide

<400> 14

Val Ile Pro Gly Asp Val Asn Gly Asp Gly Arg Val Asn Ser Ser Asp
1               5                   10                  15

Leu Thr Leu Met Lys Arg Tyr Leu Leu Lys Ser Ile Asp Phe Asp Phe
            20                  25                  30

Pro Thr Pro Glu Gly Lys Ile Ala
        35                  40

<210> 15
<211> 35
<212> PRT
<213> Artificial

<220>

<223> Synthetic peptide

<400> 15

Val Val Thr Gly Asp Val Asn Gly Asp Gly Asn Val Asn Ser Thr Asp
1 5 10 15

Leu Thr Ile Leu Lys Arg Tyr Leu Leu Lys Ser Val Thr Asn Ile Asn
20 25 30

Arg Glu Ala
35

<210> 16
<211> 36
<212> PRT
<213> Artificial

<220>
<223> Synthetic peptide

<400> 16

Val Thr Tyr Gly Asp Val Asn Gly Asp Gly Arg Val Asn Ser Ser Asp
1 5 10 15

Leu Ala Leu Leu Lys Arg Tyr Leu Leu Gly Leu Val Glu Asn Ile Asn
20 25 30

Lys Glu Ala Ala
35

<210> 17
<211> 39
<212> PRT
<213> Artificial

<220>
<223> Synthetic peptide

<400> 17

Val Leu Tyr Gly Asp Val Asn Asp Asp Gly Lys Val Asn Ser Thr Asp
1 5 10 15

Leu Thr Leu Leu Lys Arg Tyr Val Leu Lys Ala Val Ser Thr Leu Pro
20 25 30

Ser Ser Lys Ala Glu Lys Ala
35

<210> 18
<211> 38
<212> PRT
<213> Artificial

<220>
<223> Synthetic peptide

<400> 18

Ile Lys His Gly Asp Leu Asn Phe Asp Asn Ala Val Asn Ser Thr Asp
1 5 10 15

Leu Leu Met Leu Lys Arg Tyr Ile Leu Lys Ser Leu Glu Leu Gly Thr
20 25 30

Ser Glu His Glu Glu Lys
35

<210> 19
<211> 35
<212> PRT
<213> Artificial

<220>
<223> Synthetic peptide

<400> 19

Val Val Tyr Gly Asp Leu Asn Asn Asp Ser Lys Val Asn Ala Val Asp
1 5 10 15

Ile Met Met Leu Lys Arg Tyr Ile Leu Gly Ile Ile Asp Asn Ile Asn
20 25 30

Leu Thr Ala
35

<210> 20
<211> 35
<212> PRT
<213> Artificial

<220>
<223> Synthetic peptide

<400> 20

Lys Leu Tyr Gly Asp Val Asn Asp Asp Gly Lys Val Asn Ser Thr Asp
1 5 10 15

Ala Val Ala Leu Lys Arg Tyr Val Leu Arg Ser Gly Ile Ser Ile Asn
20 25 30

Thr Asp Asn
35

<210> 21
<211> 37

<212> PRT
<213> Artificial

<220>
<223> Synthetic peptide

<400> 21

Pro Leu Lys Gly Asp Val Asn Gly Asp Gly His Val Ser Ser Asp Tyr
1 5 10 15

Ser Leu Phe Lys Arg Tyr Leu Leu Arg Val Ile Asp Arg Phe Pro Val
20 25 30

Gly Asp Gln Ser Val
35

<210> 22
<211> 38
<212> PRT
<213> Artificial

<220>
<223> Synthetic peptide

<400> 22

Ala Asp Val Asp Gly Asp Gln Gln Ile Thr Ala Leu Asp Phe Ser Leu
1 5 10 15

Ile Lys Gln Tyr Leu Leu Gly Thr Ile Asn Lys Phe Pro Ala Gln Thr
20 25 30

Ala Ser Lys Ile Lys Pro
35

<210> 23
<211> 23
<212> PRT
<213> Artificial

<220>
<223> Synthetic peptide

<400> 23

Ala Asp Met Asn Asn Asp Gly Asn Ile Asp Ala Ile Asp Phe Ala Gln
1 5 10 15

Leu Lys Val Lys Leu Leu Asn
20

<210> 24
<211> 25

<212> PRT
<213> Artificial

<220>
<223> Synthetic peptide

<400> 24

```
Ala Asp Met Asn Gly Asp Gly Ala Ile Asp Ala Ile Asp Tyr Ala Leu
1               5                   10                  15

Leu Lys Lys Ala Leu Leu Ala Asn Gln
            20                  25
```

<210> 25
<211> 30
<212> PRT
<213> Artificial

<220>
<223> Synthetic peptide

<400> 25

```
Ala Asp Leu Asn Gly Asp Gly Lys Val Asp Ser Thr Asp Leu Met Ile
1               5                   10                  15

Leu His Arg Tyr Leu Leu Gly Ile Ile Ser Ser Phe Pro Arg
            20                  25                  30
```

<210> 26
<211> 29
<212> PRT
<213> Artificial

<220>
<223> Synthetic peptide

<400> 26

```
Ala Asp Val Asn Arg Asp Gly Lys Val Asp Ser Thr Asp Leu Met Met
1               5                   10                  15

Leu His Arg Tyr Leu Leu Arg Ile Ile Ser Lys Leu Gly
            20                  25
```

<210> 27
<211> 25
<212> PRT
<213> Artificial

<220>
<223> Synthetic peptide

<400> 27

Ala Asp Leu Asn Glu Asp Gly Lys Val Asn Ser Thr Asp Leu Leu Ala
1 5 10 15

Leu Lys Lys Leu Val Leu Arg Glu Leu
20 25

<210> 28
<211> 30
<212> PRT
<213> Artificial

<220>
<223> Synthetic peptide

<400> 28

Ala Asp Val Asn Arg Asp Gly Ala Ile Asn Ser Ser Asp Met Thr Ile
1 5 10 15

Leu Lys Arg Tyr Leu Ile Lys Ser Ile Pro His Leu Pro Tyr
20 25 30

<210> 29
<211> 30
<212> PRT
<213> Artificial

<220>
<223> Synthetic peptide

<400> 29

Ala Asp Val Asn Val Ser Gly Thr Val Asn Ser Thr Asp Leu Ala Ile
1 5 10 15

Met Lys Arg Tyr Val Leu Arg Ser Ile Ser Glu Leu Pro Tyr
20 25 30

<210> 30
<211> 30
<212> PRT
<213> Artificial

<220>
<223> Synthetic peptide

<400> 30

Ala Asp Val Asn Arg Asp Gly Arg Val Asn Ser Ser Asp Val Thr Ile
1 5 10 15

Leu Ser Arg Tyr Leu Ile Arg Val Ile Glu Lys Leu Pro Ile
20 25 30

<210> 31
<211> 30
<212> PRT
<213> Artificial

<220>
<223> Synthetic peptide

<400> 31

```
Ala Asp Leu Asn Arg Asp Asn Lys Val Asp Ser Thr Asp Leu Thr Ile
1               5                   10                  15

Leu Lys Arg Tyr Leu Leu Tyr Ala Ile Ser Glu Ile Pro Ile
            20                  25                  30
```

<210> 32
<211> 30
<212> PRT
<213> Artificial

<220>
<223> Synthetic peptide

<400> 32

```
Ala Asp Ile Tyr Phe Asp Gly Val Val Asn Ser Ser Asp Tyr Asn Ile
1               5                   10                  15

Met Lys Arg Tyr Leu Leu Lys Ala Ile Glu Asp Ile Pro Tyr
            20                  25                  30
```

<210> 33
<211> 30
<212> PRT
<213> Artificial

<220>
<223> synthetic peptide

<400> 33

```
Ala Asp Leu Asn Glu Asp Gly Arg Val Asn Ser Asp Leu Gly Ile Leu
1               5                   10                  15

Lys Arg Tyr Ile Leu Lys Glu Ile Asp Thr Leu Pro Lys Asn
            20                  25                  30
```

<210> 34
<211> 28
<212> PRT
<213> Artificial

<220>

<223> Synthetic peptide

<400> 34

```
Ala Asp Val Asn Arg Asp Gly Arg Ile Asp Ser Thr Asp Leu Thr Met
1               5                   10                  15

Leu Lys Arg Tyr Leu Ile Arg Ala Val Pro Ser Leu
            20                  25
```

<210> 35
<211> 59
<212> PRT
<213> Artificial

<220>
<223> synthetic peptide

<400> 35

```
Gly Asp Val Asn Gly Asp Gly Thr Ile Asn Ser Thr Asp Leu Thr Met
1               5                   10                  15

Leu Lys Arg Ser Val Leu Arg Ala Ile Thr Leu Thr Asp Asp Ala Lys
            20                  25                  30

Ala Arg Ala Asp Val Asp Lys Asn Gly Ser Ile Asn Ser Thr Asp Val
        35                  40                  45

Leu Leu Leu Ser Arg Tyr Leu Leu Arg Val Ile
    50                  55
```

<210> 36
<211> 65
<212> PRT
<213> Artificial

<220>
<223> Synthetic peptide

<400> 36

Gly Asp Val Asn Asp Asp Gly Lys Val Asn Ser Thr Asp Ala Val Ala
1 5 10 15

Leu Lys Arg Tyr Val Leu Arg Ser Gly Ile Ser Ile Asn Thr Asp Asn
20 25 30

Ala Asp Leu Asn Glu Asp Gly Arg Val Asn Ser Thr Asp Leu Gly Ile
35 40 45

Leu Lys Arg Tyr Ile Leu Lys Glu Ile Asp Thr Leu Pro Tyr Lys Asn
50 55 60

Gly
65

<210> 37
<211> 64
<212> PRT
<213> Artificial

<220>
<223> Synthetic peptide

<400> 37

Gly Asp Val Asn Asp Asp Gly Lys Val Asn Ser Thr Asp Ala Val Ala
1 5 10 15

Leu Lys Arg Tyr Val Leu Arg Ser Gly Ile Ser Ile Asn Thr Asp Asn
20 25 30

Ala Asp Leu Asn Glu Asp Gly Arg Val Asn Ser Thr Asp Leu Gly Ile
35 40 45

Leu Lys Arg Tyr Ile Leu Lys Glu Ile Asp Thr Leu Pro Tyr Lys Asn
50 55 60

<210> 38
<211> 64
<212> PRT
<213> Artificial

<220>
<223> Synthetic peptide

<400> 38

Gly Asp Val Asn Asp Asp Gly Lys Val Asn Ser Thr Asp Ala Val Ala
1 5 10 15

Leu Lys Arg Tyr Val Leu Arg Ser Gly Ile Ser Ile Asn Thr Asp Asn
20 25 30

Ala Asp Leu Asn Glu Asp Gly Arg Val Asn Ser Thr Asp Leu Gly Ile
35 40 45

Leu Lys Arg Tyr Ile Leu Lys Glu Ile Asp Thr Leu Pro Tyr Lys Asn
50 55 60

<210> 39
<211> 63
<212> PRT
<213> Artificial

<220>
<223> Synthetic peptide

<400> 39

Gly Asp Leu Asn Gly Asp Lys Gln Val Asn Ser Thr Asp Tyr Thr Ala
1 5 10 15

Leu Lys Arg His Leu Leu Asn Ile Thr Arg Leu Ser Gly Thr Ala Leu
20 25 30

Ala Asn Ala Asp Val Asn Arg Asp Gly Lys Val Asp Ser Thr Asp Leu
35 40 45

Met Met Leu His Arg Tyr Leu Leu Arg Ile Ile Ser Lys Leu Gly
50 55 60

<210> 40
<211> 75
<212> PRT
<213> Artificial

<220>
<223> Synthetic peptide

<400> 40

Gly Asp Leu Asn Gly Asp Lys Gln Val Asn Ser Thr Asp Tyr Thr Ala
1               5                   10                  15

Leu Lys Arg His Leu Leu Asn Ile Thr Arg Leu Ser Gly Thr Ala Leu
            20                  25                  30

Ala Asn Ala Asp Leu Asn Gly Asp Gly Lys Val Asp Ser Thr Asp Leu
        35                  40                  45

Met Ile Leu His Arg Tyr Leu Leu Gly Ile Ile Ser Ser Phe Pro Arg
    50                  55                  60

Ser Asn Pro Gln Pro Ser Ser Asn Pro Gln Pro
65                  70                  75

<210> 41
<211> 63
<212> PRT
<213> Artificial

<220>
<223> Synthetic peptide

<400> 41

Gly Asp Cys Asn Gly Asp Gly Lys Val Asn Ser Thr Asp Ala Val Ala
1               5                   10                  15

Leu Lys Arg Tyr Ile Leu Arg Ser Gly Ile Ser Ile Asn Thr Asp Asn
            20                  25                  30

Ala Asp Val Asn Ala Asp Gly Arg Val Asn Ser Thr Asp Leu Ala Ile
        35                  40                  45

Leu Lys Arg Tyr Ile Leu Lys Glu Ile Asp Val Leu Pro His Lys
    50                  55                  60

<210> 42
<211> 63
<212> PRT
<213> Artificial

<220>
<223> Synthetic peptide

<400> 42

Gly Asp Val Asn Gly Asp Gly His Val Asn Ser Ser Asp Tyr Ser Leu
1               5                   10                  15

Phe Lys Arg Tyr Leu Leu Arg Val Ile Asp Arg Phe Pro Val Gly Asp
            20                  25                  30

Gln Ser Val Ala Asp Val Asn Arg Asp Gly Arg Ile Asp Ser Thr Asp
        35                  40                  45

Leu Thr Met Leu Lys Arg Tyr Leu Ile Arg Ala Ile Pro Ser Leu
    50                  55                  60

<210> 43
<211> 73
<212> PRT
<213> Artificial

<220>
<223> Synthetic peptide

<400> 43

Gly Asp Leu Asn Asn Asp Ser Lys Val Asn Ala Val Asp Ile Met Met
1               5                   10                  15

Leu Lys Arg Tyr Ile Leu Gly Ile Ile Asp Asn Ile Asn Leu Thr Ala
            20                  25                  30

Ala Asp Ile Tyr Phe Asp Gly Val Val Asn Ser Ser Asp Tyr Asn Ile
        35                  40                  45

Met Lys Arg Tyr Leu Leu Lys Ala Ile Glu Asp Ile Pro Tyr Val Pro
    50                  55                  60

Glu Asn Gln Ala Pro Lys Ala Ile Phe
65                  70

<210> 44
<211> 63
<212> PRT
<213> Artificial

<220>
<223> Synthetic peptide

<400> 44

Gly Asp Leu Asn Gly Asp Lys Gln Val Asn Ser Thr Asp Tyr Thr Ala
1               5                   10                  15

Leu Lys Arg His Leu Leu Asn Ile Thr Arg Leu Ser Gly Thr Ala Leu
            20                  25                  30

Ala Asn Ala Asp Val Asn Arg Asp Gly Lys Val Asp Ser Thr Asp Leu
        35                  40                  45

Met Met Leu His Arg Tyr Leu Leu Arg Ile Ile Ser Lys Leu Gly
    50                  55                  60

<210> 45
<211> 75
<212> PRT
<213> Artificial

<220>
<223> synthetic peptide

<400> 45

Gly Asp Leu Asn Gly Asp Lys Gln Val Asn Ser Thr Asp Tyr Thr Ala
1               5                   10                  15

Leu Lys Arg His Leu Leu Asn Ile Thr Arg Leu Ser Gly Thr Ala Leu
            20                  25                  30

Ala Asn Ala Asp Leu Asn Gly Asp Gly Lys Val Asp Ser Thr Asp Leu
        35                  40                  45

Met Ile Leu His Arg Tyr Leu Leu Gly Ile Ile Ser Ser Phe Pro Arg
    50                  55                  60

Ser Asn Pro Gln Pro Ser Ser Asn Pro Gln Pro
65                  70                  75

<210> 46
<211> 67
<212> PRT
<213> Artificial

<220>
<223> Synthetic peptide

<400> 46

Gly Asp Leu Asn Gly Asp Gly Lys Val Asn Ser Thr Asp Leu Thr Ile
1               5                   10                  15

Met Lys Arg Tyr Ile Leu Lys Asn Phe Asp Lys Leu Ala Val Pro Glu
            20                  25                  30

Glu Ala Ala Asp Leu Asn Gly Asp Gly Arg Ile Asn Ser Thr Asp Leu
        35                  40                  45

Ser Ile Leu His Arg Tyr Leu Arg Arg Ile Ile Thr Ser Phe Pro Val
    50                  55                  60

Glu Gln Gln
65

<210> 47
<211> 63
<212> PRT
<213> Artificial

<220>
<223> synthetic peptide

<400> 47

Gly Asp Cys Asn Gly Asp Gly Lys Val Asn Ser Thr Asp Ala Val Ala
1               5                   10                  15

Leu Lys Arg Tyr Ile Leu Arg Ser Gly Ile Ser Ile Asn Thr Asp Asn
            20                  25                  30

Ala Asp Val Asn Ala Asp Gly Arg Val Asn Ser Thr Asp Leu Ala Ile
        35                  40                  45

Leu Lys Arg Tyr Ile Leu Lys Glu Ile Asp Val Leu Pro His Lys
    50                  55                  60

<210> 48
<211> 63
<212> PRT
<213> Artificial

<220>
<223> Synthetic peptide

<400> 48

Gly Asp Val Asn Gly Asp Gly Arg Val Asn Ser Ser Asp Val Ala Leu
1 5 10 15

Leu Lys Arg Tyr Leu Leu Gly Leu Val Glu Asn Ile Asn Lys Glu Ala
20 25 30

Ala Asp Val Asn Val Ser Gly Thr Val Asn Ser Thr Asp Leu Ala Ile
35 40 45

Met Lys Arg Tyr Val Leu Arg Ser Ile Ser Glu Leu Pro Tyr Lys
50 55 60

<210> 49
<211> 66
<212> PRT
<213> Artificial

<220>
<223> Synthetic peptide

<400> 49

Gly Asp Val Asn Asp Asp Gly Lys Val Asn Ser Thr Asp Leu Thr Leu
1 5 10 15

Leu Lys Arg Tyr Val Leu Lys Ala Val Ser Thr Leu Pro Ser Ser Lys
20 25 30

Ala Glu Lys Asn Ala Asp Val Asn Arg Asp Gly Arg Val Asn Ser Ser
35 40 45

Asp Val Thr Ile Leu Ser Arg Tyr Leu Ile Arg Val Ile Glu Lys Leu
50 55 60

Pro Ile
65

<210> 50
<211> 49
<212> PRT
<213> Artificial

<220>
<223> Synthetic peptide

<400> 50

Gly Asp Val Asn Gly Asp Gly His Val Asn Ser Ser Asp Tyr Ser Leu
1               5                   10                  15

Phe Lys Arg Tyr Leu Leu Arg Val Ile Asp Arg Phe Pro Val Gly Asp
            20                  25                  30

Pro Gln Asp Gly Cys Gly Arg His Asp Arg Val Val Asp Ser Gly Ser
        35                  40                  45

Lys

<210> 51
<211> 63
<212> PRT
<213> Artificial

<220>
<223> synthetic peptide

<400> 51

Gly Asp Val Asn Gly Asp Gly His Val Asn Ser Ser Asp Tyr Ser Leu
1               5                   10                  15

Phe Lys Arg Tyr Leu Leu Arg Val Ile Asp Arg Phe Pro Val Gly Asp
            20                  25                  30

Gln Ser Val Ala Asp Val Asn Arg Asp Gly Arg Ile Asp Ser Thr Asp
        35                  40                  45

Leu Thr Met Leu Lys Arg Tyr Leu Ile Arg Ala Ile Pro Ser Leu
    50                  55                  60

<210> 52
<211> 63
<212> PRT
<213> Artificial

<220>
<223> Synthetic peptide

<400> 52

Gly Asp Cys Asn Asp Asp Gly Lys Val Asn Ser Thr Asp Val Ala Val
1 5 10 15

Met Lys Arg Tyr Leu Lys Lys Glu Asn Val Asn Ile Asn Leu Asp Asn
20 25 30

Ala Asp Val Asn Ala Asp Gly Lys Val Asn Ser Thr Asp Phe Ser Ile
35 40 45

Leu Lys Arg Tyr Val Met Lys Asn Ile Glu Glu Leu Pro Tyr Arg
50 55 60

<210> 53
<211> 75
<212> PRT
<213> Artificial

<220>
<223> Synthetic peptide

<400> 53

Gly Asp Val Asn Gly Asp Gly Thr Ile Asn Ser Thr Asp Leu Thr Met
1 5 10 15

Leu Lys Arg Ser Val Leu Arg Ala Ile Thr Leu Thr Asp Asp Ala Lys
20 25 30

Ala Arg Ala Asp Val Asp Lys Asn Gly Ser Ile Asn Ser Thr Asp Val
35 40 45

Leu Leu Leu Ser Arg Tyr Leu Leu Arg Val Ile Asp Lys Phe Pro Val
50 55 60

Ala Glu Asn Pro Ser Ser Ser Phe Lys Tyr Glu
65 70 75

<210> 54
<211> 68
<212> PRT
<213> Artificial

<220>
<223> Synthetic peptide

<400> 54

Gly Asp Leu Asn Gly Asp Gly Lys Val Asn Ser Ser Asp Leu Ala Ile
1               5                   10                  15

Leu Lys Arg Tyr Met Leu Arg Ala Ile Ser Asp Phe Pro Ile Pro Glu
            20                  25                  30

Gly Arg Lys Leu Ala Asp Leu Asn Arg Asp Gly Asn Val Asn Ser Thr
        35                  40                  45

Asp Tyr Ser Ile Leu Lys Arg Tyr Ile Leu Lys Ala Ile Asp Asn Ile
    50                  55                  60

Pro Val Asp Asp
65

<210> 55
<211> 67
<212> PRT
<213> Artificial

<220>
<223> Synthetic peptide

<400> 55

Gly Asp Leu Asn Gly Asp Gly Lys Val Asn Ser Thr Asp Leu Thr Ile
1               5                   10                  15

Met Lys Arg Tyr Ile Leu Lys Asn Phe Asp Lys Leu Ala Val Pro Glu
            20                  25                  30

Glu Ala Ala Asp Leu Asn Gly Asp Gly Arg Ile Asn Ser Thr Asp Leu
        35                  40                  45

Ser Ile Leu His Arg Tyr Leu Leu Arg Ile Ile Thr Ser Phe Pro Val
    50                  55                  60

Glu Gln Gln
65

<210> 56
<211> 63
<212> PRT
<213> Artificial

<220>
<223> Synthetic peptide

<400> 56

Gly Asp Leu Asn Gly Asp Gly Asn Ile Asn Ser Thr Asp Phe Thr Met
1 5 10 15

Leu Lys Arg Ala Ile Leu Gly Asn Pro Ala Pro Gly Thr Asn Leu Ala
20 25 30

Ala Gly Asp Leu Asn Arg Asp Gly Asn Thr Asn Ser Thr Asp Leu Met
35 40 45

Ile Leu Arg Arg Tyr Leu Leu Lys Leu Ile Gly Ser Leu Pro Ile
50 55 60

<210> 57
<211> 62
<212> PRT
<213> Artificial

<220>
<223> Synthetic peptide

<400> 57

Gly Asp Ile Asn Leu Asp Gly Lys Ile Asn Ser Thr Asp Leu Ser Ala
1 5 10 15

Leu Lys Arg His Ile Leu Arg Ile Thr Thr Leu Ser Gly Lys Gln Leu
20 25 30

Glu Asn Ala Asp Val Asn Asn Asp Gly Ser Val Asn Ser Thr Asp Ala
35 40 45

Ser Ile Leu Lys Lys Tyr Ile Ala Lys Ala Ile Pro Ser Leu
50 55 60

<210> 58
<211> 72
<212> PRT
<213> Artificial

<220>
<223> Synthetic peptide

<400> 58

Gly Asp Val Asn Gly Asp Phe Ala Val Asn Ser Asn Asp Leu Thr Leu
1               5                   10                  15

Ile Lys Arg Tyr Val Leu Lys Asn Ile Asp Glu Phe Pro Ser Pro His
            20                  25                  30

Gly Leu Lys Ala Ala Asp Val Asp Gly Asn Glu Lys Ile Thr Ser Ser
        35                  40                  45

Asp Ala Ala Leu Val Lys Arg Tyr Val Leu Arg Ala Ile Thr Ser Phe
    50                  55                  60

Pro Val Glu Glu Asn Gln Asn Glu
65                  70

<210> 59
<211> 67
<212> PRT
<213> Artificial

<220>
<223> Synthetic peptide

<400> 59

Gly Asp Leu Asn Gly Asp Asn Arg Ile Asn Ser Thr Asp Leu Thr Leu
1               5                   10                  15

Met Lys Arg Tyr Ile Leu Lys Ser Ile Glu Asp Leu Pro Val Glu Asp
            20                  25                  30

Asp Leu Trp Ala Ala Asp Ile Asn Gly Asp Gly Lys Ile Asn Ser Thr
        35                  40                  45

Asp Tyr Thr Tyr Leu Lys Lys Tyr Leu Leu Gln Ala Ile Pro Glu Leu
    50                  55                  60

Pro Lys Lys
65

<210> 60
<211> 66
<212> PRT
<213> Artificial

<220>
<223> Synthetic peptide

<400> 60

Gly Asp Leu Asn Gln Asp Gly Gln Val Ser Ser Thr Asp Leu Val Ala
1               5                   10                  15

Met Lys Arg Tyr Leu Leu Lys Asn Phe Glu Leu Ser Gly Val Gly Leu
20 25 30

Glu Ala Ala Asp Leu Asn Ser Asp Gly Lys Val Asn Ser Thr Asp Leu
35 40 45

Val Ala Leu Lys Arg Phe Leu Leu Lys Glu Ile Asp Glu Leu Pro Leu
50 55 60

Lys Arg
65

<210> 61
<211> 71
<212> PRT
<213> Artificial

<220>
<223> synthetic peptide

<400> 61

Gly Asp Ile Asn Leu Asp Gly Lys Ile Asn Ser Ser Asp Val Thr Leu
1 5 10 15

Leu Lys Arg Tyr Ile Val Lys Ser Ile Asp Val Phe Pro Thr Ala Asp
20 25 30

Pro Glu Arg Ser Leu Ile Ala Ser Asp Val Asn Gly Asp Gly Arg Val
35 40 45

Asn Ser Thr Asp Tyr Ser Tyr Leu Lys Arg Tyr Val Leu Lys Ile Ile
50 55 60

Pro Thr Ile Pro Gly Asn Ser
65 70

<210> 62
<211> 66
<212> PRT
<213> Artificial

<220>
<223> Synthetic peptide

<400> 62

```
Gly Asp Val Asn Asp Asp Gly Lys Val Asn Ser Thr Asp Leu Thr Leu
1               5                   10                  15

Leu Lys Arg Tyr Val Leu Lys Ala Val Ser Thr Leu Pro Ser Ser Lys
            20                  25                  30

Ala Glu Lys Asn Ala Asp Val Asn Arg Asp Gly Arg Val Asn Ser Ser
        35                  40                  45

Asp Val Thr Ile Leu Ser Arg Tyr Leu Ile Arg Val Ile Glu Lys Leu
    50                  55                  60

Pro Ile
65
```

<210> 63
<211> 77
<212> PRT
<213> Artificial

<220>
<223> Synthetic peptide

<400> 63

```
Gly Asp Val Asn Gly Asp Gly Lys Ile Asn Ser Thr Asp Cys Thr Met
1               5                   10                  15

Leu Lys Arg Tyr Ile Leu Arg Gly Ile Glu Glu Phe Pro Ser Pro Ser
            20                  25                  30

Gly Ile Ile Ala Ala Asp Val Asn Ala Asp Leu Lys Ile Asn Ser Thr
        35                  40                  45

Asp Leu Val Leu Met Lys Lys Tyr Leu Leu Arg Ser Ile Asp Lys Phe
    50                  55                  60

Pro Ala Glu Asp Ser Gln Thr Pro Asp Glu Asp Asn Pro
65                  70                  75
```

<210> 64
<211> 64
<212> PRT
<213> Artificial

<220>
<223> Synthetic peptide

<400> 64

Gly Asp Val Asn Ala Asp Gly Val Ile Asn Ser Ser Asp Ile Met Val
1               5                   10                  15

Leu Lys Arg Phe Leu Leu Arg Thr Ile Thr Leu Thr Glu Glu Met Leu
            20                  25                  30

Leu Asn Ala Asp Thr Asn Gly Asp Gly Ala Val Asn Ser Ser Asp Phe
        35                  40                  45

Thr Leu Leu Lys Arg Tyr Ile Leu Arg Ser Ile Asp Ser Phe Pro Val
    50                  55                  60

<210> 65
<211> 69
<212> PRT
<213> Artificial

<220>
<223> Synthetic peptide

<400> 65

Gly Asp Ile Asn Gly Asp Asn Ser Val Asn Ser Thr Asp Leu Thr Ile
1               5                   10                  15

Leu Lys Arg Tyr Leu Leu Gly Ser Thr Val Pro Thr Ala Pro Asn Trp
            20                  25                  30

Arg Leu Ala Ala Asp Leu Asn Leu Asp Gly Asn Ile Asn Ser Thr Asp
        35                  40                  45

Phe Thr Ile Leu Lys Arg Tyr Ile Leu Gly Arg Ile Glu Ala Pro Pro
    50                  55                  60

Trp Val Asn Gln Thr
65

<210> 66
<211> 69
<212> PRT
<213> Artificial

<220>
<223> Synthetic peptide

<400> 66

Gly Asp Val Asp Gly Asn Gly Thr Val Asn Ser Thr Asp Val Asn Tyr
1               5                   10                  15

Met Lys Arg Tyr Leu Leu Arg Gln Ile Glu Glu Phe Pro Tyr Glu Lys
            20                  25                  30

Ala Leu Met Ala Gly Asp Val Asp Gly Asn Gly Asn Ile Asn Ser Thr
        35                  40                  45

Asp Leu Ser Tyr Leu Lys Lys Tyr Ile Leu Lys Leu Ile Ser Ala Phe
    50                  55                  60

Pro Ala Glu Thr Asn
65

<210> 67
<211> 72
<212> PRT
<213> Artificial

<220>
<223> Synthetic peptide

<400> 67

Gly Asp Val Asn Gly Asp Phe Ala Val Asn Ser Asn Asp Leu Thr Leu
1               5                   10                  15

Ile Lys Arg Tyr Val Leu Lys Asn Ile Asp Glu Phe Pro Ser Ser His
            20                  25                  30

Gly Leu Lys Ala Ala Asp Val Asp Gly Asp Glu Lys Ile Thr Ser Ser
        35                  40                  45

Asp Ala Ala Leu Val Lys Arg Tyr Val Leu Arg Ala Ile Thr Ser Phe
    50                  55                  60

Pro Val Glu Glu Asn Gln Asn Glu
65                  70

<210> 68
<211> 60
<212> PRT
<213> Artificial

<220>
<223> Synthetic peptide

<400> 68

Gly Asp Leu Asn Arg Asn Gly Ile Val Asn Asp Glu Asp Tyr Ile Leu
1               5                   10                  15

Leu Lys Asn Tyr Leu Leu Arg Gly Asn Lys Leu Val Ile Asp Leu Asn
            20                  25                  30

Val Ala Asp Val Asn Lys Asp Gly Lys Val Asn Ser Thr Asp Cys Leu
        35                  40                  45

Phe Leu Lys Lys Tyr Ile Leu Gly Leu Ile Thr Ile
    50                  55                  60

<210> 69
<211> 69
<212> PRT
<213> Artificial

<220>
<223> Synthetic peptide

<400> 69

Gly Asp Leu Asn Phe Asp Asn Ala Val Asn Ser Thr Asp Leu Leu Met
1               5                   10                  15

Leu Lys Arg Tyr Ile Leu Lys Ser Leu Glu Leu Gly Thr Ser Glu Gln
            20                  25                  30

Glu Glu Lys Phe Lys Lys Ala Ala Asp Leu Asn Arg Asp Asn Lys Val
        35                  40                  45

Asp Ser Thr Asp Leu Thr Ile Leu Lys Arg Tyr Leu Leu Lys Ala Ile
    50                  55                  60

Ser Glu Ile Pro Ile
65

<210> 70
<211> 62
<212> PRT
<213> Artificial

<220>
<223> Synthetic peptide

<400> 70

Gly Asp Ile Asn Asp Asp Gly Asn Ile Asn Ser Thr Asp Leu Gln Met
1 5 10 15

Leu Lys Arg His Leu Leu Arg Ser Ile Arg Leu Thr Glu Lys Gln Leu
20 25 30

Leu Asn Ala Asp Thr Asn Arg Asp Gly Arg Val Asp Ser Thr Asp Leu
35 40 45

Ala Leu Leu Lys Arg Tyr Ile Leu Arg Val Ile Thr Thr Leu
50 55 60

<210> 71
<211> 62
<212> PRT
<213> Artificial

<220>
<223> Synthetic peptide

<400> 71

Gly Asp Leu Asn Gly Asp Gly Asn Ile Asn Ser Thr Asp Leu Gln Ile
1 5 10 15

Leu Lys Lys His Leu Leu Arg Ile Thr Leu Leu Thr Gly Lys Glu Leu
20 25 30

Ser Asn Ala Asp Val Thr Lys Asp Gly Lys Val Asp Ser Thr Asp Leu
35 40 45

Thr Leu Leu Lys Arg Tyr Ile Leu Arg Phe Val Thr Asn Phe
50 55 60

<210> 72
<211> 62
<212> PRT
<213> Artificial

<220>
<223> Synthetic peptide

<400> 72

Gly Asp Leu Asn Asp Asp Gly Lys Val Asn Ser Thr Asp Phe Gln Ile
1 5 10 15

Leu Lys Lys His Leu Leu Arg Ile Thr Leu Leu Thr Gly Lys Asn Leu
20 25 30

Ser Asn Ala Asp Leu Asn Lys Asp Gly Lys Val Asp Ser Ser Asp Leu
35 40 45

Ser Leu Met Lys Arg Tyr Leu Leu Gln Ile Ile Pro Thr Phe
50 55 60

<210> 73
<211> 65
<212> PRT
<213> Artificial

<220>
<223> Synthetic peptide

<400> 73

Gly Asp Leu Asn Asn Asp Gly Lys Val Asn Ser Thr Asp Phe Gln Leu
1 5 10 15

Leu Lys Met His Val Leu Arg Gln Glu Leu Pro Ala Gly Thr Asp Leu
20 25 30

Ser Asn Ala Asp Val Asn Arg Asp Gly Lys Val Asp Ser Ser Asp Cys
35 40 45

Thr Leu Leu Lys Arg Tyr Ile Leu Arg Val Ile Ser Asp Phe Pro Gln
50 55 60

Asn
65

<210> 74
<211> 68
<212> PRT
<213> Artificial

<220>
<223> Synthetic peptide

<400> 74

Gly Asp Leu Asn Gly Asp Gly Lys Val Asn Ser Thr Asp Leu Gln Leu
1 5 10 15

Met Lys Met His Val Leu Arg Gln Arg Gln Leu Thr Gly Thr Ser Leu
20 25 30

Leu Asn Ala Asp Val Asn Arg Asp Gly Lys Val Asp Ser Thr Asp Val
35 40 45

Ala Leu Leu Lys Arg Tyr Ile Leu Arg Gln Ile Ser Ser Phe Asp Asp
50 55 60

Tyr Ala Arg Ser
65

<210> 75
<211> 65
<212> PRT
<213> Artificial

<220>
<223> Synthetic peptide

<400> 75

Gly Asp Ile Asn Asn Asp Lys Thr Val Asn Ser Thr Asp Val Thr Tyr
1 5 10 15

Leu Lys Arg Phe Leu Leu Lys Gln Ile Asn Ser Leu Pro Asn Gln Lys
20 25 30

Ala Ala Asp Val Asn Leu Asp Gly Asn Ile Asn Ser Thr Asp Leu Val
35 40 45

Ile Leu Lys Arg Tyr Val Leu Arg Gly Ile Ser Lys Leu Pro Tyr Ala
50 55 60

Pro
65

<210> 76
<211> 67
<212> PRT
<213> Artificial

<220>
<223> Synthetic peptide

<400> 76

Gly Asp Tyr Asn Gly Asp Gly Ala Val Asn Ser Thr Asp Leu Leu Ala
1 5 10 15

Cys Lys Arg Tyr Leu Leu Tyr Ala Leu Lys Pro Glu Gln Ile Asn Val
20 25 30

Ile Ala Gly Asp Leu Asp Gly Asn Gly Lys Ile Asn Ser Thr Asp Tyr
35 40 45

Ala Tyr Leu Lys Arg Tyr Leu Leu Lys Gln Ile Asp Lys Phe Pro Val
50 55 60

Gln Leu Lys
65

<210> 77
<211> 66
<212> PRT
<213> Artificial

<220>
<223> Synthetic peptide

<400> 77

Gly Asp Leu Asn Gly Asp Gly Asn Val Asn Ser Thr Asp Ser Thr Leu
1 5 10 15

Met Ser Arg Tyr Leu Leu Gly Ile Ile Thr Thr Leu Pro Ala Gly Glu
20 25 30

Lys Ala Ala Asp Leu Asn Gly Asp Gly Lys Val Asn Ser Thr Asp Tyr
35 40 45

Asn Ile Leu Lys Arg Tyr Leu Leu Lys Tyr Ile Asp Lys Phe Pro Val
50 55 60

Glu Ser
65

<210> 78
<211> 69
<212> PRT
<213> Artificial

<220>
<223> Synthetic peptide

<400> 78

Gly Asp Leu Asn Gly Asp Asn Asn Val Asn Ser Thr Asp Leu Thr Leu
1 5 10 15

Leu Lys Arg Tyr Leu Thr Arg Val Ile Asn Asp Phe Pro His Pro Asp
20 25 30

Gly Ser Val Asn Ala Asp Val Asn Gly Asp Gly Lys Ile Asn Ser Thr
35 40 45

Asp Tyr Ser Ala Met Ile Arg Tyr Ile Leu Arg Ile Ile Asp Lys Phe
50 55 60

Pro Ala Glu Lys Ser
65

<210> 79
<211> 64
<212> PRT
<213> Artificial

<220>
<223> Synthetic peptide

<400> 79

Gly Asp Leu Asn Gly Asp Gly Leu Val Asn Ser Ser Asp Tyr Ser Leu
1 5 10 15

Leu Lys Arg Tyr Ile Leu Lys Gln Ile Asp Leu Thr Glu Glu Lys Leu
20 25 30

Lys Ala Ala Asp Leu Asn Arg Asn Gly Ser Val Asp Ser Val Asp Tyr
35 40 45

Ser Ile Leu Lys Arg Phe Leu Leu Lys Thr Ile Thr Gln Leu Pro Val
50 55 60

<210> 80
<211> 75
<212> PRT
<213> Artificial

<220>
<223> Synthetic peptide

<400> 80

Gly Asp Leu Asn Asn Asp Gly Arg Thr Asn Ser Thr Asp Tyr Ser Leu
1 5 10 15

Met Lys Arg Tyr Leu Leu Gly Ser Ile Ser Phe Thr Asn Glu Gln Leu
20 25 30

Lys Ala Ala Asp Val Asn Leu Asp Gly Lys Val Asn Ser Ser Asp Tyr
35 40 45

Thr Val Leu Arg Arg Phe Leu Leu Gly Ser Ile Asp Leu Leu Pro Tyr
50 55 60

Asn Gly Thr Ala Thr Tyr Gln Ala Glu Asp Ala
65 70 75

<210> 81
<211> 75
<212> PRT
<213> Artificial

<220>
<223> Synthetic peptide

<400> 81

Gly Asp Leu Asn Gly Asp Gly Asn Ile Asn Ser Ser Asp Leu Gln Ala
1 5 10 15

Leu Lys Arg His Leu Leu Gly Ile Ser Pro Leu Thr Gly Glu Ala Leu
20 25 30

Leu Arg Ala Asp Val Asn Arg Ser Gly Lys Val Asp Ser Thr Asp Tyr
35 40 45

Ser Val Leu Lys Arg Tyr Ile Leu Arg Ile Ile Thr Glu Phe Pro Gly
50 55 60

Gln Gly Asp Val Gln Thr Pro Asn Pro Ser Val
65 70 75

<210> 82
<211> 75
<212> PRT
<213> Artificial

<220>
<223> Synthetic peptide

<400> 82

Gly Asp Val Asn Gly Asp Gly Thr Ile Asn Ser Thr Asp Leu Thr Met
1 5 10 15

Leu Lys Arg Ser Val Leu Arg Ala Ile Thr Leu Thr Asp Asp Ala Lys
20 25 30

Ala Arg Ala Asp Val Asp Lys Asn Gly Ser Ile Asn Ser Thr Asp Val
35 40 45

Leu Leu Leu Ser Arg Tyr Leu Leu Arg Val Ile Asp Lys Phe Pro Val
50 55 60

Ala Glu Asn Pro Ser Ser Ser Phe Lys Tyr Glu
65 70 75

<210> 83
<211> 67
<212> PRT
<213> Artificial

<220>
<223> Synthetic peptide

<400> 83

Gly Asp Leu Asn Gly Asp Gly Arg Val Asn Ser Thr Asp Tyr Thr Leu
1 5 10 15

Leu Lys Arg Tyr Leu Leu Gly Ala Ile Gln Thr Phe Pro Tyr Glu Arg
20 25 30

Gly Ile Lys Ala Ala Asp Leu Asn Leu Asp Gly Arg Ile Asn Ser Thr
35 40 45

Asp Tyr Thr Val Leu Lys Arg Tyr Leu Leu Asn Ala Ile Pro Ser Leu
50 55 60

Pro Val Lys
65

<210> 84
<211> 67
<212> PRT
<213> Artificial

<220>
<223> Synthetic peptide

<400> 84

Gly Asp Leu Asn Gly Asp Asn Arg Ile Asn Ser Thr Asp Leu Thr Leu
1               5                   10                  15

Met Lys Arg Tyr Ile Leu Lys Ser Ile Glu Asp Leu Pro Val Glu Asp
            20                  25                  30

Asp Leu Trp Ala Ala Asp Ile Asn Gly Asp Gly Lys Ile Asn Ser Thr
        35                  40                  45

Asp Tyr Thr Tyr Leu Lys Lys Tyr Leu Leu Gln Ala Ile Pro Glu Leu
    50                  55                  60

Pro Lys Lys
65

<210> 85
<211> 63
<212> PRT
<213> Artificial

<220>
<223> synthetic peptide

<400> 85

Gly Asp Leu Asn Gly Asp Gly Lys Ile Asn Ser Thr Asp Ile Ser Leu
1               5                   10                  15

Met Lys Arg Tyr Leu Leu Lys Gln Ile Val Asp Leu Pro Val Glu Asp
            20                  25                  30

Asp Ile Lys Ala Ala Asp Ile Asn Lys Asp Gly Lys Val Asn Ser Thr
        35                  40                  45

Asp Met Ser Ile Leu Lys Arg Val Ile Leu Arg Asn Tyr Pro Leu
    50                  55                  60

<210> 86
<211> 76
<212> PRT
<213> Artificial

<220>
<223> Synthetic peptide

<400> 86

Gly Asp Val Asn Leu Asp Gly Ser Val Asp Ser Ile Asp Leu Ala Leu
1 5 10 15

Leu Tyr Asn Thr Thr Tyr Tyr Ala Val Pro Leu Pro Asn Arg Leu Gln
20 25 30

Tyr Ile Ala Ala Asp Val Asn Tyr Asp Ser Ser Cys Thr Met Leu Asp
35 40 45

Phe Tyr Met Leu Glu Asp Tyr Leu Leu Gly Arg Ile Ser Ser Phe Pro
50 55 60

Ala Gly Gln Thr Tyr Thr Val Tyr Tyr Gly Asp Leu
65 70 75

<210> 87
<211> 75
<212> PRT
<213> Artificial

<220>
<223> synthetic peptide

<400> 87

Gly Asp Leu Asn Gly Asp Lys Gln Val Asn Ser Thr Asp Tyr Thr Ala
1 5 10 15

Leu Lys Arg His Leu Leu Asn Ile Thr Arg Leu Ser Gly Thr Ala Leu
20 25 30

Ala Asn Ala Asp Leu Asn Gly Asp Gly Lys Val Asp Ser Thr Asp Leu
35 40 45

Met Ile Leu His Arg Tyr Leu Leu Gly Ile Ile Ser Ser Phe Pro Arg
50 55 60

Ser Asn Pro Gln Pro Ser Ser Asn Pro Gln Pro
65 70 75

<210> 88
<211> 64
<212> PRT
<213> Artificial

<220>
<223> Synthetic peptide

<400> 88

Gly Asp Leu Asn Tyr Asp Gly Lys Val Asn Ser Thr Asp Tyr Leu Val
1 5 10 15

Leu Lys Arg Tyr Leu Leu Gly Thr Ile Asp Lys Glu Ser Asp Pro Asn
20 25 30

Phe Leu Lys Ala Ala Asp Leu Asn Arg Asp Gly Arg Val Asn Ser Thr
35 40 45

Asp Met Ser Leu Met Lys Arg Tyr Leu Leu Gly Ile Ile Thr Ser Phe
50 55 60

<210> 89
<211> 68
<212> PRT
<213> Artificial

<220>
<223> Synthetic peptide

<400> 89

Gly Asp Val Asn Gly Asp Gly Lys Val Asn Ser Thr Asp Cys Ser Ile
1 5 10 15

Val Lys Arg Tyr Leu Leu Lys Asn Ile Glu Asp Phe Pro Tyr Glu Tyr
20 25 30

Gly Lys Glu Ala Gly Asp Val Asn Gly Asp Gly Lys Val Asn Ser Thr
35 40 45

Asp Tyr Ser Leu Leu Lys Arg Phe Val Leu Arg Asn Ile Asp Lys Phe
50 55 60

Pro Val Glu Gln
65

<210> 90
<211> 65
<212> PRT
<213> Artificial

<220>
<223> synthetic peptide

<400> 90

Gly Asp Ile Asn Ser Asp Gly Asn Val Asn Ser Thr Asp Leu Gly Ile
1               5                   10                  15

Leu Lys Arg Ile Ile Val Lys Asn Pro Pro Ala Ser Ala Asn Met Asp
            20                  25                  30

Ala Ala Asp Val Asn Ala Asp Gly Lys Val Asn Ser Thr Asp Tyr Thr
        35                  40                  45

Val Leu Lys Arg Tyr Leu Leu Arg Ser Ile Asp Lys Leu Pro His Thr
    50                  55                  60

Thr
65

<210> 91
<211> 77
<212> PRT
<213> Artificial

<220>
<223> Synthetic peptide

<400> 91

Gly Asp Val Asn Gly Asp Gly Lys Ile Asn Ser Thr Asp Cys Thr Met
1               5                   10                  15

Leu Lys Arg Tyr Ile Leu Arg Gly Ile Glu Glu Phe Pro Ser Pro Ser
            20                  25                  30

Gly Ile Ile Ala Ala Asp Val Asn Ala Asp Leu Lys Ile Asn Ser Thr
        35                  40                  45

Asp Leu Val Leu Met Lys Lys Tyr Leu Leu Arg Ser Ile Asp Lys Phe
    50                  55                  60

Pro Ala Glu Asp Ser Gln Thr Pro Asp Glu Asp Asn Pro
65                  70                  75

<210> 92
<211> 58
<212> PRT
<213> Artificial

<220>
<223> Synthetic peptide

<400> 92

Gly Asp Val Asn Leu Asp Gly Gln Val Asn Ser Thr Asp Phe Ser Leu
1 5 10 15

Leu Lys Arg Tyr Ile Leu Lys Val Val Asp Ile Asn Ser Ile Asn Val
20 25 30

Thr Asn Ala Asp Met Asn Asn Asp Gly Asn Ile Asn Ser Thr Asp Ile
35 40 45

Ser Ile Leu Lys Arg Ile Leu Leu Arg Asn
50 55

<210> 93
<211> 73
<212> PRT
<213> Artificial

<220>
<223> Synthetic peptide

<400> 93

Gly Asp Ile Asn Arg Asp Gly Lys Ile Asn Ser Thr Asp Leu Gly Met
1 5 10 15

Leu Asn Arg His Ile Leu Lys Leu Val Ile Leu Asp Asp Asn Leu Lys
20 25 30

Leu Ala Ala Ala Asp Ile Asp Gly Asn Gly Asn Ile Asn Ser Thr Asp
35 40 45

Tyr Ser Trp Leu Lys Lys Tyr Ile Leu Lys Val Ile Ser Glu Phe Pro
50 55 60

Gly Gly Asp Thr Arg Ile Val Thr Pro
65 70

<210> 94
<211> 66
<212> PRT
<213> Artificial

<220>
<223> Synthetic peptide

<400> 94

```
Gly Asp Val Asn Asp Asp Gly Lys Val Asn Ser Thr Asp Leu Thr Leu
1               5                   10                  15

Leu Lys Arg Tyr Val Leu Lys Ala Val Ser Thr Leu Pro Ser Ser Lys
            20                  25                  30

Ala Glu Lys Asn Ala Asp Val Asn Arg Asp Gly Arg Val Asn Ser Ser
        35                  40                  45

Asp Val Thr Ile Leu Ser Arg Tyr Leu Ile Arg Val Ile Glu Lys Leu
    50                  55                  60

Pro Ile
65
```

<210> 95
<211> 64
<212> PRT
<213> Artificial

<220>
<223> Synthetic peptide

<400> 95

```
Gly Asp Leu Asn Gly Asp Gly Arg Val Asn Ser Thr Asp Leu Ala Val
1               5                   10                  15

Met Lys Arg Tyr Leu Leu Lys Gln Val Gln Ile Ser Asp Ile Arg Pro
            20                  25                  30

Ala Asp Leu Asn Gly Asp Gly Lys Ala Asn Ser Thr Asp Tyr Gln Leu
        35                  40                  45

Leu Lys Arg Tyr Ile Leu Lys Thr Ile Asp Ile Phe Pro Val Glu Lys
    50                  55                  60
```

<210> 96
<211> 75
<212> PRT
<213> Artificial

<220>
<223> Synthetic peptide

<400> 96

Gly Asp Val Asn Ala Asp Gly Lys Ile Asp Ser Thr Asp Leu Thr Leu
1               5                   10                  15

Leu Lys Arg Tyr Leu Leu Arg Ser Ala Thr Leu Thr Glu Glu Lys Ile
            20                  25                  30

Leu Asn Ala Asp Thr Asp Gly Asn Gly Thr Val Asn Ser Thr Asp Leu
        35                  40                  45

Asn Tyr Leu Lys Lys Tyr Ile Leu Arg Val Ile Ser Val Phe Pro Ala
    50                  55                  60

Glu Gly Asn Lys Pro Pro Thr Pro Thr Pro Thr
65                  70                  75

<210> 97
<211> 77
<212> PRT
<213> Artificial

<220>
<223> Synthetic peptide

<400> 97

Gly Asp Val Asn Ala Asp Gly Val Val Asn Ile Ser Asp Tyr Val Leu
1               5                   10                  15

Met Lys Arg Tyr Ile Leu Arg Ile Ile Ala Asp Phe Pro Ala Asp Asp
            20                  25                  30

Asp Met Trp Val Gly Asp Val Asn Gly Asp Asn Val Ile Asn Asp Ile
        35                  40                  45

Asp Cys Asn Tyr Leu Lys Arg Tyr Leu Leu His Met Ile Arg Glu Phe
    50                  55                  60

Pro Lys Asn Ser Tyr Asn Ser Ala Pro Thr Phe Thr Pro
65                  70                  75

<210> 98
<211> 65 Page 49
<212> PRT
<213> Artificial

<220>
<223> Synthetic peptide

<400> 98

Gly Asp Leu Asn Gly Asp Gly Arg Val Asn Ser Ser Asp Leu Ala Leu
1 5 10 15

Met Lys Arg Tyr Val Val Lys Gln Ile Glu Lys Leu Asn Val Pro Val
20 25 30

Lys Ala Ala Asp Leu Asn Gly Asp Asp Lys Val Asn Ser Thr Asp Tyr
35 40 45

Ser Val Leu Lys Arg Tyr Leu Leu Arg Ser Ile Glu Val Ile Pro Ile
50 55 60

Lys
65

<210> 99
<211> 63
<212> PRT
<213> Artificial

<220>
<223> Synthetic peptide

<400> 99

Gly Asp Cys Asn Asp Asp Gly Lys Val Asn Ser Thr Asp Val Ala Val
1 5 10 15

Met Lys Arg Tyr Leu Lys Lys Glu Asn Val Asn Ile Asn Leu Asp Asn
20 25 30

Ala Asp Val Asn Ala Asp Gly Lys Val Asn Ser Thr Asp Phe Ser Ile
35 40 45

Leu Lys Arg Tyr Val Met Lys Asn Ile Glu Glu Leu Pro Tyr Arg
50 55 60

<210> 100
<211> 63
<212> PRT
<213> Artificial

<220>
<223> Synthetic peptide

<400> 100

Gly Asp Cys Asn Gly Asp Gly Lys Val Asn Ser Thr Asp Ala Val Ala
1 5 10 15

Leu Lys Arg Tyr Ile Leu Arg Ser Gly Ile Ser Ile Asn Thr Asp Asn
20 25 30

Ala Asp Val Asn Ala Asp Gly Arg Val Asn Ser Thr Asp Leu Ala Ile
35 40 45

Leu Lys Arg Tyr Ile Leu Lys Glu Ile Asp Val Leu Pro His Lys
50 55 60

<210> 101
<211> 62
<212> PRT
<213> Artificial

<220>
<223> Synthetic peptide

<400> 101

Gly Asp Val Asn Gly Asp Gly Asn Val Asn Ser Thr Asp Val Val Trp
1 5 10 15

Leu Arg Arg Phe Leu Leu Lys Leu Val Glu Asp Phe Pro Val Pro Ser
20 25 30

Gly Lys Gln Ala Ala Asp Met Asn Asp Asp Gly Asn Ile Asn Ser Thr
35 40 45

Asp Met Ile Ala Leu Lys Arg Lys Val Leu Lys Ile Pro Ile
50 55 60

<210> 102
<211> 67
<212> PRT
<213> Artificial

<220>
<223> Synthetic peptide

<400> 102

Gly Asp Leu Asn Gly Asp Gly Lys Val Asn Ser Thr Asp Leu Thr Ile
1 5 10 15

Met Lys Arg Tyr Ile Leu Lys Asn Phe Asp Lys Leu Ala Val Pro Glu
20 25 30

Glu Ala Ala Asp Leu Asn Gly Asp Gly Arg Ile Asn Ser Thr Asp Leu
35 40 45

Ser Ile Leu His Arg Tyr Leu Leu Arg Ile Ile Thr Ser Phe Pro Val
50 55 60

Glu Gln Gln
65

<210> 103
<211> 64
<212> PRT
<213> Artificial

<220>
<223> Synthetic peptide

<400> 103

Gly Asp Leu Asn Gly Asp Gln Lys Val Thr Ser Thr Asp Tyr Thr Met
1 5 10 15

Leu Lys Arg Tyr Leu Met Lys Ser Ile Asp Arg Phe Asn Thr Ser Glu
20 25 30

Gln Ala Ala Asp Leu Asn Arg Asp Gly Lys Ile Asn Ser Thr Asp Leu
35 40 45

Thr Ile Leu Lys Arg Tyr Leu Leu Tyr Ser Ile Pro Ser Leu Pro Ile
50 55 60

<210> 104
<211> 66
<212> PRT
<213> Artificial

<220>
<223> Synthetic peptide

<400> 104

Gly Asp Leu Asn Gly Asp Gly Val Val Asn Ser Thr Asp Ser Val Ile
1               5                   10                  15

Leu Lys Arg His Ile Ile Lys Phe Ser Glu Ile Thr Asp Pro Val Lys
            20                  25                  30

Leu Lys Ala Ala Asp Leu Asn Gly Asp Gly Asn Ile Asn Ser Ser Asp
        35                  40                  45

Val Ser Leu Met Lys Arg Tyr Leu Leu Arg Ile Ile Asp Lys Phe Pro
    50                  55                  60

Val Glu
65

<210> 105
<211> 73
<212> PRT
<213> Artificial

<220>
<223> Synthetic peptide

<400> 105

Gly Asp Leu Asn Asn Asp Ser Lys Val Asn Ala Val Asp Ile Met Met
1               5                   10                  15

Leu Lys Arg Tyr Ile Leu Gly Ile Ile Asp Asn Ile Asn Leu Thr Ala
            20                  25                  30

Ala Asp Ile Tyr Phe Asp Gly Val Val Asn Ser Ser Asp Tyr Asn Ile
        35                  40                  45

Met Lys Arg Tyr Leu Leu Lys Ala Ile Glu Asp Ile Pro Tyr Val Pro
    50                  55                  60

Glu Asn Gln Ala Pro Lys Ala Ile Phe
65                  70

<210> 106
<211> 64
<212> PRT
<213> Artificial

<220>
<223> Synthetic peptide

<400> 106

Gly Asp Ile Val Leu Asp Gly Asn Ile Asn Ser Leu Asp Met Met Lys
1 5 10 15

Leu Lys Lys Tyr Leu Ile Arg Glu Thr Gln Phe Asn Tyr Asp Glu Leu
20 25 30

Leu Arg Ala Asp Val Asn Ser Asp Gly Glu Val Asn Ser Thr Asp Tyr
35 40 45

Ala Tyr Leu Lys Arg Tyr Ile Leu Arg Ile Ile Asp Ala Phe Pro Gln
50 55 60

<210> 107
<211> 64
<212> PRT
<213> Artificial

<220>
<223> Synthetic peptide

<400> 107

Gly Asp Val Asn Asp Asp Gly Lys Val Asn Ser Thr Asp Ala Val Ala
1 5 10 15

Leu Lys Arg Tyr Val Leu Arg Ser Gly Ile Ser Ile Asn Thr Asp Asn
20 25 30

Ala Asp Leu Asn Glu Asp Gly Arg Val Asn Ser Thr Asp Leu Gly Ile
35 40 45

Leu Lys Arg Tyr Ile Leu Lys Glu Ile Asp Thr Leu Pro Tyr Lys Asn
50 55 60

<210> 108
<211> 61
<212> PRT
<213> Artificial

<220>
<223> Synthetic peptide

<400> 108

Gly Asp Val Asn Gly Asp Gly Arg Val Asn Ser Ser Asp Leu Thr Leu
1               5                   10                  15

Met Lys Arg Tyr Leu Leu Lys Ser Ile Ser Asp Phe Pro Thr Pro Glu
            20                  25                  30

Gly Lys Ile Ala Ala Asp Leu Asn Glu Asp Gly Lys Val Asn Ser Thr
        35                  40                  45

Asp Leu Leu Ala Leu Lys Lys Leu Val Leu Arg Glu Leu
    50                  55                  60

<210> 109
<211> 65
<212> PRT
<213> Artificial

<220>
<223> Synthetic peptide

<400> 109

Gly Asp Leu Asn Gly Asp Gly Arg Val Asn Ser Thr Asp Leu Leu Leu
1               5                   10                  15

Met Lys Lys Arg Ile Ile Arg Glu Ile Asp Lys Phe Asn Val Pro Asp
            20                  25                  30

Glu Asn Ala Asp Leu Asn Leu Asp Gly Lys Ile Asn Ser Ser Asp Tyr
        35                  40                  45

Thr Ile Leu Lys Arg Tyr Val Leu Lys Ser Ile Glu Lys Leu Pro Val
    50                  55                  60

Lys
65

<210> 110
<211> 63
<212> PRT
<213> Artificial

<220>
<223> Synthetic peptide

<400> 110

Gly Asp Val Asn Gly Asp Gly Arg Val Asn Ser Ser Asp Val Ala Leu
1 5 10 15

Leu Lys Arg Tyr Leu Leu Gly Leu Val Glu Asn Ile Asn Lys Glu Ala
20 25 30

Ala Asp Val Asn Val Ser Gly Thr Val Asn Ser Thr Asp Leu Ala Ile
35 40 45

Met Lys Arg Tyr Val Leu Arg Ser Ile Ser Glu Leu Pro Tyr Lys
50 55 60

<210> 111
<211> 71
<212> PRT
<213> Artificial

<220>
<223> Synthetic peptide

<400> 111

Gly Asp Val Asn Phe Asp Gly Arg Ile Asn Ser Thr Asp Tyr Ser Arg
1 5 10 15

Leu Lys Arg Tyr Val Ile Lys Ser Leu Glu Phe Thr Asp Pro Glu Glu
20 25 30

His Gln Lys Phe Ile Ala Ala Ala Asp Val Asp Gly Asn Gly Arg Ile
35 40 45

Asn Ser Thr Asp Leu Tyr Val Leu Asn Arg Tyr Ile Leu Lys Leu Ile
50 55 60

Glu Lys Phe Pro Ala Glu Gln
65 70

<210> 112
<211> 71
<212> PRT
<213> Artificial

<220>
<223> Synthetic peptide

<400> 112

Gly Asp Ile Asn Leu Asp Gly Lys Ile Asn Ser Ser Asp Val Thr Leu
1 5 10 15

Leu Lys Arg Tyr Ile Val Lys Ser Ile Asp Val Phe Pro Thr Ala Asp
20 25 30

Pro Glu Arg Ser Leu Ile Ala Ser Asp Val Asn Gly Asp Gly Arg Val
35 40 45

Asn Ser Thr Asp Tyr Ser Tyr Leu Lys Arg Tyr Val Leu Lys Ile Ile
50 55 60

Pro Thr Ile Pro Gly Asn Ser
65 70

<210> 113
<211> 62
<212> PRT
<213> Artificial

<220>
<223> Synthetic peptide

<400> 113

Gly Asp Ile Asn Leu Asp Gly Lys Ile Asn Ser Thr Asp Leu Ser Ala
1 5 10 15

Leu Lys Arg His Ile Leu Arg Ile Thr Thr Leu Ser Gly Lys Gln Leu
20 25 30

Glu Asn Ala Asp Val Asn Asn Asp Gly Ser Val Asn Ser Thr Asp Ala
35 40 45

Ser Ile Leu Lys Lys Tyr Ile Ala Lys Ala Ile Pro Ser Leu
50 55 60

<210> 114
<211> 63
<212> PRT
<213> Artificial

<220>
<223> Synthetic peptide

<400> 114

Gly Asp Leu Asn Gly Asp Gly Asn Ile Asn Ser Thr Asp Phe Thr Met
1 5 10 15

Leu Lys Arg Ala Ile Leu Gly Asn Pro Ala Pro Gly Thr Asn Leu Ala
20 25 30

Ala Gly Asp Leu Asn Arg Asp Gly Asn Thr Asn Ser Thr Asp Leu Met
35 40 45

Ile Leu Arg Arg Tyr Leu Leu Lys Leu Ile Gly Ser Leu Pro Ile
50 55 60

<210> 115
<211> 67
<212> PRT
<213> Artificial

<220>
<223> Synthetic peptide

<400> 115

Gly Asp Leu Asn Asn Asp Gly Asn Ile Asn Ser Thr Asp Tyr Met Ile
1 5 10 15

Leu Lys Lys Tyr Ile Leu Lys Val Leu Glu Arg Met Asn Val Pro Glu
20 25 30

Lys Ala Ala Asp Leu Asn Gly Asp Gly Ser Ile Asn Ser Thr Asp Leu
35 40 45

Thr Ile Leu Lys Arg Phe Ile Met Lys Ala Ile Thr Lys Phe Pro Val
50 55 60

Thr Gln Lys
65

<210> 116
<211> 69
<212> PRT
<213> Artificial

<220>
<223> Synthetic peptide

<400> 116

Gly Asp Val Asn Lys Asp Gly Arg Ile Asn Ser Thr Asp Ile Met Tyr
1               5                   10                  15

Leu Lys Gly Tyr Leu Leu Arg Asn Ser Ala Phe Asn Leu Asp Glu Tyr
            20                  25                  30

Gly Leu Met Ala Ala Asp Val Asp Gly Asn Gly Ser Val Ser Ser Leu
        35                  40                  45

Asp Leu Thr Tyr Leu Lys Arg Tyr Ile Leu Arg Arg Ile Ser Asp Phe
    50                  55                  60

Pro Ala Asn Lys Lys
65

<210> 117
<211> 66
<212> PRT
<213> Artificial

<220>
<223> Synthetic peptide

<400> 117

Gly Asp Leu Asn Gln Asp Gly Gln Val Ser Ser Thr Asp Leu Val Ala
1               5                   10                  15

Met Lys Arg Tyr Leu Leu Lys Asn Phe Glu Leu Ser Gly Val Gly Leu
            20                  25                  30

Glu Ala Ala Asp Leu Asn Ser Asp Gly Lys Val Asn Ser Thr Asp Leu
        35                  40                  45

Val Ala Leu Lys Arg Phe Leu Leu Lys Glu Ile Asp Glu Leu Pro Leu
    50                  55                  60

Lys Arg
65

<210> 118
<211> 64
<212> PRT
<213> Artificial

<220>
<223> Synthetic peptide

<400> 118

Gly Asp Thr Asn Ser Asp Gly Lys Ile Asn Ser Thr Asp Val Thr Ala
1               5                   10                  15

Leu Lys Arg His Leu Leu Arg Val Thr Gln Leu Thr Gly Asp Asn Leu
            20                  25                  30

Ala Asn Ala Asp Val Asn Gly Asp Gly Asn Val Asn Ser Thr Asp Leu
        35                  40                  45

Leu Leu Leu Lys Arg Tyr Ile Leu Gly Glu Ile Glu Asn Phe Pro Ile
    50                  55                  60

<210> 119
<211> 80
<212> PRT
<213> Artificial

<220>
<223> Synthetic peptide

<400> 119

Gly Asp Leu Asn Val Asp Gly Ser Ile Asn Ser Val Asp Ile Thr Tyr
1               5                   10                  15

Met Lys Arg Tyr Leu Leu Arg Ser Ile Ser Val Leu Pro Tyr Gln Glu
            20                  25                  30

Asn Glu Arg Ile Arg Ile Pro Ala Ala Asp Thr Asn Gly Asp Gly Ala
        35                  40                  45

Ile Asn Ser Ser Asp Met Val Leu Leu Lys Arg Tyr Val Leu Arg Ser
    50                  55                  60

Ile Ser Glu Phe Pro Val Lys Tyr Asp Ile Tyr Gly Asn Ile Ile Asn
65                  70                  75                  80

<210> 120
<211> 68
<212> PRT
<213> Artificial

<220>
<223> Synthetic peptide

<400> 120

Gly Asp Leu Asn Gly Asp Gly Lys Val Asn Ser Ser Asp Leu Ala Ile
1 5 10 15

Leu Lys Arg Tyr Met Leu Arg Ala Ile Ser Asp Phe Pro Ile Pro Glu
20 25 30

Gly Arg Lys Leu Ala Asp Leu Asn Arg Asp Gly Asn Val Asn Ser Thr
35 40 45

Asp Tyr Ser Ile Leu Lys Arg Tyr Ile Leu Lys Ala Ile Asp Asn Ile
50 55 60

Pro Val Asp Asp
65

<210> 121
<211> 62
<212> PRT
<213> Artificial

<220>
<223> Synthetic peptide

<400> 121

Gly Asp Val Asn Gly Asp Gly Asn Val Asn Ser Thr Asp Leu Thr Met
1 5 10 15

Leu Lys Arg Tyr Leu Leu Lys Ser Val Thr Asn Ile Asn Arg Glu Ala
20 25 30

Ala Asp Val Asn Arg Asp Gly Ala Ile Asn Ser Ser Asp Met Thr Ile
35 40 45

Leu Lys Arg Tyr Leu Ile Lys Ser Ile Pro His Leu Pro Tyr
50 55 60

<210> 122
<211> 63
<212> PRT
<213> Artificial

<220>
<223> Synthetic peptide

<400> 122

Gly Asp Val Asn Gly Asp Gly His Val Asn Ser Ser Asp Tyr Ser Leu
1 5 10 15

Phe Lys Arg Tyr Leu Leu Arg Val Ile Asp Arg Phe Pro Val Gly Asp
20 25 30

Gln Ser Val Ala Asp Val Asn Arg Asp Gly Arg Ile Asp Ser Thr Asp
35 40 45

Leu Thr Met Leu Lys Arg Tyr Leu Ile Arg Ala Ile Pro Ser Leu
50 55 60

<210> 123
<211> 455
<212> PRT
<213> Artificial

<220>
<223> Synthetic peptide

<400> 123

Met Gly Ser Gly Asp Val Asn Asp Asp Gly Lys Val Asn Ser Thr Asp
1               5                   10                  15

Ala Val Ala Leu Lys Arg Tyr Val Leu Arg Ser Gly Ile Ser Ile Asn
            20                  25                  30

Thr Asp Asn Ala Asp Leu Asn Glu Asp Gly Arg Val Asn Ser Thr Asp
        35                  40                  45

Leu Gly Ile Leu Lys Arg Tyr Ile Leu Lys Glu Ile Asp Thr Leu Pro
    50                  55                  60

Tyr Lys Asn Glu Phe Lys Asn Ala Asp Ser Tyr Ala Lys Lys Pro His
65                  70                  75                  80

Ile Ser Ala Leu Asn Ala Pro Gln Leu Asp Gln Arg Tyr Lys Asn Glu
                85                  90                  95

Phe Thr Ile Gly Ala Ala Val Glu Pro Tyr Gln Leu Gln Asn Glu Lys
            100                 105                 110

Asp Val Gln Met Leu Lys Arg His Phe Asn Ser Ile Val Ala Glu Asn
        115                 120                 125

Val Met Lys Pro Ile Ser Ile Gln Pro Glu Glu Gly Lys Phe Asn Phe
    130                 135                 140

Glu Gln Ala Asp Arg Ile Val Lys Phe Ala Lys Ala Asn Gly Met Asp
145                 150                 155                 160

Ile Arg Phe His Thr Leu Val Trp His Ser Gln Val Pro Gln Trp Phe
                165                 170                 175

Phe Leu Asp Lys Glu Gly Lys Pro Met Val Asn Glu Thr Asp Pro Val
180 185 190

Lys Arg Glu Gln Asn Lys Gln Leu Leu Leu Lys Arg Leu Glu Thr His
195 200 205

Ile Lys Thr Ile Val Glu Arg Tyr Lys Asp Asp Ile Lys Tyr Trp Asp
210 215 220

Val Val Asn Glu Val Val Gly Asp Asp Gly Lys Leu Arg Asn Ser Pro
225 230 235 240

Trp Tyr Gln Ile Ala Gly Ile Asp Tyr Ile Lys Val Ala Phe Gln Ala
245 250 255

Ala Arg Lys Tyr Gly Gly Asp Asn Ile Lys Leu Tyr Met Asn Asp Tyr
260 265 270

Asn Thr Glu Val Glu Pro Lys Arg Thr Ala Leu Tyr Asn Leu Val Lys
275 280 285

Gln Leu Lys Glu Glu Gly Val Pro Ile Asp Gly Ile Gly His Gln Ser
290 295 300

His Ile Gln Ile Gly Trp Pro Ser Glu Ala Glu Ile Glu Lys Thr Ile
305 310 315 320

Asn Met Phe Ala Ala Leu Gly Leu Asp Asn Gln Ile Thr Glu Leu Asp
325 330 335

Val Ser Met Tyr Gly Trp Pro Pro Arg Ala Tyr Pro Thr Tyr Asp Ala
340 345 350

Ile Pro Lys Gln Lys Phe Leu Asp Gln Ala Ala Arg Tyr Asp Arg Leu
355 360 365

Phe Lys Leu Tyr Glu Lys Leu Ser Asp Lys Ile Ser Asn Val Thr Phe
370 375 380

Trp Gly Ile Ala Asp Asn His Thr Trp Leu Asp Ser Arg Ala Asp Val
385 390 395 400

Tyr Tyr Asp Ala Asn Gly Asn Val Val Val Asp Pro Asn Ala Pro Tyr
405 410 415

Ala Lys Val Glu Lys Gly Lys Gly Lys Asp Ala Pro Phe Val Phe Gly
420 425 430

Pro Asp Tyr Lys Val Lys Pro Ala Tyr Trp Ala Ile Ile Asp His Leu
435 440 445

```
Glu His His His His His His
    450                 455
```

<210> 124
<211> 438
<212> PRT
<213> Artificial

<220>
<223> Synthetic peptide

<400> 124

```
Met Gly Ser Lys Arg Tyr Val Leu Arg Ser Gly Ile Ser Ile Asn Thr
1               5                   10                  15

Asp Asn Ala Asp Leu Asn Glu Asp Gly Arg Val Asn Ser Thr Asp Leu
            20                  25                  30

Gly Ile Leu Lys Arg Tyr Ile Leu Lys Glu Ile Asp Thr Leu Pro Tyr
        35                  40                  45

Lys Asn Glu Phe Lys Asn Ala Asp Ser Tyr Ala Lys Lys Pro His Ile
    50                  55                  60

Ser Ala Leu Asn Ala Pro Gln Leu Asp Gln Arg Tyr Lys Asn Glu Phe
65                  70                  75                  80

Thr Ile Gly Ala Ala Val Glu Pro Tyr Gln Leu Gln Asn Glu Lys Asp
                85                  90                  95

Val Gln Met Leu Lys Arg His Phe Asn Ser Ile Val Ala Glu Asn Val
            100                 105                 110

Met Lys Pro Ile Ser Ile Gln Pro Glu Glu Gly Lys Phe Asn Phe Glu
        115                 120                 125

Gln Ala Asp Arg Ile Val Lys Phe Ala Lys Ala Asn Gly Met Asp Ile
    130                 135                 140

Arg Phe His Thr Leu Val Trp His Ser Gln Val Pro Gln Trp Phe Phe
145                 150                 155                 160

Leu Asp Lys Glu Gly Lys Pro Met Val Asn Glu Thr Asp Pro Val Lys
                165                 170                 175

Arg Glu Gln Asn Lys Gln Leu Leu Leu Lys Arg Leu Glu Thr His Ile
            180                 185                 190

Lys Thr Ile Val Glu Arg Tyr Lys Asp Asp Ile Lys Tyr Trp Asp Val
        195                 200                 205
```

Val Asn Glu Val Val Gly Asp Asp Gly Lys Leu Arg Asn Ser Pro Trp
210 215 220

Tyr Gln Ile Ala Gly Ile Asp Tyr Ile Lys Val Ala Phe Gln Ala Ala
225 230 235 240

Arg Lys Tyr Gly Gly Asp Asn Ile Lys Leu Tyr Met Asn Asp Tyr Asn
245 250 255

Thr Glu Val Glu Pro Lys Arg Thr Ala Leu Tyr Asn Leu Val Lys Gln
260 265 270

Leu Lys Glu Glu Gly Val Pro Ile Asp Gly Ile Gly His Gln Ser His
275 280 285

Ile Gln Ile Gly Trp Pro Ser Glu Ala Glu Ile Glu Lys Thr Ile Asn
290 295 300

Met Phe Ala Ala Leu Gly Leu Asp Asn Gln Ile Thr Glu Leu Asp Val
305 310 315 320

Ser Met Tyr Gly Trp Pro Pro Arg Ala Tyr Pro Thr Tyr Asp Ala Ile
325 330 335

Pro Lys Gln Lys Phe Leu Asp Gln Ala Ala Arg Tyr Asp Arg Leu Phe
340 345 350

Lys Leu Tyr Glu Lys Leu Ser Asp Lys Ile Ser Asn Val Thr Phe Trp
355 360 365

Gly Ile Ala Asp Asn His Thr Trp Leu Asp Ser Arg Ala Asp Val Tyr
370 375 380

Tyr Asp Ala Asn Gly Asn Val Val Val Asp Pro Asn Ala Pro Tyr Ala
385 390 395 400

Lys Val Glu Lys Gly Lys Gly Lys Asp Ala Pro Phe Val Phe Gly Pro
405 410 415

Asp Tyr Lys Val Lys Pro Ala Tyr Trp Ala Ile Ile Asp His Leu Glu
420 425 430

His His His His His His
435

<210> 125
<211> 315
<212> PRT
<213> Artificial

<220>
<223> Synthetic peptide

<400> 125

```
Met Gly Ser Gly Asp Val Asn Asp Asp Gly Lys Val Asn Ser Thr Asp
1               5                   10                  15

Ala Val Ala Leu Lys Arg Tyr Val Leu Arg Ser Gly Ile Ser Ile Asn
            20                  25                  30

Thr Asp Asn Ala Asp Leu Asn Glu Asp Gly Arg Val Asn Ser Thr Asp
        35                  40                  45

Leu Gly Ile Leu Lys Arg Tyr Ile Leu Lys Glu Ile Asp Thr Leu Pro
    50                  55                  60

Tyr Lys Asn Glu Phe Met Ser Lys Gly Glu Glu Leu Phe Thr Gly Val
65                  70                  75                  80

Val Pro Ile Leu Val Glu Leu Asp Gly Asp Val Asn Gly His Lys Phe
                85                  90                  95

Ser Val Ser Gly Glu Gly Glu Gly Asp Ala Thr Tyr Gly Lys Leu Thr
            100                 105                 110

Leu Lys Phe Ile Cys Thr Thr Gly Lys Leu Pro Val Pro Trp Pro Thr
        115                 120                 125

Leu Val Thr Thr Leu Thr Tyr Gly Val Gln Cys Phe Ser Arg Tyr Pro
    130                 135                 140

Asp His Met Lys Arg His Asp Phe Phe Lys Ser Ala Met Pro Glu Gly
145                 150                 155                 160

Tyr Val Gln Glu Arg Thr Ile Ser Phe Lys Asp Asp Gly Asn Tyr Lys
                165                 170                 175

Thr Arg Ala Glu Val Lys Phe Glu Gly Asp Thr Leu Val Asn Arg Ile
            180                 185                 190

Glu Leu Lys Gly Ile Asp Phe Lys Glu Asp Gly Asn Ile Leu Gly His
        195                 200                 205

Lys Leu Glu Tyr Asn Tyr Asn Ser His Asn Val Tyr Ile Thr Ala Asp
    210                 215                 220

Lys Gln Lys Asn Gly Ile Lys Ala Asn Phe Lys Ile Arg His Asn Ile
225                 230                 235                 240

Glu Asp Gly Ser Val Gln Leu Ala Asp His Tyr Gln Gln Asn Thr Pro
                245                 250                 255

Ile Gly Asp Gly Pro Val Leu Leu Pro Asp Asn His Tyr Leu Ser Thr
            260                 265                 270
```

Gln Ser Ala Leu Ser Lys Asp Pro Asn Glu Lys Arg Asp His Met Val
275 280 285

Leu Leu Glu Phe Val Thr Ala Ala Gly Ile Thr His Gly Met Asp Glu
290 295 300

Leu Tyr Lys Leu Glu His His His His His His
305 310 315

<210> 126
<211> 298
<212> PRT
<213> Artificial

<220>
<223> Synthetic peptide

<400> 126

Met Gly Ser Lys Arg Tyr Val Leu Arg Ser Gly Ile Ser Ile Asn Thr
1 5 10 15

Asp Asn Ala Asp Leu Asn Glu Asp Gly Arg Val Asn Ser Thr Asp Leu
20 25 30

Gly Ile Leu Lys Arg Tyr Ile Leu Lys Glu Ile Asp Thr Leu Pro Tyr
35 40 45

Lys Asn Glu Phe Met Ser Lys Gly Glu Glu Leu Phe Thr Gly Val Val
50 55 60

Pro Ile Leu Val Glu Leu Asp Gly Asp Val Asn Gly His Lys Phe Ser
65 70 75 80

Val Ser Gly Glu Gly Glu Gly Asp Ala Thr Tyr Gly Lys Leu Thr Leu
85 90 95

Lys Phe Ile Cys Thr Thr Gly Lys Leu Pro Val Pro Trp Pro Thr Leu
100 105 110

Val Thr Thr Leu Thr Tyr Gly Val Gln Cys Phe Ser Arg Tyr Pro Asp
115 120 125

His Met Lys Arg His Asp Phe Phe Lys Ser Ala Met Pro Glu Gly Tyr
130 135 140

Val Gln Glu Arg Thr Ile Ser Phe Lys Asp Asp Gly Asn Tyr Lys Thr
145 150 155 160

Arg Ala Glu Val Lys Phe Glu Gly Asp Thr Leu Val Asn Arg Ile Glu
165 170 175

Leu Lys Gly Ile Asp Phe Lys Glu Asp Gly Asn Ile Leu Gly His Lys
180 185 190

Leu Glu Tyr Asn Tyr Asn Ser His Asn Val Tyr Ile Thr Ala Asp Lys
195 200 205

Gln Lys Asn Gly Ile Lys Ala Asn Phe Lys Ile Arg His Asn Ile Glu
210 215 220

Asp Gly Ser Val Gln Leu Ala Asp His Tyr Gln Gln Asn Thr Pro Ile
225 230 235 240

Gly Asp Gly Pro Val Leu Leu Pro Asp Asn His Tyr Leu Ser Thr Gln
245 250 255

Ser Ala Leu Ser Lys Asp Pro Asn Glu Lys Arg Asp His Met Val Leu
260 265 270

Leu Glu Phe Val Thr Ala Ala Gly Ile Thr His Gly Met Asp Glu Leu
275 280 285

Tyr Lys Leu Glu His His His His His His
290 295

<210> 127
<211> 312
<212> PRT
<213> Artificial

<220>
<223> Synthetic peptide

<400> 127

Met Ser His His His His His His Met Ser Lys Gly Glu Glu Leu Phe
1               5                   10                  15

Thr Gly Val Val Pro Ile Leu Val Glu Leu Asp Gly Asp Val Asn Gly
            20                  25                  30

His Lys Phe Ser Val Ser Gly Glu Gly Glu Gly Asp Ala Thr Tyr Gly
        35                  40                  45

Lys Leu Thr Leu Lys Phe Ile Cys Thr Thr Gly Lys Leu Pro Val Pro
    50                  55                  60

Trp Pro Thr Leu Val Thr Thr Leu Thr Tyr Gly Val Gln Cys Phe Ser
65                  70                  75                  80

Arg Tyr Pro Asp His Met Lys Arg His Asp Phe Phe Lys Ser Ala Met
                85                  90                  95

Pro Glu Gly Tyr Val Gln Glu Arg Thr Ile Ser Phe Lys Asp Asp Gly
            100                 105                 110

Asn Tyr Lys Thr Arg Ala Glu Val Lys Phe Glu Gly Asp Thr Leu Val
115 120 125

Asn Arg Ile Glu Leu Lys Gly Ile Asp Phe Lys Glu Asp Gly Asn Ile
130 135 140

Leu Gly His Lys Leu Glu Tyr Asn Tyr Asn Ser His Asn Val Tyr Ile
145 150 155 160

Thr Ala Asp Lys Gln Lys Asn Gly Ile Lys Ala Asn Phe Lys Ile Arg
165 170 175

His Asn Ile Glu Asp Gly Ser Val Gln Leu Ala Asp His Tyr Gln Gln
180 185 190

Asn Thr Pro Ile Gly Asp Gly Pro Val Leu Leu Pro Asp Asn His Tyr
195 200 205

Leu Ser Thr Gln Ser Ala Leu Ser Lys Asp Pro Asn Glu Lys Arg Asp
210 215 220

His Met Val Leu Leu Glu Phe Val Thr Ala Ala Gly Ile Thr His Gly
225 230 235 240

Met Asp Glu Leu Tyr Lys Val Val Pro Asp Val Asn Asp Asp Gly Lys
245 250 255

Val Asn Ser Thr Asp Ala Val Ala Leu Lys Arg Tyr Val Leu Arg Ser
260 265 270

Gly Ile Ser Ile Asn Thr Asp Asn Ala Asp Leu Asn Glu Asp Gly Arg
275 280 285

Val Asn Ser Thr Asp Leu Gly Ile Leu Lys Arg Tyr Ile Leu Lys Glu
290 295 300

Ile Asp Thr Leu Pro Tyr Lys Asn
305 310

<210> 128
<211> 296
<212> PRT
<213> Artificial

<220>
<223> Synthetic peptide

<400> 128

Met Ser His His His His His His Met Ser Lys Gly Glu Glu Leu Phe
1 5 10 15

```
Thr Gly Val Val Pro Ile Leu Val Glu Leu Asp Gly Asp Val Asn Gly
            20                  25                  30

His Lys Phe Ser Val Ser Gly Glu Gly Glu Gly Asp Ala Thr Tyr Gly
        35                  40                  45

Lys Leu Thr Leu Lys Phe Ile Cys Thr Thr Gly Lys Leu Pro Val Pro
    50                  55                  60

Trp Pro Thr Leu Val Thr Thr Leu Thr Tyr Gly Val Gln Cys Phe Ser
65                  70                  75                  80

Arg Tyr Pro Asp His Met Lys Arg His Asp Phe Phe Lys Ser Ala Met
                85                  90                  95

Pro Glu Gly Tyr Val Gln Glu Arg Thr Ile Ser Phe Lys Asp Asp Gly
            100                 105                 110

Asn Tyr Lys Thr Arg Ala Glu Val Lys Phe Glu Gly Asp Thr Leu Val
        115                 120                 125

Asn Arg Ile Glu Leu Lys Gly Ile Asp Phe Lys Glu Asp Gly Asn Ile
    130                 135                 140

Leu Gly His Lys Leu Glu Tyr Asn Tyr Asn Ser His Asn Val Tyr Ile
145                 150                 155                 160

Thr Ala Asp Lys Gln Lys Asn Gly Ile Lys Ala Asn Phe Lys Ile Arg
                165                 170                 175

His Asn Ile Glu Asp Gly Ser Val Gln Leu Ala Asp His Tyr Gln Gln
            180                 185                 190

Asn Thr Pro Ile Gly Asp Gly Pro Val Leu Leu Pro Asp Asn His Tyr
        195                 200                 205

Leu Ser Thr Gln Ser Ala Leu Ser Lys Asp Pro Asn Glu Lys Arg Asp
    210                 215                 220

His Met Val Leu Leu Glu Phe Val Thr Ala Ala Gly Ile Thr His Gly
225                 230                 235                 240

Met Asp Glu Leu Tyr Lys Val Val Pro Lys Arg Tyr Val Leu Arg Ser
                245                 250                 255

Gly Ile Ser Ile Asn Thr Asp Asn Ala Asp Leu Asn Glu Asp Gly Arg
            260                 265                 270

Val Asn Ser Thr Asp Leu Gly Ile Leu Lys Arg Tyr Ile Leu Lys Glu
        275                 280                 285
```

Ile Asp Thr Leu Pro Tyr Lys Asn
290 295

<210> 129
<211> 243
<212> PRT
<213> Artificial

<220>
<223> Synthetic peptide

<400> 129

Met Gly Ser Lys Arg Tyr Val Leu Arg Ser Gly Ile Ser Ile Asn Thr
1 5 10 15

Asp Asn Ala Asp Leu Asn Glu Asp Gly Arg Val Asn Ser Thr Asp Leu
20 25 30

Gly Ile Leu Lys Arg Tyr Ile Leu Lys Glu Ile Asp Thr Leu Pro Tyr
35 40 45

Lys Asn Glu Phe Met Ala Asp Thr Leu Leu Ile Leu Gly Asp Ser Leu
50 55 60

Ser Ala Gly Tyr Arg Met Ser Ala Ser Ala Ala Trp Pro Ala Leu Leu
65 70 75 80

Asn Asp Lys Trp Gln Ser Lys Thr Ser Val Val Asn Ala Ser Ile Ser
85 90 95

Gly Asp Thr Ser Gln Gln Gly Leu Ala Arg Leu Pro Ala Leu Leu Lys
100 105 110

Gln His Gln Pro Arg Trp Val Leu Val Glu Leu Gly Gly Asn Asp Gly
115 120 125

Leu Arg Gly Phe Gln Pro Gln Gln Thr Glu Gln Thr Leu Arg Gln Ile
130 135 140

Leu Gln Asp Val Lys Ala Ala Asn Ala Glu Pro Leu Leu Met Gln Ile
145 150 155 160

Arg Leu Pro Ala Asn Tyr Gly Arg Arg Tyr Asn Glu Ala Phe Ser Ala
165 170 175

Ile Tyr Pro Lys Leu Ala Lys Glu Phe Asp Val Pro Leu Leu Pro Phe
180 185 190

Leu Met Glu Glu Val Tyr Leu Lys Pro Gln Trp Met Gln Asp Asp Gly
195 200 205

Ile His Pro Asn Arg Asp Ala Gln Pro Phe Ile Ala Asp Trp Met Ala
210 215 220

Lys Gln Leu Gln Pro Leu Val Asn His Asp Ser Leu Glu His His His
225 230 235 240

His His His

<210> 130
<211> 508
<212> PRT
<213> Artificial

<220>
<223> synthetic peptide

<400> 130

Met Gly Ser Lys Arg Tyr Val Leu Arg Ser Gly Ile Ser Ile Asn Thr
1               5                   10                  15

Asp Asn Ala Asp Leu Asn Glu Asp Gly Arg Val Asn Ser Thr Asp Leu
            20                  25                  30

Gly Ile Leu Lys Arg Tyr Ile Leu Lys Glu Ile Asp Thr Leu Pro Tyr
        35                  40                  45

Lys Asn Glu Phe Met Ser Lys Ile Thr Phe Pro Lys Asp Phe Ile Trp
    50                  55                  60

Gly Ser Ala Thr Ala Ala Tyr Gln Ile Glu Gly Ala Tyr Asn Glu Asp
65                  70                  75                  80

Gly Lys Gly Glu Ser Ile Trp Asp Arg Phe Ser His Thr Pro Gly Asn
                85                  90                  95

Ile Ala Asp Gly His Thr Gly Asp Val Ala Cys Asp His Tyr His Arg
            100                 105                 110

Tyr Glu Glu Asp Ile Lys Ile Met Lys Glu Ile Gly Ile Lys Ser Tyr
        115                 120                 125

Arg Phe Ser Ile Ser Trp Pro Arg Ile Phe Pro Glu Gly Thr Gly Lys
    130                 135                 140

Leu Asn Gln Lys Gly Leu Asp Phe Tyr Lys Arg Leu Thr Asn Leu Leu
145                 150                 155                 160

Leu Glu Asn Gly Ile Met Pro Ala Ile Thr Leu Tyr His Trp Asp Leu
                165                 170                 175

Pro Gln Lys Leu Gln Asp Lys Gly Gly Trp Lys Asn Arg Asp Thr Thr
            180                 185                 190

```
Asp Tyr Phe Thr Glu Tyr Ser Glu Val Ile Phe Lys Asn Leu Gly Asp
        195                 200                 205

Ile Val Pro Ile Trp Phe Thr His Asn Glu Pro Gly Val Val Ser Leu
    210                 215                 220

Leu Gly His Phe Leu Gly Ile His Ala Pro Gly Ile Lys Asp Leu Arg
225                 230                 235                 240

Thr Ser Leu Glu Val Ser His Asn Leu Leu Leu Ser His Gly Lys Ala
                245                 250                 255

Val Lys Leu Phe Arg Glu Met Asn Ile Asp Ala Gln Ile Gly Ile Ala
            260                 265                 270

Leu Asn Leu Ser Tyr His Tyr Pro Ala Ser Glu Lys Ala Glu Asp Ile
        275                 280                 285

Glu Ala Ala Glu Leu Ser Phe Ser Leu Ala Gly Arg Trp Tyr Leu Asp
    290                 295                 300

Pro Val Leu Lys Gly Arg Tyr Pro Glu Asn Ala Leu Lys Leu Tyr Lys
305                 310                 315                 320

Lys Lys Gly Ile Glu Leu Ser Phe Pro Glu Asp Asp Leu Lys Leu Ile
                325                 330                 335

Ser Gln Pro Ile Asp Phe Ile Ala Phe Asn Asn Tyr Ser Ser Glu Phe
            340                 345                 350

Ile Lys Tyr Asp Pro Ser Ser Glu Ser Gly Phe Ser Pro Ala Asn Ser
        355                 360                 365

Ile Leu Glu Lys Phe Glu Lys Thr Asp Met Gly Trp Ile Ile Tyr Pro
    370                 375                 380

Glu Gly Leu Tyr Asp Leu Leu Met Leu Leu Asp Arg Asp Tyr Gly Lys
385                 390                 395                 400

Pro Asn Ile Val Ile Ser Glu Asn Gly Ala Ala Phe Lys Asp Glu Ile
                405                 410                 415

Gly Ser Asn Gly Lys Ile Glu Asp Thr Lys Arg Ile Gln Tyr Leu Lys
            420                 425                 430

Asp Tyr Leu Thr Gln Ala His Arg Ala Ile Gln Asp Gly Val Asn Leu
        435                 440                 445

Lys Ala Tyr Tyr Leu Trp Ser Leu Leu Asp Asn Phe Glu Trp Ala Tyr
    450                 455                 460
```

Gly Tyr Asn Lys Arg Phe Gly Ile Val His Val Asn Phe Asp Thr Leu
465 470 475 480

Glu Arg Lys Ile Lys Asp Ser Gly Tyr Trp Tyr Lys Glu Val Ile Lys
485 490 495

Asn Asn Gly Phe Leu Glu His His His His His His
500 505

<210> 131
<211> 198
<212> PRT
<213> Artificial

<220>
<223> Synthetic peptide

<400> 131

Met Gly Ser Lys Arg Tyr Val Leu Arg Ser Gly Ile Ser Ile Asn Thr
1 5 10 15

Asp Asn Ala Asp Leu Asn Glu Asp Gly Arg Val Asn Ser Thr Asp Leu
20 25 30

Gly Ile Leu Lys Arg Tyr Ile Leu Lys Glu Ile Asp Thr Leu Pro Tyr
35 40 45

Lys Asn Glu Phe Leu Val Pro Arg Gly Ser Met Val Asp Asn Lys Phe
50 55 60

Asn Lys Glu Gln Gln Asn Ala Phe Tyr Glu Ile Leu His Leu Pro Asn
65 70 75 80

Leu Asn Glu Glu Gln Arg Asn Ala Phe Ile Gln Ser Leu Lys Asp Asp
85 90 95

Pro Ser Gln Ser Ala Asn Leu Leu Ala Glu Ala Lys Lys Leu Asn Asp
100 105 110

Ala Gln Ala Pro Lys Val Asp Asn Lys Phe Asn Lys Glu Gln Gln Asn
115 120 125

Ala Phe Tyr Glu Ile Leu His Leu Pro Asn Leu Asn Glu Glu Gln Arg
130 135 140

Asn Ala Phe Ile Gln Ser Leu Lys Asp Asp Pro Ser Gln Ser Ala Asn
145 150 155 160

Leu Leu Ala Glu Ala Lys Lys Leu Asn Asp Ala Gln Ala Pro Lys Ala
165 170 175

Ala Ala Gly Gly Asp Tyr Lys Asp Asp Asp Asp Lys Leu Ala Leu Glu
180 185 190

His His His His His His
195

<210> 132
<211> 33
<212> PRT
<213> Artificial

<220>
<223> synthetic peptide

<400> 132

Cys Cys Cys Cys Ala Thr Gly Gly Gly Ala Thr Cys Cys Gly Gly Cys
1 5 10 15

Gly Ala Cys Gly Thr Cys Ala Ala Thr Gly Ala Thr Gly Ala Cys Gly
20 25 30

Gly

<210> 133
<211> 30
<212> PRT
<213> Artificial

<220>
<223> synthetic peptide

<400> 133

Gly Gly Gly Gly Ala Ala Thr Thr Cys Gly Thr Thr Cys Thr Thr Gly
1 5 10 15

Thr Ala Cys Gly Gly Cys Ala Ala Thr Gly Thr Ala Thr Cys
20 25 30

<210> 134
<211> 46
<212> PRT
<213> Artificial

<220>
<223> Synthetic peptide

<400> 134

Gly Ala Thr Ala Cys Ala Thr Thr Gly Cys Cys Gly Thr Ala Cys Ala
1 5 10 15

Ala Gly Ala Ala Cys Gly Ala Ala Thr Thr Cys Ala Thr Gly Ala Gly
20 25 30

Thr Ala Ala Ala Gly Gly Ala Gly Ala Ala Gly Ala Ala Cys
35 40 45

<210> 135
<211> 48
<212> PRT
<213> Artificial

<220>
<223> Synthetic peptide

<400> 135

Cys Thr Cys Ala Gly Thr Gly Gly Thr Gly Gly Thr Gly Gly Thr Gly
1               5                   10                  15

Gly Thr Gly Gly Thr Gly Cys Thr Cys Gly Ala Gly Thr Thr Thr Gly
            20                  25                  30

Thr Ala Gly Ala Gly Cys Thr Cys Ala Thr Cys Cys Ala Thr Gly Cys
        35                  40                  45

<210> 136
<211> 48
<212> PRT
<213> Artificial

<220>
<223> Synthetic peptide

<400> 136

Ala Cys Cys Ala Thr Gly Ala Gly Cys Cys Ala Cys Cys Ala Thr Cys
1               5                   10                  15

Ala Cys Cys Ala Thr Cys Ala Cys Cys Ala Thr Ala Thr Gly Ala Gly
            20                  25                  30

Thr Ala Ala Ala Gly Gly Ala Gly Ala Ala Gly Ala Ala Cys Thr Thr
        35                  40                  45

<210> 137
<211> 48
<212> PRT
<213> Artificial

<220>
<223> Synthetic peptide

<400> 137

Gly Thr Cys Ala Thr Cys Ala Thr Thr Gly Ala Cys Gly Thr Cys Gly
1               5                   10                  15

Cys Cys Ala Gly Gly Thr Ala Cys Cys Ala Cys Thr Thr Thr Gly Thr
            20                  25                  30

Ala Gly Ala Gly Cys Thr Cys Ala Thr Cys Cys Ala Thr Gly Cys Cys
        35                  40                  45

<210> 138
<211> 34
<212> PRT
<213> Artificial

<220>
<223> Synthetic peptide

<400> 138

Cys Cys Ala Thr Cys Ala Gly Ala Ala Thr Thr Cys Ala Thr Gly Gly
1 5 10 15

Cys Gly Gly Ala Cys Ala Cys Gly Thr Thr Ala Thr Thr Gly Ala Thr
20 25 30

Thr Cys

<210> 139
<211> 32
<212> PRT
<213> Artificial

<220>
<223> Synthetic peptide

<400> 139

Cys Ala Gly Ala Thr Ala Cys Thr Cys Gly Ala Gly Thr Gly Ala Gly
1 5 10 15

Thr Cys Ala Thr Gly Ala Thr Thr Thr Ala Cys Thr Ala Ala Ala Gly
20 25 30

<210> 140
<211> 32
<212> PRT
<213> Artificial

<220>
<223> Synthitic peptide

<400> 140

Gly Thr Ala Thr Cys Cys Gly Ala Ala Thr Thr Cys Ala Thr Gly Thr
1 5 10 15

Cys Ala Ala Ala Gly Ala Thr Ala Ala Cys Thr Thr Thr Cys Cys Cys
20 25 30

<210> 141
<211> 33
<212> PRT
<213> Artificial

<220>
<223> Synthetic peptide

<400> 141

Gly Cys Ala Thr Ala Ala Cys Thr Cys Gly Ala Gly Ala Ala Ala Ala
1 5 10 15

Cys Cys Gly Thr Thr Gly Thr Thr Thr Thr Thr Gly Ala Thr Thr Ala
20 25 30

Cys

<210> 142
<211> 35
<212> PRT
<213> Artificial

<220>
<223> Synthetic peptide

<400> 142

Gly Thr Ala Cys Ala Ala Gly Ala Ala Cys Gly Ala Ala Thr Thr Cys
1 5 10 15

Ala Thr Gly Thr Cys Ala Ala Ala Gly Ala Thr Ala Ala Cys Thr Thr
20 25 30

Thr Cys Cys
35

<210> 143
<211> 37
<212> PRT
<213> Artificial

<220>
<223> Synthetic peptide

<400> 143

Gly Gly Thr Gly Gly Thr Gly Gly Thr Gly Cys Thr Cys Gly Ala Gly
1 5 10 15

Ala Ala Ala Ala Cys Cys Gly Thr Thr Gly Thr Thr Thr Thr Thr Gly
20 25 30

Ala Thr Thr Ala Cys
35

<210> 144
<211> 33
<212> PRT
<213> Artificial

<220>
<223> Synthetic peptide

<400> 144

Cys Ala Cys Gly Gly Thr Gly Ala Ala Thr Thr Cys Cys Thr Gly Gly
1 5 10 15

Thr Gly Cys Cys Ala Cys Gly Cys Gly Gly Thr Thr Cys Cys Ala Thr
20 25 30

Gly

<210> 145
<211> 33
<212> PRT
<213> Artificial

<220>
<223> Synthetic peptide

<400> 145

Cys Cys Ala Ala Thr Gly Cys Thr Cys Gly Ala Gly Thr Gly Cys Ala
1 5 10 15

Ala Gly Cys Thr Thr Gly Thr Cys Ala Thr Cys Gly Thr Cys Gly Thr
20 25 30

Cys

<210> 146
<211> 34
<212> PRT
<213> Artificial

<220>
<223> Synthetic peptide

<400> 146

Thr Ala Thr Ala Cys Cys Ala Thr Gly Gly Gly Ala Thr Cys Cys Ala
1 5 10 15

Ala Gly Ala Gly Ala Thr Ala Thr Gly Thr Thr Thr Thr Gly Ala Gly
20 25 30

Ala Thr

<210> 147
<211> 40
<212> PRT
<213> Artificial

<220>
<223> Synthetic peptide

<400> 147

Thr Cys Thr Thr Gly Ala Ala Thr Thr Cys Gly Thr Thr Cys Thr Thr
1 5 10 15

Gly Thr Ala Cys Gly Gly Cys Ala Ala Thr Gly Thr Ala Thr Cys Thr
20 25 30

Ala Thr Thr Thr Cys Thr Thr Thr
35 40

<210> 148
<211> 46
<212> PRT
<213> Artificial

<220>
<223> Synthetic peptide

<400> 148

Gly Tyr Val Leu Arg Ser Gly Ile Ser Ile Asn Thr Asp Asn Ala Asp
1 5 10 15

Leu Asn Glu Asp Gly Arg Val Asn Ser Thr Asp Leu Gly Ile Leu Lys
20 25 30

Arg Tyr Ile Leu Lys Glu Ile Asp Thr Leu Pro Tyr Lys Asn
35 40 45

<210> 149
<211> 44
<212> PRT
<213> Artificial

<220>
<223> Synthetic peptide

<400> 149

Gly Leu Arg Ser Gly Ile Ser Ile Asn Thr Asp Asn Ala Asp Leu Asn
1 5 10 15

Glu Asp Gly Arg Val Asn Ser Thr Asp Leu Gly Ile Leu Lys Arg Tyr
20 25 30

Ile Leu Lys Glu Ile Asp Thr Leu Pro Tyr Lys Asn
35 40

<210> 150
<211> 42
<212> PRT
<213> Artificial

<220>
<223> Synthetic peptide

<400> 150

Gly Ser Gly Ile Ser Ile Asn Thr Asp Asn Ala Asp Leu Asn Glu Asp
1 5 10 15

Gly Arg Val Asn Ser Thr Asp Leu Gly Ile Leu Lys Arg Tyr Ile Leu
20 25 30

Lys Glu Ile Asp Thr Leu Pro Tyr Lys Asn
35 40

<210> 151
<211> 40
<212> PRT
<213> Artificial

<220>
<223> Synthetic peptide

<400> 151

Gly Ile Ser Ile Asn Thr Asp Asn Ala Asp Leu Asn Glu Asp Gly Arg
1 5 10 15

Val Asn Ser Thr Asp Leu Gly Ile Leu Lys Arg Tyr Ile Leu Lys Glu
20 25 30

Ile Asp Thr Leu Pro Tyr Lys Asn
35 40

<210> 152
<211> 38
<212> PRT
<213> Artificial

<220>
<223> synthetic peptide

<400> 152

Gly Ile Asn Thr Asp Asn Ala Asp Leu Asn Glu Asp Gly Arg Val Asn
1 5 10 15

Ser Thr Asp Leu Gly Ile Leu Lys Arg Tyr Ile Leu Lys Glu Ile Asp
20 25 30

Thr Leu Pro Tyr Lys Asn
35